# EUROPÄISCHE PATENTSCHRIFT

(11) **EP 1 368 355 B1**
(45) Veröffentlichungstag und Bekanntmachung des Hinweises auf die Patenterteilung: **07.06.2006**
(21) Anmeldenummer: 02729998.1
(22) Anmeldetag: 13.03.2002
(51) Int. Cl.: C07D 487/04, C07D 513/04, A61K 31/505, A61P 25/00, A61P 29/00

(54) **SUBSTITUIERTE PYRAZOLO- UND THIAZOLOPYRIMIDINE ALS ANALGETIKA**
SUBSTITUTED PYRAZOLOPYRIMIDINES AND THIAZOLOPYRIMIDINES
PYRAZOLOPYRIMIDINES ET THIAZOLOPYRIMIDINES SUBSTITUEES

(30) Priorität: 14.03.2001 DE 10112197; 29.10.2001 DE 10153344
(43) Veröffentlichungstag der Anmeldung: 10.12.2003
(60) Teilanmeldung: 05026811.9
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE); JAGUSCH, Utz-Peter, 52066 Aachen (DE); SUNDERMANN, Bernd, 52066 Aachen (DE); FUHR, Martin, 47799 Krefeld (DE); IJZERMAN, Adriaan, P., NL-2036 MB Haarlem (NL); DISSEN-DE GROOTE, Miriam, NL 2624 CW Delft (NL)
(86) Internationale Anmeldenummer: PCT/EP2002/002722
(87) Internationale Veröffentlichungsnummer: WO 2002/072585

(56) Entgegenhaltungen:
- EP-A- 0 398 283
- FR-A- 2 722 786
- US-A- 5 426 106
- US-A- 5 602 144
- US-A- 5 789 406
- KROGSGAARD-LARSEN P ET AL: "Design of excitatory amino acid receptor agonists, partial agonists and antagonists: ibotenic acid as a key lead structure" EUROPEAN JOURNAL OF MEDICINAL CHEMISTRY, EDITIONS SCIENTIFIQUE ELSEVIER, PARIS, FR, Bd. 31, Nr. 7, 1996, Seiten 515-537, XP004040229 ISSN: 0223-5234

## Beschreibung

Die vorliegende Anmeldung betrifft substituierte Pyrazolopyrimidine, Verfahren zu ihrer Herstellung, Substanzbibliotheken, Arzneimittel, die diese Verbindungen enthalten, die Verwendung dieser Verbindungen zur Herstellung von Arzneimitteln zur Behandlung und/oder Prophylaxe von Schmerz, Epilepsie, Schizophrenie, neurodegenerativen Erkrankungen, insbesondere Morbus Alzheimer, Morbus Huntington und Morbus Parkinson, von cerebralen Ischämien und Infarkten, von Psychosen bedingt durch erhöhten Aminosäurespiegel, Himödemen, Unterversorgungszuständen des zentralen Nervensystems, insbesondere bei Hypoxien; insbesondere Neugeborenen-Hypoxie, und Anoxien, von AIDS-Demens, von Encephalomyelitis, des Tourette-Syndroms, der perinatalen Asphyxie, bei Tinnitus, neuropathischem Schmerz, Atemwegserkrankungen, Krebs, kardialen Arrythmien, Immunstörungen und -erkrankungen, Entzündungszuständen und - erkrankungen, neurodegenerativen Erkrankungen, Morbus Parkinson, Nierenversagen, Schizophrenie, Schlafstörungen, Schlaganfall, Thrombosen, Haminkontinenz, Diabetes, Psoriasis, septischem Schock, Gehimtraumata, Glaukom und/oder Stauungsinsuffizienz, sowie diese Verbindungen enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist

Klassische Opiolde wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.
Opioide entfalten ihre analgetische Wirkung durch Bindung an zellmebranständige Rezeptoren, die zu der Familie der so genannten G-Proteingekoppelten Rezeptoren gehören. Neben diesen gibt es weitere Rezeptoren sowie Ionenkanäle, die wesentlich an dem System der Schmerzentstehung und der Schmerzweiterleitung beteiligt sind, beispielsweise der N-Methyl-D-Aspartat-Ionenkanal (NMDA-Ionenkanal), über den ein wesentlicher Teil der Kommunikation von Synapsen abläuft und durch den der Calcium-lonenaustausch zwischen einer neuronalen Zelle und ihrer Umgebung gesteuert wird (s. zB. P.D. Leeson, L. L Iversen, *J. Med. Chem.* 37 (1994) 4053-4067).

Wichtige Erkenntnisse über die physiologische Bedeutung von ionenkanaiselektiven Substanzen sind durch die Entwicklung der "patch-clamp"-Technik ermöglicht worden, mit deren Hilfe sich die Wirkung von NMDA-Antagonisten (d.h. Antagonisten des NMDA-Ionenkanals) auf den Calciumhaushalt im Zellinneren nachweisen läßt.

Im nichtaktivierten Zustand sind die NMDA-Ionenkanäle jeweils durch einzelne Magnesiumionen verschlossen, die sich im Inneren des Kanals befinden und diesen aufgrund ihrer Größe nicht passieren können. Im aktivierten Zustand können die kleineren Calcium- und Natriumionen den Kanal passieren. Die (+)-MK801-Bindungsstelle des NMDA-Ionenkanals (ionotroper NMDA-Rezeptor) befindet sich ebenfalls im Inneren dieses Membranproteins. Substanzen mit NMDA-antagonistischer Wirkung, wie Phencyclidin (PCP), Ketamin oder MK801, besetzen diese Bindungsstelle (sogenannte "Channelblocker") und verschließen daher den betreffenden NMDA-lonenkanal.

Der vorliegenden Erfindung liegt als eine Aufgabe zugrunde, analgetisch wirksame Verbindungen zur Verfügung zu stellen, die sich zur Schmerztherapie - ggf. auch zur Therapie chronischer und neuropathischer Schmerzen - eignen. Darüber hinaus sollten diese Substanzen möglichst keine der Nebenwirkungen, die üblicherweise bei der Anwendung von Opioiden wie Morphin auftreten, wie z.B. Übelkeit, Erbrechen, Abhängigkeit, Atemdepression oder Obstipation, hervorrufen.

Die US 5,789,406 offenbart Indeno[1,2-E]pyrazin-4-on-Verbindungen mit antagonistischer Wirkung für den NMDA-Rezeptor und deren Verwendung in Arzneimitteln.

Aus der US 5,426,106 sind Pyrrolo-pyridazinon-Derivate und deren Verwendung als NMDA-Rezeptor-Antagonisten in Arzneimitteln bekannt.

Der FR 2 722 786 sind Derivate von 4-Hydroxy-3-Phenyl-indeno-[1,2-B]pyridin-2(1 H)-on sowie Arzneimittel auf Basis dieser Verbindungen zu entnehmen.

Diese Aufgabe wird durch die Verbindungen der allgemeinen Struktur (I A) bzw. (I B) gelöst, die analgetisch wirksam sind und an die MK801-Bindungsstelle des NMDA-Rezeptors binden. Bei den erfindungsgemäßen Verbindungen handelt es sich um substituierte Pyrazolopyrimidine der allgemeinen Struktur (I A) bzw. (I B). 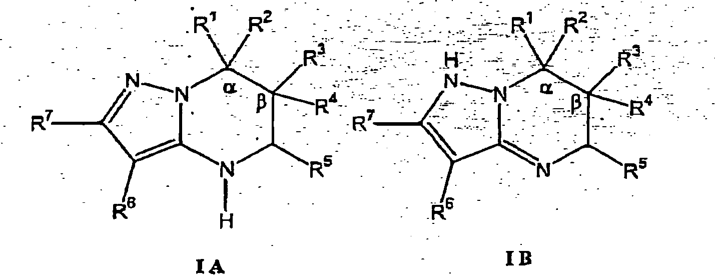 worin
- R¹ und R²: unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
- R³ und R⁴: H, Aryl', -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴
W bildet, wobei W α'-CH=CH-CH₂-β', α'-CH=CH-CH₂₋CH₂-β',α'-O-(CH₂)ₘ-β' mit m = 2, 3, 4 oder 5, 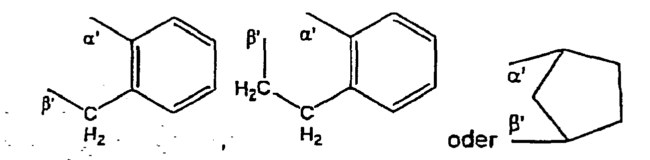 bedeutet, das mit α' gekennzeichnete Ende von W mit dem mit a gekennzeichneten Atom der Verbindung der allgemeinen Struktur (I A) oder (I B) verbunden ist und das mit β' gekennzeichnete Ende von W mit dem mit β gekennzeichneten Atom der Verbindung der allgemeinen Struktur (I A) oder (I B) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
- R⁵: Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NH₂, -NO₂, -SH, -OH, -CO₂H und -CO₂Alkyl, Aryl' oder
-(CH₂)ₖ-Aryl', wobei k = 1,2,3 oder 4 ist, Heterocyclyl oder C(=O)R¹¹ bedeutet;
- R⁶: H, Methyl, Ethyl, -CN, Fluor, Chlor, Brom, Iod, -C(=O)R¹⁷ oder -N=N-Aryl¹ bedeutet;
- R⁷: H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
- R⁹: unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
- R¹⁰: Aryl' bedeutet;
- R¹¹: Aryl' oder OR²⁵ bedeutet;
- R¹⁷: OR²⁶ bedeutet;
- R¹⁸: H oder Methyl bedeutet;
- R¹⁹: H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
- R²⁵: H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
- R²⁶: H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet; und
- Heterocyclyl: Furan-2-yl, Furan-3-yl, Thien-2-yl, Thien-3-yl, Pyridin-2-yl-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
- Aryl': Aryl¹, Aryl² oder Aryl³ bedeutet;
- Aryl¹: für 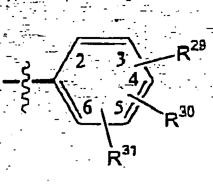 steht;
- Aryl²: für 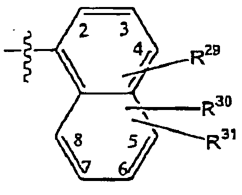 steht;
- Aryl³: für 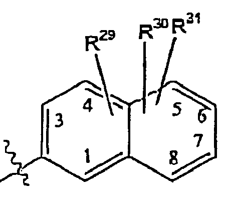 steht;
- R²⁹, R³⁰ und R³¹: unabhängig voneinander H, C₁₋₆-[Alk], C₃₋₈₋[Cycloalk], -(C₁₋₆-Alkyl)-C₃₋₈-[Cycloalk], [Ar], -(C₁₋₆₋Alkyl)-[Ar]. [Het], -(C₁₋₆-Alkyl)-[Het], F, Cl, Br, I, -CN,-NC, -OR³², -SR³³, -NO, -NO₂, NH₂, NHR³⁴, NR³⁵R³⁶,-N-OH. -N-OC₁₋₆-[Alk], -NHNH₂, -N=N-[Ar], -(C=O)R³⁷, 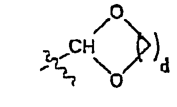 mit d = 1, 2 oder 3, oder -(C=S)R³⁷ bedeuten und in jeder beliebigen Ringposition sein können;
- R³² und R³³: unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
- R³⁴: C₁₋₆-[Alk], -CH₂-[Ar] oder -(C=O)O-tert.-Butyl bedeutet;
- R³⁵ und R³⁶: unabhängig voneinander C₁₋₆-[Alk] bedeuten oder gemeinsam für -(CH₂)_{g}- mit g = 4 oder 5 stehen;
- R³⁷: H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
- R³⁸: H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar] bedeutet;
und
- R³⁹: H, C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het] oder -(C₁₋₆-Alkyl)-[Het] bedeutet,

[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;

[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;

[Ar] für einen unsubstituierten Rest ausgewählt aus der Gruppe bestehend aus Phenyl, Naphthyl, Anthracenyl und Biphenyl steht;
und
[Het] für einen unsubstituierten Heterocyclyl Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht.

Die erfindungsgemäßen Verbindungen der allgemeinen Struktur (I A) bzw. (IB) in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate; in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; vorliegen. Die erfindungsgemäßen Pyrazolopyrimidine (I) können in tautomeren Formen (I A) und (I B) vorliegen, wobei die ggf. bevorzugte tautomere Form von Verbindung zu Verbindung und z.B. in Abhängigkeit vom Aggregatzustand oder vom gewählten Lösungsmittel variieren kann.

Folgende Verbindungen der allgemeinen Struktur (I A) oder (I B) sind im Stand der Technik bereits bekannt, ohne daß deren Verwendung in einem Arzneimittel oder zur Herstellung eines Arzneimittels zur Therapie und/oder Prophylaxe von Schmerz, Epilepsie, Schizophrenie, neurodegenerativen Erkrankungen, insbesondere Morbus Alzheimer, Morbus Huntington und Morbus Parkinson, cerebralen Ischämien und Infarkten, Psychosen bedingt durch erhöhten Aminosäurespiegel, Himödemen, Unterversorgungszuständen des zentralen Nervensystems, insbesondere bei Hypoxien und Anoxien, der AIDS-Demens, der Encephalomyelitis, des Tourette-Syndroms, der perinatalen Asphyxie und bei Tinnitus beschrieben wird:
4,5,6,7-Tetrahydro-2-methyl-5,7-diphenyl-pyrazolo[1,5-a]pyrimidin, 4,5,6,7-Tetrahydro-2,5-dimethyl-7-phanyl-pyrazolo[1,5-a]pyrimidin, 4,5,6,7-Tetrahydro-5,7-dimethyl-3-phenyl-pyrazolo[1,5-a]pyrimidin, 4,5,6,7-Tetrahydro-2,5,7-trimethyl-pyrazolo[1,5-a]pyrimidin, 4,5,6,7-Tetrahydro-5,7-dimethyl-2-phenyl-pyrazolo[1,5-a]pyrimidin (B. Koren et al., Tetrahedron (1976) 32, 493-497);
4,5,6,7-Tetrahydro-2-methyl-5,7-di-n-propyl-pyrazolo[1,5-]pyrimidin-3-carbonitril,4,5,6,7-Tetrahydro-5-methyl-7-[3-(trifluormethyl)-phenyl]pyrazolo[1,5-a]pyrimidin-3-carbonitril, 7-[4-(Chlor)-phenyl]-4,5,6,7-tetrahydro-5-methyl-pyrazolo[1,5-a]pyrimidin-3-carbonitril, 7-[3-(Chlor)-phenyl]-4,5,6,7-tetrahydro-5-methyl-pyrazolo[1,5-a]pyrimidin-3-carbonitril (EP 0 264 773 A1).

Diese Verbindungen sind daher insoweit ebenfalls Gegenstand der vorliegenden Erfindung, als erfindungsgemäße Verfahren zu ihrer Herstellung, Arzneimittel sowie ihre Verwendung zur Herstellung von Medikamenten zur Behandlung und/oder Prophylaxe von Schmerz, Epilepsie, Schizophrenie, neurodegenerativen Erkrankungen, insbesondere Morbus Alzheimer, Morbus Huntington und Morbus Parkinson, cerebralen Ischämien und Infarkten, Psychosen bedingt durch erhöhten Aminosäurespiegel, Himödemen, Unterversorgungszuständen des zentralen Nervensystems, insbesondere bei Hypoxien und Anoxien, der AIDS-Demens, der Encephalomyelitis, des Tourette-Syndroms, der perinatalen Aspftyxie und bei Tinnitus sowie von weiteren, in dieser Offenbarung genannten Krankheitszuständen betroffen sind.

Die Begriffe "Alkyl", "C₁₋₆-[Alk]" bzw, "C₁₋₆-Alkyl" umfassen im Sinne dieser Erfindung acyclische gesättigte oder ungesättigte Kohlenwasserstoffreste, die verzweigt oder geradkettig sowie unsubstituiert oder einfach substituiert oder mehrfach gleich oder verschieden substituiert sein können, mit (wie im Fall von C₁₋₆-Alkyl) 1 bis 6 (d.h. 1, 2, 3, 4, 5 oder 6) C-Atomen, d.h. C₁₋₆-Alkanyle, C₂₋₆-Alkenyle und C₂₋₆-Alkinyle. Dabei weisen "Alkenyle" mindestens eine C-C-Doppelbindung und "Alkinyle" mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sek-Butyl, tert-Butyl, n-Pentyl, iso-Pentyl, neoPentyl, n-Hexyl,- 2-Hexyl, n-Heptyl, n-Octyl, n-Nonyl, n-Decyl, n-Dodecyl; Ethenyl (Vinyl), Ethinyl, Propenyl (-CH₂CH=CH₂, =CH=CH-CH₃, -C(=CH₂)-CH₃), Propinyl (-CH₂-C≡CH, -C≡C-CH₃), Butenyl, Butinyl, Pentanyl, Pentinyl, Hexenyl, Hexinyl, Octenyl und Octinyl umfaßt.

"C₃₋₈-[Cycloalk]" bedeutet im Sinne dieser Erfindung einen cyclischen gesättigten oder ungesättigten Kohlenwasserstoff-Rest mit 3, 4, 5, 6, 7 oder 8 C-Atomen, wobei der Rest unsubstituiert oder einfach substituiert oder mehrfach gleich oder verschieden substituiert und ggf. benzokondensiert sein kann. Beispielhaft steht Cycloalkyl für Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, Für die Zwecke der vorliegenden Erfindung besonders bevorzugt sind Cyclopropyl, Cyclopropyl-2-carbonsäure, Cyclopropyl-2-carbonsäureethylester und Cyclohexyl.

Die Ausdrücke "(C₁₋₆-Alkyl)-C₃₋₈-[Cycloalk]", "(C₁₋₆-Alkyl)-[Het]" und "(C₁₋₆-Alkyl)-[Ar]" bedeuten für die Zwecke der vorliegenden Erfindung, daß der [Cycloalk]-, [Het] bzw.[An]-Rest über eine C₁₋₆-Alkyl-Gruppe an die mit ihm substituierte Verbindung gebunden ist. Entsprechendes gilt für den Ausdruck "CH₂-C₃₋₈-[Cycloalk]yl".

Im Zusammenhang mit "[Alk]", "Alkanyl", "Alkenyl", "Alkinyl" und "[Cycloalk]" versteht man unter dem Begriff "substituiert" im Sinne dieser Erfindung die Substitution eines Wasserstoffatoms durch beispielsweise F. Cl, Br, I, -CN, -NC, NH₂, NO₂, SH, OH, CO₂H, CO₂-Alkyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Besonders bevorzugt für die Zwecke der vorliegenden Erfindung sind CF₃ und CH₂₋CH₂-OH als substituiertes Alkyl sowie Cydopropyl-2-carbonsäure und Cyclopropyl-2-carbonsäureethylester als substituiertes Cycloalkyl.

In Bezug auf "Heterocyclyl" versteht man im Sinne dieser Erfindung unter "einfach substituiert" oder "mehrfach substituiert" die ein- oder mehrfache, z.B. zwei-, drei- oder vierfache, Substitution eines oder mehrerer Wasserstoffatome des Ringsystems durch einen geeigneten Substituenten. Soweit die Bedeutung dieser geeigneten Substituenten im Zusammenhang mit "Heterocyclyl" nicht an anderer Stelle der Beschreibung oder in den Ansprüchen definiert ist, sind als geeignete Substituenten F, Cl, Br, l, -CN, NO₂, CO₂H, CO₂-Alkyl, an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

"Benzokondensiert" bedeutet für die Zwecke der vorliegenden Erfindung, daß ein Benzol-Ring an einen anderen Cyclus ankondensiert ist.

Pharmazeutisch annehmbare bzw, physiologisch verträgliche Salze im Sinne dieser Erfindung sind solche Salze der erfindungsgemäßen

Verbindungen gemäß der allgemeinen Struktur (I A) bzw. (I B), die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren oder für den Fall, daß die erfindungsgemäßen Verbindungen Carbonsäuren sind, mit Basen gebildet werden.

Vorzugsweise werden die pharmazeutisch annehmbaren Salze der erfindungsgemäßen Verbindungen gemäß der allgemeinen Struktur (I A) oder (I B) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bemsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Carbonsäuren, können die pharmazeutisch annehmbaren Salze auch durch Umsetzung mit Basen, wie z.B. Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um NatriumSalze. Besonders bevorzugt sind die Hydrochloride. Ebenfalls bevorzugt sind die Hydrate der erfindungsgemäßen Verbindungen, die z.B. durch Kristallisation aus wäßriger Lösung erhalten werden können.

Alle efindungsgemäßen Verbindungen enthalten mindestens ein Asymmetriezentrum, nämlich das mit R⁵ substituierte Kohlenstoffatom der Struktur (I A) bzw. (I B). Daher können die erfindungsgemäßen Verbindungen der allgemeinen Struktur (I A) bzw. (I B) in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Bevorzugt liegen die Verbindungen der allgemeinen Struktur (I A) bzw. (I B) als enantiomerenreine Verbindungen vor.

Unter diesen Verbindungen sind solche insbesondere bevorzugt, bei denen
- R¹ und R²: unabhängig voneinander H, O-R⁹, S-R¹⁰, unsubstituiertes oder einfach substituiertes oder mehrfach gleich oder verschieden substituiertes Methyl, Ethyl, n-Propyl, 2-Propyl, n-Butyl, tart.-Butyl oder n-Hexyl, wobei die Substituenten ausgewählt sind aus der Gruppe bestehend aus F, Cl, Br, I, -CN, -NC, -NH₂, -NO₂, -SH und -OH; Aryl' oder-CH₂-Aryl', wobei die Aryl'-Substituenten R²⁹, R³⁰ und R³¹ unabhängig voneinander H, Methyl, Ethyl, 2-Propyl, n-Butyl, tert.-Butyl, n-Hexyl, F, Cl, Br, I, OH, O-Methyl, O-Ethyl sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder Methyl, Ethyl, n-Propyl, 2-Propyl, n-Butyl, tert.-Butyl oder n-Hexyl bedeutet,
- R³ und R⁴: H, Methyl oder Aryl¹ bedeutet, wobei die Aryl¹⁻Substiuenten R²⁹, R³⁰ und R³¹ unabhängig voneinander H, Methyl oder O-Methyl sind,
wobei mindestens einer der Reste R³ und R⁴ H ist,
- oder: einer der Reste R¹ und R² zusammen mit einem der Reste R³ und R⁴
W bildet, wobei W α'-CH=CH-CH₂-β', α'-CH=CH-CH₂₋CH₂-β'. α'-O-(CH₂)ₘ-β' mit m = 2, 3, 4 oder 5, 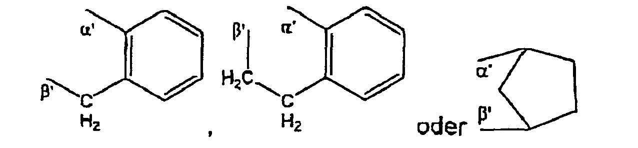 bedeutet, das mit α' gekennzeichnete Ende von W mit dem mit α gekennzeichneten Atom der Verbindung der allgemeinen Struktur (I A) oder (I B) verbunden ist und das mit β' gekennzeichnete Ende von W mit dem mit β gekennzeichneten Atom der Verbindung der allgemeinen Struktur (I A) oder (I B) verbunden ist, der andere Rest von R¹ und R² und der andere Rest von R³ und R⁴ jeweils H bedeuten;
- R⁵: Methyl, Ethyl, n-Propyl, 2-Propyl, n-Butyl, iso-Butyl, sek.-Butyl, tert.-Butyl, -(CH₂)₄-OH, Cyclopropyl, wobei Cyclopropyl unsubstituiert oder einfach mit C(=O)OH, C(=O)O-Methyl oder C(=O)O-Ethyl substituiert ist, Cyclopentyl, Cyclohexyl, Aryl¹ oder -(CH₂)ₖ-Aryl¹, wobei die Aryl¹-Substituenten R²⁹, R³⁰ und R³¹ unabhängig voneinander H, -OH, -O-Methyl, O-C₆H₅, CH₃, CF₃ oder C(=O)OH sind und k = 1 oder 2 ist, Hoterocyclyl oder C(=O)R¹¹ bedeutet,
- R⁶: H, -CN, Brom, -C(=O)R¹⁷ oder -N=N-Phenyl bedeutet;
- R⁷: H, Aryl¹ mit R²⁹, R³⁰ und R³¹ gleich H, OH, S(O)_{q}R¹⁹, wobei q = 0 oder 2 ist, oder Methyl, Ethyl, n-Propyl, 2-Propyl, n-Butyl, iso-Butyl, sek.-Butyl oder tert.-Butyl bedeutet.
- R⁹: Methyl, Ethyl, n-Propyl, 2-Propyl, n-Eutyl, iso-Butyl, sek.-Butyl, tert.-Buiyl, n-Amyl, iso-Amyl, sek.-Amyl, n-Hexyl, iso-Hexyl, sek.-Hexyl, Cyclopropyl, Cyclopentyl oder Cyclohexyl bedeutet oder -[(CH₂)ᵣ-O]ₛ-H mit r = 1, 2 oder 3 und s = 1 oder 2 bedeutet;
- R¹⁰: Aryl¹ bedeutet;
- R¹¹: Aryl¹ mit R²⁹, R³⁰ und R³¹ gleich H oder OR²⁵ bedeutet;
- R¹⁷: OR²⁶ bedeutet;
- R¹⁹: Methyl oder Aryl¹ bedeutet, wobei einer der Aryl¹⁻Substiuenten R²⁹, R³⁰ und R³¹ gleich H oder -NO₂ ist und die beiden anderen Aryl¹-Substituenten von R²⁹, R³⁰ und R³¹ H sind,
- R²⁵: H, Methyl oder Ethyl bedeutet, wobei R²⁵ nicht H bedeutet, wenn zugleich R¹ Aryl und R² Alkyl bedeuten;
- R²⁶: H, Methyl oder Ethyl bedeutet; und
- Heterocyclyl: Furan-2-yl, Furan-3-yl, Thien-2-yl, Thien-3-yl, Pyridin-2-y-, Pyridin-3-yl oder Pyridin-4-yl bedeutet, wobei Furanyl, Thienyl und Pyridinyl jeweils unsubstituiert oder einfach substituiert oder mehrfach gleich oder verschieden mit -NO₂, -CH₃ oder C(=O)OH substituiert sind.

Ganz besonders bevorzugte erfindungsgemäße Verbindungen der allgemeinen Struktur (I A) bzw. (I B) sind solche, in denen
- R¹ und R²: unabhängig voneinander H, O-CH₂-CH₂-OH, O-Cyclohexyl, S-Phenyl, Methyl, Phenyl, 3-Fluor-phenyl, 3-Brom-phenyl, 4-Brom-phenyl, 4-Chlor-phenyl, 4-Fluor-phenyl, 3-Methyl-phenyl, 4-Hydroxy-phenyl, 4-Methoxy-phenyl, 2,4-Dimethyl-phenyl, 3,4-Dimethoxy-phenyl, 2,3,4-Trimethoxyphenyl, 2-Naphthyl oder -CH₂₋Phenyl bedeuten,
- R³ und R⁴: H, Methyl oder 4-Methoxy-phenyl bedeuten, wobei mindestens einer der Reste R³ und R⁴ H ist, oder
- einer der Reste R¹ und R²: zusammen mit einem der Reste R³ und R⁴ W bildet, wobei W α'-CH=CH-CH₂-β', α'-CH=CH-CH₂₋CH₂-β', α'-O-(CH₂)ₘ-β' mit m = 2, 3, 4 oder 5, 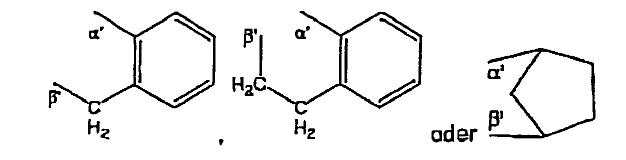 bedeutet, das mit α' gekennzeichnete Ende von W mit dem mit α gekennzeichneten Atom der Verbindung der allgemeinen Struktur (I A) oder (I B) verbunden ist und das mit β' gekennzeichnete Ende von W mit dem mit β gekennzeichneten Atom der Verbindung der allgemeinen Struktur (I A) oder (I B) verbunden ist, der andere Rest von R¹ und R² und der andere Rest von R³ und R⁴ jeweils H bedeuten;
- R⁵: n-Propyl, n-Butyl, tert,-Butyl, -(CH₂)₄-OH, Cyclopropyl, Cycloprop-2-yl-1-carbonsäureethylester, Cyclohexyl, 4-Trifluorphenyl, 4-Phenoxy-phenyl, 2-Hydroxy-3-methoxy-phenyl, 4-Hydroxy-3-methoxy-phenyl, 3-Carboxy-2-hydroxy-phenyl, -(CH₂)₂-Phenyl, 5-Carboxy-furan-2-yl, 5-Methyl-furan-2-yl, 5-Nitro-furan-2-yl, 5-Nitro-thien-2-yl, Pyridin-2-yl, Pyridin-3-yl, C(=O)Phenyl, C(=O)OH oder C(=O)OEthyl bedeutet, wobei R⁵ nicht C(=O)OH-bedeutet, wenn zugleich R¹ Aryl und R² Alkyl
- R⁶: H, -CN, Brom, -C(=O)OH, -C(=O)OEthyl oder -N=N-Phenyl bedeutet;
- R⁷: H, Phenyl, OH, -S-Methyl, -SO₂-(4-nitrophenyl) oder tert-Butyl bedeutet,

Beispielhafte und vorteilhafte Verbindungen der vorliegenden Erfindung sind aus der Gruppe ausgewählt, die
- 3-Brom-5-(5-nitro-furan-2-yl)-7-m-tolyl-tetrahydro-pyrazolo[1,5-a]pyrimidin
- 3-Brom-7-(4-fluor-phenyl)-7-methyl-5-(5-nitro-furan-2-yl)-tetrahydro-pyrazolo[1,5-a]pyrimidin
- 3-Brom-7-naphthalin-2-yl-5-(5-nitro-furan-2-yl)-tetrahydropyrazolo[1,5-a]pyrimidin
- 2-(3-Brom-7-m-tolyl-tetrahydro-pyrazolo[1,5-a]pyrimidin-5-yl)-cyclopropancarbonsäureethylester
- 2-[3-Brom-7-(4-brom-phenyl)-tetrahydro-pyrazolo[1,5-a]pyrimidin-5-yl]-cyclopropancarbonsäureethylester
- 2-(3-Brom-7-naphthalin-2-yl-tetrahydro-pyrazolo[1,5-a]pyrimidin-5-yl)-cyclopropancarbonsäureethylester
- 3-Brom-7-(4-fluor-phenyl)-7-methyl-5-(5-methyl-furan-2-yl)-tetrahydro-pyrazolo[1,5-a]pyrimidin
- 3-Brom-7-(3,4-dimethoxy-phenyl)-tetrahydro-pyrazolo[1,5-a]pyrimidin-5-carbonsäureethylester
- 3-Brom-7-(4-methoxy-phenyl)-tetrahydro-pyrazolo[1,5-a]pyrimidin-5-carbonsäureethylester
- 3-Brom-7-(4-methoxy-phenyl)-tetrahydro-pyrazolo[1,5-a]pyrimidin-5-carbonsäure
- 3-Brom-7-(2,4-dimethyl-phenyl)-5-(5-nitro-furan-2-yl)-tetrahydropyrazolo[1,5-a]pyrimidin
- 3-Brom-7-(4-methoxy-phenyl)-5-(5-nitro-furan-2-yl)-tetrahydropyrazolo[1,5-a]pyrimidin
- 5,5a,6,8a-Tetrahydro-3H-1,4,8b-triaza-as-indacen-3,5-dicarbonsäurediethylester; 5,5a,6,8a-Tetrehydro-4H-1,4,8b-triaza-as-indacen-3,5-dicarbonsäurediethylester;
- 2-Hydroxy-3-phenylazo-5,5a,6,8a-tetrahydro-3H-1,4,8b-triaza-as-indacen-5-carbonsäureethylester; 2-Hydroxy-3-phenylazo-5,5a,6,8a-tetrahydro-4H-1,4,8b-triaza-as-indacen-5-carbonsäureethylester
- 2-tert-Butyl-5,5a,6,8a-tetrahydro-3H-1,4,8b-triaza-as-indacen-5-carbonsäureethylester; 2-tert-Butyl-5,5a,6,8a-tetrahydro-3H-1,4,8b-triaza-as-indacen-5-carbonsäureethylester
- 3-Brom-2-phenyl-5,5a,6,8a-tetrahydro-3H-1,4,8b-triaze-as-indacen-5-carbonsäureethylester; 3-Brom-2-phenyl-5,5a,6,8a-tetrahydro-4H-1,4,8b-triaza-as-indacen-5-carbonsäureethylester
- 7-(2,3,4-Trimethoxyphenyl)-tetrahydro-pyrazolo[1,5-a]pyrimidin-3,5-dicarbonsäurediethylester
- 3-Cyano-2-methylsulfanyl-7-(2,3,4-trimethoxy-phenyl)-tetrahydro-pyrazolo[1,5-a]pyrimidin-5-carbonsäureethylester
- 2-Hydroxy-7-(4-hydroxy-phenyl)-6-methyl-3-phenylazo-tetrahydro-pyrazolo[1,5-a]pyrimidin-5-carbonsäureethylester
- 3-Brom-7-(4-hydroxy-phenyl)-6-methyl-2-phenyl-tetrahydro-pyrazolo[1,5-a]pyrimidin-5-carbonsäureethylester
- 5,5a,6,10b-Tetrahydro-3H-1,4,10c-triaza-cyclopenta[c]fluoren-3,5-dicarbonsäurediethylester; 5,5a,6,10b-Tetrahydro-4H-1,4,10c-triaza-cyclopenta[c]fluoren-3,5-dicarbonsäurediethylester
- 2-Hydroxy-3-phenylazo-5,5a,6,10b-tetrahydro-3H-1,4,10c-triaza-cyclopenta[c]fluoren-5-carbonsäureethylester; 2-Hydroxy-3-phenylazo-5,5a,6,10b-tetrahydro-4H-1,4,10c-triaza-cyclopenta[c]fluoren-5-carbonsäureethylester
- 7-Phenylsulfanyl-tetrahydro-pyrazolo[1,5-a]pyrimidin-3,5-dicarbonsäurediethylester
- 3-Cyano-2-methylsulfanyl-7-phenylsulfanyl-tetrahydro-pyrazolo[1,5-a]pyrimidin-5-carbonsäureethylester
- 3-Cyano-2-methylsulfanyl-7-(2,3,4-trimethoxy-phenyl)-tetrahydro-pyrazolo[1,5-a]pyrimidin-5-carbonsäure
- 7-Phenylsulfanyl-tetrahydro-pyrazolo[1,5-a]pyrimidin-3,5-dicarbonsäure3-ethyl ester
- 3-Cyano-7-(2,4-dimethyl-phenyl)-2-methylsulfanyl-tetrahydro-pyrazolo[1,5-a]pyrimidin-5-carbonsäureethylester
- 3-Cyano-7-(2,4-dimethyl-phenyl)-tetrahydro-pyrazolo[1,5-a]pyrimidin-5-carbonsäureethylester
- 7-(2,4-Dimethyl-phenyl)-tetrahydro-pyrazolo[1,5-a]pyrimidin-3,5-dicarbonsäure3-ethyl ester
- 3-Brom-7-(2,4-dimethyl-phenyl)-2-phenyl-tetrahydro-pyrazolo[1,5-a]pyrimidin-5-carbonsäure
- 3-Cyano-7-(2,4-dimethyl-phenyl)-2-methylsulfanyl-tetrahydro-pyrazolo[1,5-a]pyrimidin-5-carbonsäure
- 3-Cyano-7-(2,4-dimethyl-phenyl)-tetrahydro-pyrazolo[1,5-a]pyrimidin-5-carbonsäure
- 3-Cyano-7-(3,4-dimethoxy-phenyl)-2-methylsulfanyl-tetrahydro-pyrazolo[1,5-a]pyrimidin-5-carbonsäure
- 7-(2,4-Dimethyl-phenyl)-5-(5-nitro-furan-2-yl)-tetrahydro-pyrazolo[1,5-a]pyrimidin-3-carbonsäureethylester
- 7-(2,4-Dimethyl-phenyl)-5-(5-nitro-furan-2-yl)-3-phenylazo-tetrahydro-pyrazolo[1,5-a]pyrimidin-2-ol
- 3-Brom-7-(2,4-dimethyl-phenyl)-5-(5-nitro-furan-2-yl)-2-phenyl-tetrahydro-pyrazolo[1,5-a]pyrimidin
- 7-(2,4-Dimethyl-phenyl)-2-methylsulfanyl-5-(5-nitro-furan-2-yl)-tetrahydro-pyrazolo[1,5-a]pyrimidin-3-carbonitril
- 7-(2,4-Dimethyl-phenyl)-5-(5-nitro-furan-2-yl)-tetrahydro-pyrazolo[1,5-a]pyrimidin-3-carbonitril
- 3-Brom-7-(3,4-dimethoxy-phenyl)-5-(5-nitro-furan-2-yl)-2-phenyl-tetrahydro-pyrazolo[1,5-a]pyrimidin
- 7-(4-Methoxy-phenyl)-2-msthylsulfanyl-5-(5-nitro-furan-2-yl)-tetrahydro-pyrazolo[1,5-a]pyrimidin-3-carbonitril
- 7 -(2,4-Dimethyl-phenyl)-5-(2-ethoxycarbonyl-cyclopropyl)-tetrahydro-pyrazolo[1,5-a]pyrimidin-3-carbonsäureethylester
- 2-[7-(2,4-Dimethyl-phenyl)-2-hydroxy-3-phenylazo-tetrahydro-pyrazolo[1,5-a]pyrimidin-5-yl]-cyclopropancarbonsäureethylester
- 2-[2-tert-Butyl-7-(2,4-dimethyl-phenyl)-tetrahydro-pyrazolo[1,5-a]pyrimidin-5-yl]-cyclopropancarbonsäureethylester
- 2-[3-Brom-7-(2,4-dimethyl-phenyl)-2-phenyl-tetrahydro-pyrazolo[1,5-a]pyrimidin-5-yl]-cyclopropancarbonsäureethylester
- 2-[3-Cyano-7-(2,4-dimethyl-phenyl)-2-methylsulfanyl-tetrahydro-pyrazolo[1,5-a]pyrimidin-5-yl]-cyclopropancarbonsäureethylester
- 5-(2-Ethoxycarbonyl-cyclopropyl)-7-(3-fluor-phenyl)-tetrahydro-pyrazolo[1,5-a]pyrimidin-3-carbonsäureethylester
- 2-[3-Brom-7-(3-brom-phenyl)-2-phenyl-tetrahydro-pyrazolo[1,5-a]pyrimidin-5-yl]-cyclopropancarbonsäureethylester
- 2-[7-(3-Brom-phenyl)-3-cyano-2-methylsulfanyl-tetrahydro-pyrazolo[1,5-a]pyrimidin-5-yl]-cyclopropancarbonsäureethylester
- 7-(2,4-Dimethyl-phenyl)-5-(5-nitro-thiophen-2-yl)-3-phenylazo-tetrahydro-pyrazolo[1,5-a]pyrimidin-2-ol
- 7-(2,4-Dimethyl-phenyl)-2-methylsulfanyl-5-(5-nitro-thiophen-2-yl)-tetrahydro-pyrazolo[1,5-a]pyrimidin-3-carbonitril
- 7-(2,4-Dimethyl-phenyl)-5-(5-nitro-thiophen-2-yl)-tetrahydro-pyrazolo[1,5-a]pyrimidin-3-carbonitril
- 7-(3,4-Dimethoxy-phenyl)-5-(5-nitro-thiophen-2-yl)-tetrahydro-pyrazolo[1,5-a]pyrimidin-3-carbonsäureethylester
- 7-(3,4-Dimethoxy-phenyl)-5-(5-nitro-thiophen-2-yl)-3-phenylazo-tetrahydro-pyrazolo[1,5-a]pyrimidin-2-ol
- 3-Brom-7-(3,4-dimethoxy-phenyl)-5-(5-nitro-thiophen-2-yl)-2-phenyl-tetrahydro-pyrazolo[1,5-a]pyrimidin
- 7-(3,4-Dimethoxy-phenyl)-2-methylsulfanyl-5-(5-nitro-thiophen-2-yl)-tetrahydro-pyrazolo[1,5-a]pyrimidin-3-carbonitril
- 7-(3,4-Dimethoxy-phenyl)-5-(5-nitro-thiophen-2-yl)-tetrahydro-pyrazolo[1,5-a]pyrimidin-3-carbonitril
- 7-(4-Methoxy-phenyl)-5-(5-nitro-thiophen-2-yl)-tetrahydro-pyrazolo[1,5-a]pyrimidin-3-carbonsäureethylester
- 5-[3-Brom-7-(4-methoxy-phenyl)-2-phenyl-tetrahydro-pyrazolo[1,5-a]pyrimidin-5-yl]-furan-2-carbonsäure
- 5-Benzoyl-7-(2,4-dimethyl-phenyl)-tetrahydro-pyrazolo[1,5-a]pyrimidin-3-carbonsäureethylester
- 5-Benzoyl-7-(2,4-dimethyl-phenyl)-2-methylsulfanyl-tetrahydro-pyrazolo[1,5-a]pyrimidin-3-carbonitril
- 5-Benzoyl-7-(2,4-dimethyl-phenyl)-tetrahydro-pyrazolo[1,5-a]pyrimidin-3-carbonitril
- 5-Benzoyl-7-(3,4-dimethoxy-phenyl)-tetrahydro-pyrazolo[1,5-a]pyrimidin-3-carbonsäureethylester
- [3-Brom-7-(3,4-dimethoxy-phenyl)-2-phenyl-tetrahydro-pyrazolo[1,5-a]pyrimidin-5-yl]-phenyl-methanon
- 5-Benzoyl-7-(3,4-dimethoxy-phenyl)-2-methylsulfanyl-tetrahydro-pyrazolo[1,5-a]pyrimidin-3-carbonitril
- 5-Benzoyl-7-(3,4-dimethoxy-phenyl)-tetrahydro-pyrazolo[1,5-a]pyrimidin-3-carbonitril
- 5-Benzoyl-7-(4-methoxy-phenyl)-tetrahydro-pyrazolo[1,5-a]pyrimidin-3-carbonsäureethylester
- 5-Benzoyl-7-(4-methoxy-phenyl)-2-mathylsulfanyl-tetrahydro-pyrazolo[1,5-a]pyrimidin-3-carbonitril
- 5-Benzoly-7-(4-methoxy-phenyl)-tetrahydro-pyrazolo[1,5-a]pyrimidin-3-carbonitril
- 5-Benzoyl-7-(3-fluor-phenyl)-tetrahydro-pyrazolo[1,5-a]pyrimidin-3-carbonsäureethylester
- [3-Brom-7-(3-fluor-phenyl)-2-phenyl-tetrahydro-pyrazolo[1,5-a]pyrimidin-5-yl]-phenyl-methanon
- [3-Brom-7-(3-brom-phenyl)-2-phenyl-tetrahydro-pyrazolo[1,5-a]pyrimidin-5-yl]-phenyl-methanon
- 7-(2,4-Dimethyl-phenyl)-5-(4-phenoxy-phenyl)-tetrahydro-pyrazolo[1,5-a]pyrimidin-3-carbonsäureethylester
- 3-Brom-7-(2,4-dimethyl-phenyl)-5-(4-phenoxy-phenyl)-2-phenyl-tetrahydro-pyrazolo[1,5-a]pyrimidin
- 7-(2,4-Dimethyl-phenyl)-2-methylsulfanyl-5-(4-phenoxy-phenyl)-tetrahydro-pyrazolo[1,5-a]pyrimidin-3-carbonitril
- 7-(2,4-Dimethyl-phenyl)-5-(4-phenoxy-phenyl)-tetrahydro-pyrazolo[1,5-a]pyrimidin-3-carbonitril
- 7-(3,4-Dimethoxy-phenyl)-5-(4-phenoxy-phenyl)-tetrahydro-pyrazolo[1,5-a]pyrimidin-3-carbonsäureethylester
- 7-(3,4-Dimethoxy-phenyl)-2-methylsulfanyl-5-(4-phenoxy-phenyl)-tetrahydro-pyrazolo[1,5-a]pyrimidin-3-carbonitril
- 3-[3-Cyano-7-(4-hydroxy-phenyl)-6-methyl-tetrahydro-pyrazolo[1,5-a]pyrimidin-5-yl]-2-hydroxy-benzoesäure
- 3-(3-Cyano-5,5a,6,10b-tetrahydro-3H-1,4,10c-triaza-cyclopenta[c]fluoren-5-yl)-2-hydroxy-benzoesäure; 3-(3-Cyano-5,5a,6,10b-tetrahydro-4H-1,4,10c-triaza-cyclopenta[c]fluoren-5-yl)-2-hydroxy-benzoesäure
- 3-(3-Cyano-7-phenylsulfanyl-tetrahydro-pyrazolo[1,5-a]pyrimidin-5-yl)-2-hydroxy-benzoesäure
- 3-[2-tert-Butyl-7-(4-chlor-phenyl)-7-methyl-tetrahydro-pyrazolo[1,5-a]pyrimidin-5-yl]-2-hydroxy-benzoesäure
- 5-(4-Hydroxy-3-methoxy-phenyl)-7-(4-hydroxy-phenyl)-6-methyl-tetrahydro-pyrazolo[1,5-a]pyrimidin-3-carbonsäureethylester
- 5-(4-Hydroxy-3-methoxy-phenyl)-7-(4-hydroxy-phenyl)-6-methyl-tetrahydro-pyrazolo[1,5-a]pyrimidin-3-carbonitril
- 5-(4-Hydroxy-3-methoxy-phenyl)-5,5a,6,10b-tetrahydro-3H-1,4,10c-triaza-cyclopenta[c]fluoren-3-carbonsäureethylester; 5-(4-Hydroxy-3-methoxy-phenyl)-5,5a,6,10b-tetrahydro-4H-1,4,10c-triaza-cyclopenta[c]fluoren-3-cartionsäureethylester
- 4-(2-tert-Butyl-5,5a,5,6,10b-tetrahydro-3H-1,4,10c-triaza-cyclopenta[c]fluoren-5-yl)-2-methoxy-phenol; 4-(2-tert-Butyl-5,5a,6,1 0b-tetrahydro-4H-1,4,10c-triaza-cyclopenta[c]fluoren-5-yl)-2-methoxy-phenol
- 5-(4-Hydroxy-3-methoxy-phehyl)-2-methylsulfanyl-5,5a,6,10b-tetrahydro-3H-1,4,10c-triaza-cyclopenta[c]fluoren-3-carbonitril; 5-(4-Hydroxy-3-methoxy-phenyl)-2-methylsulfanyl-5,5a,6,10b-tetrahydro-3H-1,4,10c-triaza-cyclopenta[c]fluoren-3-carbonitril
- 5-(4-Hydroxy-3-methoxy-phenyl)-7-phenylsulfanyl-tetrahydro-pyrazolo[1,5-a]pyrimidin-3-carbonsäureethylester
- 4-(2-tert-Butyl-7-phenylsulfanyl-tetrahydro-pyrazolo[1,5-a]pyrimidin-5-yl)-2-methoxy-phenol
- 4-(3-Brom-2-phenyl-7-phenylsulfanyl-tetrahydro-pyrazolo[1,5-a]pyrimidin-5-yl)-2-methoxy-phenol
- 5-(2-Hydroxy-3-methoxy-phenyl)-7-phenylsulfanyl-tetrahydro-pyrazolo[1,5-a]pyrimidin-3-carbonsäureethylester
- 7-(4-Chlor-phenyl)-5-(2-hydroxy-3-methoxy-phenyl)-7-methyl-tetrahydro-pyrazolo[1,5-a]pyrimidin-3-carbonsäureethylester
- 5-(4-Hydroxy-butyl)-5,5a,6,10b-tetrahydro-3H-1,4,10c-triaza-cyclopenta[c]fluoren-3-carbonitril; 5-(4-Hydroxy-butyl)-5,5a,6,10b-tetrahydro-4H-1,4,10c-triaza-cyclopenta[c]fluoren-3-carbonitril
- 5-(4-Hydroxy-butyl)-2-methylsulfanyl-7-phenylsulfanyl-tetrahydro-pyrazolo[1,5-a]pyrimidin-3-carbonitril
- 5-(4-Hydroxy-butyl)-7-phenylsulfanyl-tetrahydro-pyrazolo[1,5-a]pyrimidin-3-carbonitril
- 7-(4-Chlor-phenyl)-5-(4-hydroxy-butyl)-7-methyl-tetrahydro-pyrazolo[1,5-a]pyrimidin-3-carbonitril
- 5-Butyl-2-methylsulfanyl-5,5a,6,10b-tetrahydro-3H-1,4,10c-triaza-cyclopenta[c]fluoren-3-carbonitril; 5-Butyl-2-methylsulfanyl-5,5a,6,10b-tetrahydro-4H-1,4,10c-triaza-cyclopenta[c]fluoren-3-carbonitril
- 5-Butyl-2-methylsulfanyl-7-phenylsulfanyl-tetrahydro-pyrazolo[1,5-a]pyrimidin-3-carbonitril
- 5-Butyl-7-phenylsulfanyl-tetrahydro-pyrazolo[1,5-a]pyrimidin-3-carbonitril
- 5-Butyl-7-(4-chlor-phenyl)-7-methyl-2-methylsulfanyl-tetrahydro-pyrazolo[1,5-a]pyrimidin-3-carbonitril.
- 5-Cyclopropyl-7-(2,4-dimethyl-phenyl)-3-phenylazo-tetrahydro-pyrazolo[1,5-a]pyrimidin-2-ol
- 2-tert-Butyl-5-cyclopropyl-7-(2,4-dimethyl-phenyl)-tetrahydro-pyrazolo[1,5-a]pyrimidin
- 5-Cyclopropyl-7-(2,4-dimethyl-phenyl)-2-methylsulfanyl-tetrahydro-pyrazolo[1,5-a]pyrimidin-3-carbonitril
- 2-tert-Butyl-5-cyclopropyl-7-(3,4-dimethoxy-phenyl)-tetrahydro-pyrazolo[1,5-a]pyrimidin
- 3-Brom-5-cyclopropyl-7-(3,4-dimethoxy-phenyl)-2-phenyl-tetrahydro-pyrazolo[1,5-a]pyrimidin
- 5-Cyclopropyl-7-(4-methoxy-phenyl)-tetrahydro-pyrazolo[1,5-a]pyrimidin-3-carbonsäureethylester
- 5-Cyclopropyl-3,5,5a,6,7,11b-hexahydro-1,4,11c-triaza-cyclopenta[c]phenanthrene-3-carbonitri
- 7-(2,4-Dimethyl-phenyl)-5-pyridin-2-yl-tetrahydro-pyrazolo[1,5-a]pyrimidin-3-carbonsäureethylester
- 7-(2,4-Dimethyl-phenyl)-3-phenylazo-5-pyridin-2-yl-tetrahydro-pyrazolo[1,5-a]pyrimidin-2-ol
- 3-Brom-7-(2,4-dimethyl-phenyl)-2-phenyl-5-pyridin-2-yl-tetrahydro-pyrazolo[1,5-a]pyrimidin
- 7-(2,4-Dimethyl-phenyl)-2-methylsulfanyl-5-pyridin-2-yl-tetrahydro-pyrazolo[1,5-a]pyrimidin-3-carbonitril
- 7-(3,4-Dimethoxy-phenyl)-2-methylsulfanyl-5-phenethyl-tetrahydro-pyrazolo[1,5-a]pyrimidin-3-carbonitril
- 7-(3,4-Dimethoxy-phenyl)-5-phenethyl-tetrahydro-pyrazolo[1,5-a]pyrimidin-3-carbonitrll
- 5-Cyclopropyl-7-(2-hydroxy-ethoxy)-tetrahydro-pyrazolo[1,5-a]pyrimidin-3-carbonsäureethylester
- 2-(2-tert-Butyl-5-cyclopropyl-tetrahydro-pyrazolo[1,5-a]pyrimidin-7-yloxy)-ethanol
- 5-Cyclopropyl-3,5,5a,6,7,8a-hexahydro-8-oxa-1,4,8b-triaza-as-indacen-3-carbonsäureethylester; 5-Cyclopropyl-4,5,5a, 6,7,8a-hexahydro-8-oxa-1,4,8b-triaza-as-indacen-3-carbonsäureethylester
- 5-Cyclopropyl-3-phenylazo-3,5,5a,6,7,8a-hexahydro-8-oxa-1,4,8b-triaza-as-indacen-2-ol; 5-Cyclopropyl-3-phonylazo-4,5,5a,6,7,8a-hexahydro-8-oxa-1,4,8b-triaza-as-indacen-2-ol
- 7-Cyclohexyloxy-5-cyclopropyl-tetrahydro-pyrazolo[1,5-a]pyrimidin-3-carbonsäureethylester
- 7-Cyclohexyloxy-5-cyclopropyl-2-methylsulfanyl-tetrahydro-pyrazolo[1,5-a]pyrimidin-3-carbonitril
- 7-(4-Chlor-phenyl)-5-cyclohexyl-tetrahydro-pyrazolo[1,5-a]pyrimidin-3-carbonitril
- 5-Cyclohexyl-7-(2-hydroxy-ethoxy)-tetrahydro-pyrazolo[1,5-a]pyrimidin-3-carbonsäureethylester
- 5-Cyclohexyl-3,5,5a,6,7,8a-hexahydro-8-oxa-1,4,8b-triaza-as-indacen-3-carbonsäureethylester; 5-Cyclohexyl-4,5,5a,6,7,8a-hexahydro-8-oxa-1,4,8b-triaza-as-indacen-3-carbonsäureethylester
- 5-Cyclohexyl-7-cyclohexyloxy-tetrahydro-pyrazolo[1,5-a]pyrimidin-3-carbonsäureethylester
- 7-(2,4-Dimethyl-phenyl)-3-phenylazo-5-propyl-tetrahydro-pyrazolo[1,5-a]pyrimidin-2-ol
- 7-(2,4-Dimethyl-phenyl)-2-methylsulfanyl-5-propyl-tetrahydro-pyrazolo[1,5-a]pyrimidin-3-carbonitril
- 5-tert-Butyl-7-(2,4-dimathyl-phenyl)-tetrahydro-pyrazolo[1,5-a]pyrimidin-3-carbonsäureethylester
- 2,5-Di-tert-butyl-7-(3,4-dimethoxy-phenyl)-tetrahydro-pyrazolo[1,5-a]pyrimidin
- 3-Brom-5-tert-butyl-7-(3,4-dimethoxy-phenyl)-2-phenyl-tetrahydro-pyrazolo[1,5-a]pyrimidin
- 2-[3-Cyano-6,7-bis-(4-methoxy-phenyl)-tetrahydro-pyrazolo[1,5-a]pyrimidin-5-yl]-cyclopropancarbonsäureethylester
- 3-Cyano-6,7-bis-(4-methoxy-phenyl)-tetrahydro-pyrazolo[1,5-a]pyrimidin-5-carbonsäure
- 4-[3-Brom-6-methyl-2-phenyl-5-(4-trifluromethyl-phenyl)-tetrahydro-pyrazolo[1,5-a]pyrimidin-7-yl]-phenol
- 7-(4-Hydruxy-phenyl)-6-methyl-2-methylsulfanyl-5-(4-trifluormethyl-phenyl)-tetrahydro-pyrazolo[1,5-a]pyrimidin-3-carbonitril
- 7-(4-Hydroxy-phenyl)-6-methyl-5-(4-trifluormethyl-phenyl)-tetrahydro-pyrazolo[1,5-a]pyrimidin-3-carbonitril
- 7-Phenyl-3-phenylazo-5-pyridin-2-yl-3,5,6,7-tetrahydro-pyrazolo[15-a]pyrimidin-2-ol
- 7-Phenylsulfanyl-5-pyridin-2-yl-3,5,6,7-tetrahydro-pyrazolo[1,5-a]pyrimidine-3-carbonsäureethylester
- 3-Phenylazo-7-phenylsulfanyl-5-pyridin-2-yl-3,5,6,7-tetrahydro-pyrazolo[1,5-a]pyrimidin-2-ol
- 3-Brom-7-phenylsulfanyl-5-pyridin-2-yl-3,5,6,7-tetrahydro-pyrazolo[15-a]pyrimidin
- 7-Phenylsulfanyl-5-pyridin-2-yl-3,5,6,7-tetrahydro-pyrazolo[1,5-a]pyrimidin-3-carbonitril
- 7-(3,4-Dimethoxy-phenyl)-2-methylsulfanyl-5-pyridin-2-yl-tetrahydro-pyrazolo[1,5-a]pyrimidin-3-carbonitril
- 3-Brom-7-(3,4-dimethoxy-phenyl)-2-phenyl-5-pyridin-2-yl-tetrahydro-pyrazolo[1,5-a]pyrimidin
- 3-Brom-7-(3,4-dimethoxy-phenyl)-2-phenyl-tetrahydro-pyrazolo[1,5-a]pyrimidin-5-carbonsäureethylester
- 3-Brom-7-(3,4-dimethoxy-phenyl)-5-(5-nitro-furan-2-yl)-2-phenyl-tetrahydro-pyrazolo[1,5-a]pyrimidin
- 3-Cyano-7-(3,4-dimethoxy-phenyl)-2-methylsulfanyl-tetrahydro-pyrazolo[1,5-a]pyrimidin-5-cartronsäureethylester
- 3-Cyano-7-(3,4-dimethoxy-phenyl)-tetrahydro-pyrazolo[1,5-a]pyrimidin-5-carbonsäureethylester
- 3-Brom-7-(3,4-dimethoxy-phenyl)-2-phenyl-5-pyridin-2-yl-4,5,6,7-tetrahydro-pyrazolo[1,5-a]pyrimidin
- 7-(3,4-Dimethoxy-phenyl)-5-pyridin-2-yl-4,5,6,7-tetrahydro-pyrazolo[1,5-a]pyrimidine-3-carbonitril
- 7-(3,4-Dimethoxy-phenyl)-2-methylsulfanyl-5-(5-nitro-furan-2-yl)-tetrahydro-pyrazolo[1,5-a]pyrimidine-3-carbonitril
- 7-(3,4-Dimethoxy-phenyl)-5-pyridin-2-yl-4,5,6,7-tetrahydro-pyrazolo[1,5-a]pyrimidine-3-carbonsäureethylester
umfaßt sowie ihre pharmazeutisch annehmbaren Salze.

Die Erfindung betrifft auch Verfahren zur Herstellung der erfindungsgemäßen Verbindungen der Struktur (I A), und (I B)

So sind die Verbindungen der allgemeinen Struktur (I A) und (I B) sowie ihre pharmazeutisch annehmbaren Salze 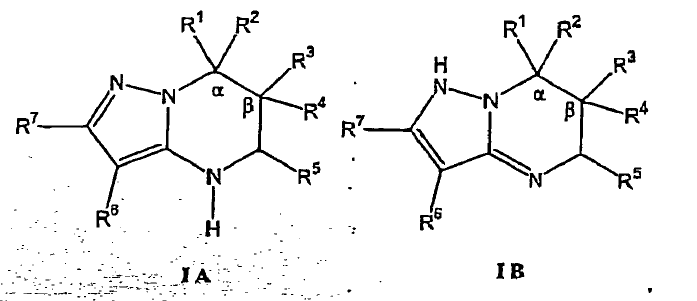 worin R¹, R², R³, R⁴, R⁵, R⁶ und R⁷ wie oben definiert sind, dadurch herstellbar, daß ein Pyrazolamin der allgemeinen Struktur (IIIA) und/oder (IIIB) 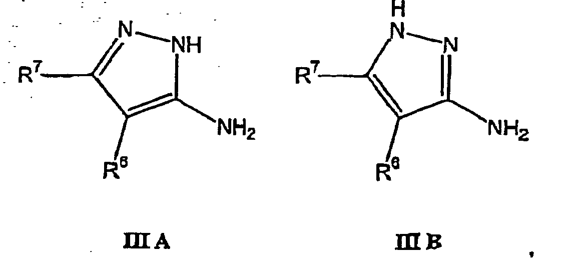 worin R⁶ und R⁷ wie oben definiert sind, mit einem Aldehyd der allgemeinen Struktur (IV) 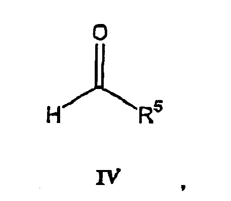 worin R⁵ wie oben definiert ist, und einem Olefin der allgemeinen Struktur (V) 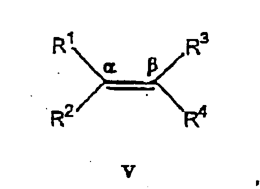 worin R¹, R², R³ und R⁴ wie oben definiert sind mit der Maßgabe, daß, wenn einer der Reste R¹ und R² zusammen mit einem der Reste R³ und R⁴ W bildet, das mit α' gekennzeichnete Ende von W mit dem α-Kohlenstoffatom des Olefins der allgemeinen Struktur (V) und das mit β' gekennzeichnete Ende von W mit dem β-Kohlenstoffatom des Olefins der allgemeinen Struktur (V) verbunden ist, in Gegenwart einer Säure umgesetzt wird.

Die erfindungsgemäßen Verfahren werden bevorzugt in einer "Eintopf'-Reaktion durchgeführt, bei der je ein Heterocyclylamin der allgemeinen Struktur (II A), (II B), je ein Aldehyd der allgemeinen Struktur (IV) und je ein Olefin der allgemeinen Struktur (V) gleichzeitig miteinander umgesetzt werden.

Bei der eingesetzten Säure handelt es sich um eine anorganische oder organische Protonen- oder Lewis-Säure. Bevorzugt wird die Reaktion in Gegenwart einer organischen Säure, z.B. Essigsäure, Trifluoressigsäure oder Methansulfonsäure, insbesondere Trifluoressigsäure, durchgeführt.

Das erfindungsgemäße Herstellungsverfahren kann in jedem geeigneten Lösungsmittel durchgeführt werden, in dem die Reaktanten sich ausreichend lösen. Bevorzugt sind als Lösungsmittel organische Solventien, z.B. Dichlormethan oder insbesondere Acetonitril.

Die erfindungsgemäßen Verfahren werden zweckmäßig bei einer Temperatur von 0 bis 100°C, insbesondere bei 15 bis 40°C durchgeführt. Die Reaktionszeit beträgt vorzugsweise 15 Minuten bis 12 Stunden und kann den jeweiligen Erfordernissen angepaßt werden.

Alle in den erfindungsgemäßen Verfahren eingesetzten Heterocyclylamine der allgemeinen Struktur (III) die Aldehyde der allgemeinen Struktur (IV) und die Olefine der allgemeinen Struktur (V) sind käuflich erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan) oder können nach im Stand der Technik allgemein bekannten Verfahren hergestellt werden.

Die erfindungsgemäßen Verfahren können auch in semi- oder vollautomatisierter Form als Parallelsynthese einer Gruppe von erfindungsgemäßen Verbindungen der allgemeinen Struktur (I A) und/oder (I B) durchgeführt werden. Somit sind auch Substanzbibliotheken zugänglion, die mindestens eine Verbindung und vorzugsweise mindestens 48, insbesondere 96 und ganz besonders bevorzugt 384 Verbindungen der allgemeinen und wie oben definierten Struktur (I A) oder (I B) enthalten.

Unter einer "Substanzbibliothek" wird dabei eine Gruppe von Verbindungen verstanden, die nach dem gleichen Verfahren unter gleichen oder nahezu gleichen Reaktionsbedingungen und unter Variation eines Reagenzes oder mehrerer Reagenzien hergestellt werden. Eine solche Substanzbibliothek kann die Bibliotheksmitglieder sowohl als einzelne reine Verbindungen als auch als Mischung dieser Verbindungen enthalten. Mit Hilfe dieser Substanzbibliothek kann beispielsweise ein medizinisches Screening in einem oder mehreren in-vitro-Screening-Verfahren in automatisierter Form durchgeführt werden.

Die Verbindungen der allgemeinen Struktur (IA) bzw. (IB) können sowohl in Substanz als auch als Salz isoliert werden. Die Substanzen der allgemeinen Struktur (IA) oder (IB) werden üblicherweise nach Umsetzung gemäß dem oben dargelegten Verfahren und anschließender herkömmlicher Aufarbeitung erhalten. Die so gewonnenen Verbindungen können dann beispielsweise durch Versetzen mit einer anorganischen oder organischen Säure, vorzugsweise mit Salzsäure, Bromwasserstoffsäure, Schwefelsäure, Phosphorsäure, Methansulfonsäure, p-Tolualsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Soweit es sich bei den Verbindungen der allgemeinen Formel (IA) bzw. (IB) um Säuren, insbesondere Carbonsäuren (z.B. wenn R⁵ = CO₂H), handelt, kann die Salzbildung durch Zugabe einer Base, z,B. Natriumhydroxid, NaHCO₃ oder Natriumcarbonat, herbeigeführt werden; für die (Carbon-)Säuren ist insbesondere die Bildung des Natriumsalzes bevorzugt. Die besonders bevorzugte Hydrochloridbildung kann insbesondere auch durch Versetzen der in einem geeigneten organischen Lösungsmittel gelösten Base (IA) bzw. (IB) mit Trimethylsilylchlorid (TMSCI) herbeigeführt werden. Die Bildung von Natriumsalzen kann z.B. durch Titrieren der in einem geeigenten Lösungsmittel, beispielsweise Wasser-Methanol-Mischung, gelösten Verbindung (IA), bzw. (IB) mit Natriumhydroxid-Lösung erfolgen.

Soweit die Verbindungen der allgemeinen Struktur (I A) bzw. (I B) in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, oder- sofern es sich um Säuren handelt - mit chiralen Basen, etwa Brucin oder (-)-Ephedrin, gebildeten diastereomeren Salzen voneinander getrennt werden.

Darüber hinaus ist ein Arzneimittel, das mindestens eine der erfindungsgemäßen und wie oben definierten Verbindungen der allgemeinen Struktur (I A) oder (I B) bzw. ihrer pharmazeutisch annehmbaren Salze umfaßt, Gegenstand der Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid oder ein Natriumsalz.

Ein weiterer Gegenstand der Erfindung ist die Verwendung mindestens einer erfindungsgemäßen Verbindung der allgemeinen Struktur (I A), oder (I B), einschließlich ihrer Diastereomeren oder Enantiomeren, auch als Racemate oder Enantiomerengemisch, in Form ihrer freien Base oder Säure oder eines mit einer physiologisch verträglichen Säure bzw. Base gebildeten Salzes, insbesondere des Hydrochloridsalzes und des Natriumsalzes, zur Herstellung eines Arzneimittels zur Behandlung von Schmerz. Die erfindungsgemäßen Verbindungen haben sich als analgetisch wirksam erwiesen und binden an die MK801-Bindungsstelle des ionotropen NMDA-Rezeptors.

Es hat sich aufgrund der Bindung an die MK801-Bindungsstelle auch herausgestellt, daß die erfindungsgemäßen Verbindungen der allgemeinen Struktur (I A), oder (I B) für weitere Indikationen, insbesondere zur Behandlung von Epilepsie, der Schizophrenie, von neurodegenerativen Erkrankungen, insbesondere Morbus Alzheimer, Morbus Huntington und Morbus Parkinson, von cerebralen Ischämien und Infarkten, Psychosen bedingt durch erhöhten Aminosäurespiegel, Himödemen, Unterversorgungszuständen des zentralen Nervensystems, insbesondere bei Hypoxien und Anoxien, von AIDS-Demens, von Encephalomyelitis, des Tourette-Syndroms, der perinatalen Asphyxie und bei Tinnitus, sehr geeignet sind. Ein weiterer Gegenstand der Anmeldung ist daher die Verwendung mindestens einer erfindungsgemäßen Verbindung der allgemeinen Struktur (I A), oder (I B) einschließlich eines pharmazeutisch annehmbaren Salzes zur Herstellung eines Arzneimittels zur Behandlung und/oder Prophylaxe von Epilepsie, der Schizophrenie, von neurodegenerativen Erkrankungen, insbesondere Morbus Alzheimer, Morbus Huntington und Morbus Parkinson, von cerebralen Ischämien und Infarkten, Psychosen bedingt durch erhöhten Aminosäurespiegel, Himödemen, Untervereorgungszuständen des zentralen Nervensystems, insbesondere bei Hypoxien und Anoxien, von AIDS-Demens, von Encephalomyelitis, des Tourette-Syndroms, der perinatalen Asphyxie und/oder bei Tirinitus,

Es hat sich femer überraschend gezeigt, daß die erfindungsgemäßen Verbindungen der allgemeinen Formel (I A) und/oder (I B) geeignete Liganden, insbesondere pharmakologisch wirksame Liganden, von Nucleosid-Transport-Proteinen und/oder der Adenosin-Kinase und/oder der Adenosin-Deaminase und/oder von A₁- und/oder von A₂- und/oder von A₃₋Rezeptoren sind.

Es ist bekannt, daß Adenosin und ATP (Adenvsin-5'-triphophat) und die sie bindenden purinergen Rezeptoren (Purinorezepturen; P1- und P2-Rezeptoren) bei der Übertragung und Weiterleitung von sensorischen Informationen sowohl in peripheren Nerven als auch im dorsalen Horn eine bedeutende Rolle spielen (M. W. Salter, A. Sollevi, "Handbook of exp. pharmacol.", Kap. 13, (2001), S. 371-401). Insbesondere spielen Adenosin und ATP sowie die P1- und P2-Rezeptoren eine Rolle in der Entstehung und Weiterleitung von Schmerz. Bei den P1- und P2-Rezeptoren unterscheidet man dabei zwischen den sogenannten ATP-Rezeptoren (= P2-Rezeptoren) und den sogenannten Adenosin-Rezeptoren (= P1-Rezeptoren). Von diesen P1-Rezeptoren sind bislang vier Subtypen - A₁, A₂ₐ, A_{2b} und A₃ - bekannt, die alle zu der Familie der G-Protein-gekoppelten Rezeptoren (GPCR) gehören. (lm folgenden werden die beiden Subtypen A₂ₐ undA_{2b} auch als A₂-Subtyp bezeichnet.)

Ein Einfluß auf die Adenosin-Spiegel im Organismus haben daneben auch die Adenosin-Kinase, die die Umwandlung von Adenosin in AMP (Adenosin-5'-monophosphat) katalysiert, die Adenosin-Deaminase, die die hydrolytische Desaminierung von Adenosin bzw. 2'-Deoxyadenosin zu Inosin bzw. 2'-Deoxyinosin katalysiert, und die Nucleosid-Transport-Proteine, die am Transport von u.a. Adenosin aus dem extrazelluären Raum in die Zelle bzw. umgekehrt beteiligt sind. Letztere spielen insbesondere eine Rolle bei neuropathischen Schmerzzuständen.

Aufgrund der nunmehr gefundenen Eigenschaften ist ein weiterer Gegenstand der vorliegenden Erfindung die Verwendung der Verbindungen der allgemeinen Formel (IA) oder (IB) in der oben dargestellten Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate; in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
wobei die Substituenten die bereits angegebene Bedeutung haben zur Prävention und/oder Behandlung von und zur Herstellung eines Medikaments zur Prävention und/oder Behandlung von Zuständen und/oder Krankheiten, die über eine Stimulierung und/oder Inhibierung von Nucleosid-Transport-Proteinen und/oder der Adenosin-Kinase und/oder Adenosin-Deaminase und/oder von A₁- und/oder von A₂- und/oder von A₃₋Rezeptoren beeinflußt werden.

Vorzugsweise werden die Verbindungen der allgemeinen Formel (IA) und/oder (IB) daher zur Prävention und/oder Behandlung von und zur Herstellung eines Medikaments zur Prävention und/oder Behandlung von Schmerz, neuropathischem Schmerz, Atemwegserkrankungen, Krebs, kardialen Arryfhmien, Ischämien, Epilepsie, Morbus Huntigton, Immunstörungen und -erkrankungen, Entzündungszuständen und - erkrankungen, Neugeborenen-Hypoxie, neurodegenerativen Erkrankungen, Morbus Parkinson, Nierenversagen. Schizophrenie, Schlafstörungen, Schlaganfall, Thrombosen, Harninkontinenz, Diabetes, Psoriasis, septischem Schock, Gehirntraumata, Glaukom und/oder Stauungsinsuffizienz verwendet Für diese Krankheitszustände haben sich die Verbindungen der allgemeinen Formel (IA) und/oder (IB) als wirksam erwiesen. Zudem werden Nebenwirkungen, die üblicherweise bei Verwendung klassischer opioider Schmerzmittel auftreten, mit diesen Verbindungen nicht oder nur selten beobachtet.

Darüber hinaus sind auch pharmazeutische Zusammensetzungen Gegenstand der vorliegenden Erfindung, die mindestens eine Verbindung der wie oben definierten allgemeinen Struktur (I A) oder (I B) oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen. Sprays. Suspensionen, Granulaten, Tabletten, Pellets, transdermale therapeutische Systeme, Kapseln, Pflastem, Zäpfchen, Salben, Cremes, Lotionen, Gelen, Emulsionen oder Aerasolen vorliegen und verabreicht werden und enthalten neben mindestens einer erfindungsgemäßen Verbindung der allgemeinen Struktur (I A) oder (I B) je nach galenischer Form pharmazeutische Hilfsstoffe, wie zB. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristattine Cellulose, Methylcallulose, Carboxymethylcellulose, Colluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, vaginal, pulmonal, intraperitoneal, transdermal, intramuskulär, nasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Struktur (I A) oder (I B) in einem Depot in gelöster Form oder in einem Pflaster, gegebenenfalls unter Zusatz von die Hautpenetration fördernden Mitteln, sind geeignete perkutane Applikationszubereitungen, Rektal, transmucosal, parenteral, oral oder perkutan anwendbare Zubereitungsformen können die erfindungsgemäßen Verbindungen der allgemeinen Struktur (I A) oder (I B) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg, A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur (I A) oder (I B) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammenseizungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur (I A) oder (I B) appliziert.

Die nachfolgenden Beispiele dienen zur näheren Erläuterung der vorliegenden Erfindung:

### Beispiele

Die eingesetzten Chemikalien und Lösungsmittel wurden kommerziell von einem der folgenden Anbieter erworben: Acros, Geel; Avocado, Port of Heyrsham; Aldrich, Deisenhofen; Fluka, Seelze; Lancaster, Mülheim; Maybridge, Tintagel; Merck, Darmstadt; Sigma, Deisenhofen; TCl, Japan; oder nach allgemeinen im Stand der Technik bekannten Verfahren hergestellt.

### Allgemeine Arbeitsvorschrift AAV (Semiautomatisierte Synthese)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt Anschileßend wurden nacheinander die folgenden Reagenzien hinzupipettiert:
1. 1 ml einer Lösung, die Trifluoressigsäure und die Heterocyclylamin-Komponente (III) bzw. (VI) jeweils 0,1 M enthält, in Acetonitril
2. 1- ml einer 0,11 M Aldehyd(IV)-Lösung in Acetonitril
3. 1 ml einer 0,3 M Olefin(V)-Lösung in Acetonitril

Das Reaktionsgemisch wurde bei 20°C in einem der Rührblöcke 600 min lang gerührt. Danach wurde die Reaktionslösung an der Filtrations-Station abfiltriert. Das Röhrchen wurde dabei zweimal mit 1,5 ml einer 7,5% NaHCO₃-Lösung gespült. Das Rack mit den Proben wurde manuell auf die Aufarbeitungsanlage gestellt. Das Reaktionsgemisch wurde auf einem Vortexer mit 2 ml Diethylether versetzt und geschüttelt. Zur Ausbildung der Phasengrenze wurde in der Zentrifuge kurz zentrifugiert. Die Phasengrenze wurde optisch detektiert und die organische Phase abpipettiert.

Im nächsten Schritt wurde die wäßrige Phase erneut mit 2 ml Diethylether versetzt, geschüttelt, zentrifugiert und die organische Phase abpipettiert Die vereinigten organischen Phasen wurden über 2,4 g MgSO₄ (granuliert) getrocknet Das Lösungsmittel wurde in einer Vakuumzentrifuge entfernt.

Jede Probe wurde mit ESI-MS und/oder NMR analysiert. Massenspektrometrische Untersuchungen (ESI-MS) wurden mit einem Massenspektrometer der Fa. Finnegan, LCQ Classic durchgeführt. ¹H-NMR-Untersuchungen der erfindungsgemäßen Verbindungen wurden mit einem 300 MHz DPX Advance NMR-Gerät der Fa, Bruker durchgeführt.

Nach der angegebenen AAV wurden die Beispiel-Verbindungen 1-132, 140-144; 146-151 sowie 153 und 154 (s. Tabelle 1) hergestellt.

**Tabelle 1**

| **Beispiel** | **Name** | **berechnete Masse** | **gefundene Masse** |
|---|---|---|---|
| 1 | 3-Brom-5-(5-nitro-furan-2-yl)-7-m-tolyl-tatrahydro-pyrazolo[1,5-a]pyrimidin | 403.24 | 403,2/405,1 |
| 2 | 3-Brom-7-(4-fluor-phenyl)-7-methyl-5-(5-nitro-furan-2-yl)-tetrahydro-pyrazalo[1,5-a]pyrimidin | 421.23 | 421,1/423,0 |
| 3 | 3-Brom-7-naphthalin-2-yl-5-(5-nitro-furan-2-yl)-tetrahydro-pyrazolo[1.5-a]pyrimldin | 439.27 | 439,2/441,1 |
| 4 | 2-(3-Brom-7-m-tolyl-tetrahydro-pyrazolo[1,5-a]pyrimidin-5-yl)-cyclopropancarborisäure-ethylester | 404.31 | 404,5/406,4 |
| 5 | 2-[3-Brom-7-(4-brom-phenyl)-tetrahydro-pyrazolo[1,5-a]pyrimidin-5-yl]-cyclopropancarbonsäureethylester | 469.18 | 468,3/470,1/4 72.1 |
| 6 | 2-(3-Brom-7-naphthalin-2-yl-tetrahydro-pyrazolo[1,5-a]pyrimidin-5-yl)-cyclopropancarbonsäureethylester | 440.34 | 440,5/442,5 |
| 7 | 3-Brom-7-(4-fluor-phenyl)-7-methyl-5-(5-methyl-furan-2-yl)-tetrahydro-pyrazolo[1,5-a]pyrimidin | 390.26 | 390,1/392,0 |
| 8 | 3-Brom-7-(3,4-dimethoxy-phenyl)-tetrahydro-pyrazolo[1,5-a]pyrimidin-5-carbonsäureethylester | 410.27 | 410,3/412,2 |
| 9 | 3-Brom-7-(4-methoxy-phenyl)-tetrahydro-pyrazolo[1,5-a]pyrimidin-5-carbonsaureethylester | 380.24 | 380,2/382,1 |
| 10 | 3-Brom-7-(4-methoxy-phenyl)-tetrahydro-pyrazolo[1.5-a]pyrimidin-5-carbonsäure | 352.19 | 354,2 |
| 11 | 3-Brom-7-(2,4-dimethyl-phenyl)-5-(5-nitro-furan-2-yl)-tetrahydro-pyrazolo[1,5-a]pyrimidin | 417.26 | 417,1/419,0 |
| 12 | 3-Brom-7-(4-methoxy-phenyl)-5-(5-nitro-furan-2-yl)-tetrahydro-pyrazolo[1,5-a]pyrimidin | 419.23 | 419,0/421,0 |
| 13 | 5,5a,6,8a-Tetrahydro-3H-1,4,8b-triaza-as-indacen-3,5-dicarbonsäurediethylester; 5,5a,6,8a-Tetrehydro-4H-1,4,8b-triaza-as-Indacen-3,5-dicarbonsäurediethylester; | 305.33 | 306,1 |
| 14 | 2-Hydroxy-3-phenylazo-5,5a,6,8a-tetrahydro-3H-1,4,8b-triaza-as-indacen-5-carbonsäureethylester; 2-Hydroxy-3-phenylazo-5,5a,6,8a-tetrahydro-4H-1,4,8b-triaza-as-indacen-5-carbonsäureethylester | 353.38 | 354,3 |
| 15 | 2-*tert*-Butyl-5,5a,6,8a-tetrahydro-3H-1,4,8b-triaza-as-indacen-5-carbonsäureethylester; 2-tert-Butyl-5,5a,6,8a-tetrahydro-3H-1,4,8b-triaza-as-indacen-5-carbonsäureethylester | 289.37 | 290,3 |
| 16 | 3-Brom-2-phenyl-5,5a,8,8a-tetrahydro-3H-1,4,8b-triaza-as-indacen-5-carbonsäureethylester; 3-Brom-2-phenyl-5,5a,6,8a-tetrahydro-4H-1,4,8b-triaza-as-indacen-5-carbonsäureethylester | 388.26 | 388,2/390,1 |
| 17 | 7-(2,3,4-Trimethoxy-phenyl)-tetrahydro-pyrazolo[1,5-a]pyrimidin-3,5-dicarbonsäurediethyl ester | 433.46 | 434,4 |
| 18 | 3-Cyano-2-methylsulfanyl-7-(2,3,4-trimethoxy-phenyl)-tetrahydro-pyrazolo[1,5-a]pyrimidin-5-carbonsäureethylester | 432.49 | 433,2 |
| 19 | 2-Hydroxy-7-(4-hydroxy-phenyl)-6-methyl-3-phenylazo-tetrahydro-pyrazolo[1,5-a]pyrimidin-5-carbonsäureethylester | 421.45 | 422,4 |
| 20 | 3-Brom-7-(4-hydroxy-phenyl)-6-methyl-2-phenyl-tetrahydro-pyrazolo[1,5-a]pyrimidin-5-carbonsäureethylester | 456.34 | 456,4/458,4 |
| 21 | 5,5a,6,10b-Tetrahydro-3H-1,4,10c-triaza-cyclopenta[c]fluoren-3,5-dicarbonsäurediethylester; 5,5a,6,10b-Tetrahydro-4H-1,4,10c-triaza-cyclopenta[c]fluoren-3,5-dicarbonsäurediethylester | 355.39 | 356,2 |
| 22 | 2-Hydroxy-3-phenylazo-5,5a,6,10b-tatrahydro-3H-1,4,10c-triaza-cyclopenla[c]fluoren-5-carbonsäureethylester; 2-Hydroxy-3-phenylazo-5,5a,6,10b-tetrahydro-4H-1,4,10c-triaza-cyclopenta[c]fluoren-5-carbonsäureethylester | 403.44 | 404,3 |
| 23 | 7-Phenylsulfanyl-tetrahydro-pyrazolo[1,5-a]pyrimidin-3,5-dicarbonsäurediethyl ester | 375.44 | 376,2 |
| 24 | 3-Cyano-2-methylsulfanyl-7-phenylsulfanyl-tetrahydro-pyrazolo[1,5-a]pyrimidin-5-carbonsäureethylester | 374.48 | 375,1 |
| 25 | 3-Cyano-2-methylsulfanyl-7-(2,3,4-trimethoxy-phenyl)-tetrahydro-pyrazolo[1,5-a]pyrimidin-5-carbonsäure | 404.44 | 405,2 |
| 26 | 7-Phenylsulfanyl-tetrahydro-pyrazclo[1,5-a]pyrimidin-3,5-dicarbonsäure3-ethyl ester | 347.39 | 348,2 |
| 27 | 3-Cyano-7-(2,4-dimethyl-phenyl)-2-methylsulfanyl-tetrahydro-pyrazolo[1,5-a]pyrimidln-5-carbonsäureethylester | 370.47 | 371,2 |
| 28 | 3-Cyano-7-(2,4-dimethyl-phenyl)-tetrahydro-pyrazolo[1,5-a]pyrimidin-5-carbonsäureethylester | 324.38 | 325,2 |
| 29 | 7-(2,4-Dimethyl-phenyl)-tetrahydro-pyrazolo[1,5-a]pyrimidin-3,5-dicarbonsäure-3-ethyl ester | 343.38 | 344,2 |
| 30 | 3-Brom-7-(2,4-dimethyl-phenyl)-2-phenyl-tetrahydro-pyrazolo[1,5-a]pyrimidin-5-carbonsäure | 428.31 | 426,2/428,1 |
| 31 | 3-Cyano-7-(2,4-dimethyl-phenyl)-2-methylsulfanyl-tetrahydro-pyrazolo[1,5-a]pyrimidin-5-carbonsäure | 342.42 | 343,2 |
| 32 | 3-Cyano-7-(2,4-dimethyl-phenyl)-tetrahydro-pyrazolo[1,5-a]pyrimidin-5-carbonsäure | 296.32 | 297,2 |
| 33 | 3-Cyanc>-7-(3.4-dimathoxy-phenyl)-2-methylsulfanyl-tetrahydro-pyrazolo[1,5-a]pyrimidin-5-carbonsäure | 374.41 | 376,2 |
| 34 | 7-(2,4-Dimethyl-phenyl)-5-(5-nitro-furan-2-yl)-tetrahydro-pyrazolo[1,5-a]pyrimidin-3-carbonsäureethylester | 410.42 | 411,1 |
| 35 | 7-(2,4-Dimethyl-phenyl)-5-(5-nitro-furan-2-yl)-3-phenylazo-tetrahydro-pyrazolo[1,5-a]pyrimidin-2-ol | 458.47 | 459,3 |
| 36 | 3-Brom-7-(2,4-dimethyl-phenyl)-5-(5-nitro-furan-2-yl)-2-phenyl-tetrahydro-pyrazolo[1,5-a]pyrimidin | 493.36 | 493,2/495,1 |
| 37 | 7-(2,4-Dimethyl-phenyl)-2-methylsulfanyl-5-(5-nitro-furan-2-yl)-tetrahydro-pyrazolo[1,5-a]pyrimidin-3-carbonitril | 409.46 | 410,1 |
| 38 | 7-(2,4-Dimethyl-phenyl)-5-(5-nitro-furan-2-yl)-tetrahydro-pyrazolo[1,5-a]pyrimidin-3-carbonitril | 363.37 | 364,1 |
| 39 | 3-Brom-7-(3,4-dimethoxy-phenyl)-5-(5-nitro-furan-2-yl)-2-phenyl-tetrahydro-pyrazolo[1,5-a]pyrimidin | 525.36 | 525,4/527,1 |
| 40 | 7-(4-Methoxy-phenyl)-2-methylsulfanyl-5-(5-nitro-furan-2-yl)-tetrahydro-pyrazolo[1,5-a]pyrimidin-3-carbonitril | 411.43 | 412,9 |
| 41 | 7-(2,4-Dimethyl-phenyl)-5-(2-ethoxycarbonyl-cyclopropyl)-tetrahydro-pyrazolo[1,5-a]pyrimidin-3-carbonsäureethylester | 411.5 | 412,5 |
| 42 | 2-[7-(2,4-Dimethyl-phenyl)-2-hydroxy-3-phenylazo-tetrahydro-pyrazolo[1,5-a]pyrimidin-5-yl]-cyclopropancarbonsäureethylester | 459.54 | 460,3 |
| 43 | 2-[2-tert-Butyl-7-(2,4-dimethyl-phenyl)-tetrahydro-pyrazolo[1,5-a]pyrimidin-5-yl]-cyclopropancarbonsäureethylester | 395.54 | 396,5 |
| 44 | 2-[3-Brom-7-(2,4-dimethyl-phenyl)-2-phenyl-tetrahydro-pyrazolo[1,5-a]pyrimidin-5-yl]-cyclopropancarbonsäureethylester | 494.43 | 494,4/496,2 |
| 45 | 2-[3-Cyano-7-(2,4-dimethyl-phenyl)-2-methylsulfanyl-tetrahydro-pyrazolo[1,5-a]pyrimidin-5-yl]-cyclopropancarbonsäureethylester | 410.53 | 411,3 |
| 46 | 5-(2-Ethoxycarbonyl-cyclopropyl)-7-(3-fluor-phenyl)-tetrahydro-pyrazolo[1,5-a]pyrimidin-3-carbonsäureethylester | 401.43 | 402,2 |
| 47 | 2-[3-Brom-7-(3-brom-phenyl)-2-phenyl-tetrahydro-pyrazolo[1,5-a]pyrimidin-5-yl]-cyclopropancarbonsäureethylester | 545.28 | 544,3/546,1/5 48,0 |
| 48 | 2-[7-(3-Brom-phenyl)-3-cyano-2-methylsulfanyl-tetrahydro-pyrazolo[1,5-a]pyrimidin-5-yl]-cyclopropancarbonsäureethylester | 461.38 | 461,2/463,0 |
| 49 | 7-(2,4-Dimethyl-phenyl)-5-(5-nitro-thiophen-2-yl)-3-phenylazo-tetrahydro-pyrazolo[1,5-a]pyrimidin-2-ol | 474.54 | 475,2 |
| 50 | 7-(2,4-Dimethyl-phenyl)-2-methylsulfanyl-5-(5-nitro-thiophen-2-yl)-tetrahydro-pyrazolo[1,5-a]pyrimidin-3-carbonitril | 425.53 | 426,1 |
| 51 | 7-(2,4-Dimethyl-phenyl)-5-(5-nitro-thlophen-2-yl)-tetrahydro-pyrazolo[1,5-a]pyrimidin-3-carbonitril | 379.44 | 380,1 |
| 52 | 7-(3,4-Dimethoxy-phenyl)-5-(5-nitro-thiophen-2-yl)-tetrahydro-pyrazolo[1,5-a]pyrimidin-3-carbonsäureethylester | 458.49 | 459,3 |
| 53 | 7-(3,4-Dimethoxy-phenyl)-5-(5-nitro-thiophen-2-yl)-3-phenylazo-tetrahydro-pyrazolo[1,5-a]pyrimidin-2-ol | 506.54 | 507,2 |
| 54 | 3-Brom-7-(3,4-dimethoxy-phenyl)-5-(5-nitro-thlophen-2-yl)-2-phenyl-tetrahydro-pyrazolo[1,5-a]pyrimidin | 541.42 | 541,4/543,3 |
| 55 | 7-(3,4-Dimethoxy-phenyl)-2-methylsulfanyl-5-(5-nitro-thiophen-2-yl)-tetrahydro-pyrazolo[1,5-a]pyrimidin-3-carbonitril | 457.53 | 458,1 |
| 56 | 7-(3,4-Dimethoxy-phenyl)-5-(5-nitro-thiophen-2-yl)- tetrahydro-pyrazolo[1,5-a]pyrimidin-3-carbonitril | 411.43 | 412,1 |
| 57 | 7-(4-Methoxy-phenyl)-5-(5-nitro-thiophen-2-yl)-tetrahydro-pyrazolo[1,5-a]pyrimidin-3-carbonsäureethylester | 428.46 | 429,1 |
| 58 | 5-[3-Brom-7-(4-methoxy-phenyl)-2-phenyl-tetrahydro-pyrazolo[1,5-a]pyrimidin-5-yl]-furan-2-carbonsäure | 494.34 | 494,2/496,1 |
| 59 | 5-Benzoyl-7-(2,4-dimethyl-phenyl)-tetrahydro-pyrazolo[1,5-a]pyrimidin-3-carbonsäureethylester | 403.48 | 404,2 |
| 60 | 5-Benzoyl-7-(2,4-dimethyl-phenyl)-2-methylsulfanyl-tetrahydro-pyrazolo[1,5-a]pyrimidin-3-carbonitril | 402.51 | 403,1 |
| 61 | 5-Benzoyl-7-(2,4-dimethyl-phenyl)-tetrahydro-pyrazolo[1,5-a]pyrimidin-3-carbonitril | 356.42 | 355,3/357,2 |
| 62 | 5-Benzoyl-7-(3,4-dimethoxy-phenyl)-tetrahydro-pyrazolo[1,5-a]pyrimidin-3-carbonsäurenthylester | 435.47 | 436,2 |
| 63 | [3-Brom-7-(3,4-dimethoxy-phenyl)-2-phenyl-tetrahydro-pyrazolo[1,5-a]pyrimidin-5-yl]-phenyl-methanon | 518.41 | 518,7/520,2 |
| 64 | 5-Benzoyl-7-(3,4-dimethoxy-phenyl)-2-methylsulfanyl-tetrahydro-pyrazolo[1,5-a]pyrimidin-3-carbonltril | 434.51 | 435,1 |
| 65 | 5-Benzoyl-7-(3,4-dimethoxy-phenyl)-tatrahydro-pyrazolo[1.5-a]pyrimidln-3-carbonitri | 388.42 | 389,1 |
| 66 | 5-Benzoyl-7-(4-methoxy-phenyl)-tetrahydro-pyrazolo[1,5-a]pyrimidin-3-carbonsäureethylester | 405.45 | 406,1 |
| 67 | 5-Benzoyl-7-(4-methoxy-phenyl)-2-methylsulfanyl-tetrahydro-pyrazolo[1,5-a]pyrimidin-3-carbonitril | 404.49 | 405,0 |
| 68 | 5-Benzoyl-7-(4-methoxy-phenyl)-tetrahydro-pyrazolo[1,5-a]pyrimidin-3-carbonitril | 358.4 | 359,0 |
| 69 | 5-Benzoyl-7-(3-fluor-phenyl)-tetrahydro-pyrazolo[1,5-a]pyrimidin-3-carbonsäureethylester | 393.41 | 394,4 |
| 70 | [3-Brom-7-(3-fluor-phenyl)-2-phenyl-tetrahydro-pyrazolo[1,5-a]pyrimidin-5-yl]-phenyl-methanon | 476.35 | 476,3/478,3 |
| 71 | [3-Brom-7-(3-brom-phenyl)-2-phenyl-tetrahydro-pyrazolo[1,5-a]pyrimidin-5-yl]-phenyl-methanon | 537.26 | 538,4 |
| 72 | 7-(2,4-Dimethyl-phenyl)-5-(4-phenoxy-phenyl)-tetrahydro-pyrazolo[1,5-a]pyrimidin-3-carbonsäureethylester | 467.56 | 468,2 |
| 73 | 3-Brom-7-(2,4-dimethyl-phenyl)-5-(4-phenoxy-phenyl)-2-phenyl-tetrahydro-pyrazolo[1,5-a]pyrimidin | 550.5 | 550,3/552,2 |
| 74 | 7-(2,4-Dimethyl-phenyl)-2-methylsulfanyl-5-(4-phenoxy-phenyl)-tetrahydro-pyrazolo[1,5-a]pyrimidin-3-cerbonitril | 466.6 | 467,1 |
| 75 | 1-(2,4-Dimethyl-phenyl)-5-(4-phenoxy-phenyl)-tetrahydro-pyrazolo[1,5-a]pyrimidin-3-carbonitril | 420.51 | 421,1 |
| 76 | 7-(3,4-Dimethoxy-phenyl)-5-(4-phenoxy-phenyl)-tetrahydro-pyrazolo[1,5-a]pyrimidin-3-carbonsäureethylester | 499.56 | 500,2 |
| 77 | 7-(3,4-Dimethoxy-phenyl)-2-methylsulfanyl-5-(4-phenoxy-phenyl)-tetrahydro-pyrazolo[1,5-a]pyrimidin-3-carbonitril | 498.6 | 499,1 |
| 78 | 3-[3-Cyano-7-(4-hydroxy-phenyl)-6-methyl-tetrahydro-pyrazolo[1,5-a]pyrimidin-5-yl]-2-hydroxy-benzoesäure | 390.39 | 391,1 |
| 79 | 3-(3-Cyano-5,5a,6,10b-tetrahydro-3H-1,4,10c-triaza-cyclopenta[c]fluoren-5-yl)-2-hydroxy-benzoesäure; 3-(3-Cyano-5,5a,B,10b-tetrahydro-4H-1,4,10c-triaza-cyclopenta[c]fluoren-5-yl)-2-hydroxy-benzoesäure | 372.38 | 373,0 |
| 80 | 3-(3-Cyano-7-phenylsulfanyl-tetrahydro-pyrezolo[1,5-a]pyrimidin-5-yl)-2-hydroxy-benzoesäure | 392.43 | 392,9 |
| 81 | 3-[2-tert-Butyl-7-(4-chlor-phenyl)-7-methyl-tetrahydro-pyrazalo[1,5-a]pyrimidin-5-yl]-2 hydroxy-benzoesäure | 439.94 | 440,2 |
| 82 | 5-(4-Hydroxy-3-methoxy-phenyl)-7-(4-hydroxy-phenyl)-6-methyl-tetrahydro-pyrazolo[1,5-a]pyrimidin-3-carbonsäureethylester | 423.46 | 424,1 |
| 83 | 5-(4-Hydroxy 3-methoxy-phenyl)-7-(4-hydroxy-phenyl)-6-methyl-tetrahydro-pyrazolo[1,5-a]pyrimidin-3-carbonitril | 376.41 | 377,1 |
| 84 | 5-(4-Hydroxy-3-methoxy-phenyl)-5,5a,6,10b-tetrahydro-3H-1,4,10c-triaza-cyclopenta[c]fluoren-3-carbonsäureethylester; 5-(4-Hydroxy-3-methoxy-phenyl)-5,5a,6,10b-tetrahydro-4H-1,4,10c-triaza-cyclopenta[c]fluoren-3-carbonsäureethylester | 405.45 | 406,0 |
| 85 | 4-(2-tert-Butyl-5,5a,6,10b-tetrahydro-3H-1,4,10c-triaza-cyclopenta[c]fluoren-5-yl)-2-methoxy-phenol; 4-(2-tert-Butyl-5,5a,6,10b-tetrahydro-4H-1,4,10c-triaza-cyclopenta[c]fluoren-5-yl)-2-methoxy-phenol | 389.49 | 390,2 |
| 86 | 5-(4-Hydroxy-3-methoxy-phenyl)-2-methylsulfanyl-5,5a,6,10b-tetrahydro-3H-1,4,10c-triaza-cyclopenta[c]fluoren-3-carbonitril; 5-(4-Hydroxy-3-methoxy-phenyl)-2-methylsulfanyl-5,5a,6,10b-tetrahydro-3H-1,4,10c-triaza-cyclopenta[c]fluoren-3-carbonitril | 404.49 | 404,9 |
| 87 | 5-(4-Hydroxy-3-methoxy-phenyl)-7-phenylsulfanyl-tetrahydro-pyrazolo[1,5-a]pyrimidin-3-carbansäureethytester | 425.5 | 426.0 |
| 88 | 4-(2-tert-Butyl-7-phenylsulfanyl-tetrahydro-pyrazolo[1,5-a]pyrimidin-5-yl)-2-methoxy-phenol | 409.55 | 410.1 |
| 89 | 4-(3-Brom-2-phenyl-7-phenylsulfanyl-tetrahydro-pyrazolo[1,5-a]pyrimidin-5-yl)-2-methoxy-phenol | 508.44 | 508,2/510,0 |
| 90 | 5-(2-Hydroxy-3-methoxy-phenyl)-7-phenylsulfanyl-tetrahydro-pyrazolo[1,5-a]pyrimidin-3-carbonsäureethylester | 425.5 | 425,0 |
| 91 | 7-(4-Chlor-phenyl)-5-(2-hydroxy-3-methoxy-phenyl)-7-methyl-tetrahydro-pyrazolo[1,5-a]pyrimidin-3-carbonsäureethylester | 441.91 | 441,0 |
| 92 | 5-(4-Hydroxy-butyl)-5,5a,6,10b-tetrahydro-3H-1,4,10c-triaza-cyclopenta[c]fluoren-3-carbonitril; 5-(4-Hydroxy-butyl)-5,5a,6,10b-tetrahydro-4H-1,4,10c-triaza-cyclopenta[c]fluoren-3-carbonitril | 308.38 | 309,3 |
| 93 | 5-(4-Hydroxy-butyl)-2-methylsulfanyl-7-phenylsulfanyl-tetrahydro-pyrazolo[1,5-a]pyrimidin-3-carbonitril | 374.52 | 375,2 |
| 94 | 5-(4-Hydroxy-butyl)-7-phenylsulfanyl-tetrahydro-pyrazolo[1,5-a]pyrimidin-3-carbonitril | 328.43 | 329,2 |
| 95 | 7-(4-Chlor-phenyl)-5-(4-hydroxy-butyl)-7-methyl-tetrahydro-pyrazolo[1,5-a]pyrimidin-3-carbonitril | 344.84 | 345,1 |
| 96 | 5-Butyl-2-methylsulfanyl-5,5a,6,10b-tetrahydro-3H-1,4,10c-triaza-cyclopenta[c]fluoren-3-carbonitril; Butyl-2-methylsulfanyl-5,5a,6,10b-tetrahydro-4H-1,4,10c-triaza-cyclopanta[c]fluoren-3-carbonitril | 338.47 | 339,3 |
| 97 | 5-Butyl-2-methylsulfanyl-7-phenylsulfanyl-tetrahydro-pyrazolo[1,5-a]pyrimidin-3-carbonitril | 358.52 | 359,2 |
| 98 | 5-Butyl-7-phenylsulfanyl-tetrahydro-pyrazolo[1,5-a]pyrimidin-3-carbonitril | 312.43 | 313,1 |
| 99 | 5-Butyl-7-(4-chlor-phenyl)-7-methyl-2-methylsulfanyl-tetrahydro-pyrazolo[1,5-a]pyrimidin-3-carbonitril | 374.93 | 375,3 |
| 100 | 5-Cyclopropyl-7-(2,4-dimethyl-phenyl)-3-phenylazo-tetrahydro-pyrazolo[1,5-a]pyrimidin-2-ol | 387.48 | 388,5 |
| 101 | 2-tert-Butyl-5-cyclopropyl-7-(2,4-dimethyl-phenyl)-tetrahydro-pyrazolo[1,5-a]pyrimidin | 323.48 | 324,6 |
| 102 | 5-Cyclopropyl-7-(2,4-dimethyl-phenyl)-2-methylsulfanyl-tetrahydro-pyrazolo[1,5-a]pyrimidin-3-carbonitril | 338.47 | 339,8 |
| 103 | 2-tert-Butyl-5-cyclopropyl-7-(3,4-dimethoxy-phenyl)-tetrahydro-pyrazolo[1,5-a]pyrimidin | 355.48 | 356,3/357,6 |
| 104 | 3-Brom-5-cyclopropyl-7-(3,4-dimethoxy-phenyl)-2-phenyl-tetrahydro-pyrazolo[1,5-a]pyrimidin | 454.37 | 454,3/456,1 |
| 105 | 5-Cyclopropyl-7-(4-methoxy-phenyl)-tetrahydro-pyrazolo[1,5-a]pyrimidin-3-carbonsaureethylester | 341.41 | 342,7 |
| 106 | 5-Cyclopropyl-3,5,5a,6,7,11b-hexahydro-1,4,11c-triaza-cyclopenta[c]phenanthrene-3-carbonitril | 290.36 | 291,4 |
| 107 | 7-(2,4-Dimethyl-phenyl)-5-pyridin-2-yl-tetrahydro-pyrazolo[1,5-a]pyrimidin-3-carbonsäureethylester | 376.45 | 377,3/378,4 |
| 108 | 7-(2,4-Dimethyl-phenyl)-3-phenylazo-5-pyridin-2-yl-tetrahydro-pyrazolo[1,5-a]pyrimidin-2-ol | 424.5 | 425,3 |
| 109 | 3-Brom-7-(2,4-dimethyl-phenyl)-2-phenyl-5-pyridin-2-yl-tetrahydro-pyrazolo[1,5-a]pyrimidin | 459,39 | 459,7/462,2 |
| 110 | 7-(2,4-Dimethyl-phenyl)-2-methylsulfanyl-5-pyridin-2-yl-tetrahydro-pyrazolo[1,5-a]pyrimidin-3-carbonitril | 375.49 | 376,4 |
| 111 | 7-(3,4-Dimethoxy-phenyl)-2-methylsulfanyl-5-phenethyl-tetrahydro-pyrazolo[1,5-a]pyrimidin-3-carbonitril | 434.56 | 435,3 |
| 112 | 7-(3,4-Dimethoxy-phenyl)-5-phenethyl-tetrahydro-pyrazolo[1,5-a]pyrimidin-3-carbonitril | 388.47 | 389,2 |
| 113 | 5-Cyclopropyl-7-(2-hydroxy-ethoxy)-tetrahydro-pyrazolo[1,5-a]pyrimidin-3-carbonsäureethylester | 295.33 | 298,2 |
| 114 | 2-(2-tert-Butyl-5-cyclopropyl-telrahydro-pyrazolo[1,5-a]pyrimidin-7-yloxy)-ethanol | 279.38 | 280,3 |
| 115 | 5-Cydoprapyl-3,5,5a,6,7,8a-hexahydro-8-oxa-1,4,8b-triaza-as-indacen-3-carbonsäureethylester 5-Cyclopropyl-4,5,5a,6,7,8a-hexahydro-8-oxa-1,4,8b-triaza-as-Indacen-3-carbonsäureethylester | 277.32 | 278,3 |
| 116 | 5-Cyclopropyl-3-phenylazo-3,5,5a,6,7,8a-hexahydro-8-oxa-1,4,8b-triaza-as-indacen-2-ol; 5-Cyclopropyl-3-phenylazo-4,5,5a,6,7,8a-hexahydro-8-oxa-1,4,8b-triaza-as-indacen-2-ol | 325.37 | 326,5 |
| 117 | 7-Cyclohexyloxy-5-cyclopropyl-tetrahydro-pyrazolo[1,5-a]pyrimidin-3-carbonsäureethylester | 333.43 | 334,1 |
| 118 | 7-Cyclohexyloxy-5-cyclopropyl-2-methylsulfanyl-tetrahydro-pyrazolo[1,5-a]pyrimidin-3-carbonitril | 332.46 | 333,2 |
| 119 | 7-(4-Chlor-phenyl)-5-cyclohexyl-tetrahydro-pyrazolo[1,5-a]pyrimidin-3-carbonitril | 340.85 | 341,4 |
| 120 | 5-Cyclohexyl-7-(2-hydroxy-ethoxy)-tetrahydro-pyrazolo[1,5-a]pyrimidin-3-carbonsäureethylester | 337.41 | 338,3 |
| 121 | 5-Cyclohexyl-3,5,5a,6,7,8a-hexahydro-8-oxa-1,4,8b-triaza-as-indacen-3-carbonsäureethylester; 5-Cyclohexyl-4,5,5a,6,7,8a-hexahydro-8-oxa-1,4,8b-triaza-as-indacen-3-carbansäureethylester | 319.4 | 320,3 |
| 122 | 5-Cyclohexyl-7-cyclohexyloxy-tetrahydro-pyrazolo[1,5-a]pyrimidin-3-carbonsäureethylester | 375.51 | 376,2 |
| 123 | 7-(2,4-Dimethyl-phenyl)-3-phenylazo-5-propyl-tetrahydro-pyrazolo[1,5-a]pyrimidin-2-ol | 389.5 | 390,5 |
| 124 | 7-(2,4-Dimethyl-phenyl)-2-methylsulfanyl-5-propyl-tetrahydro-pyrazolo[1,5-a]pyrimidin-3-carbonitril | 340.49 | 341,3 |
| 125 | 5-tert-Butyl-7-(2,4-dimethyl-phenyl)-tetrahydro-pyrazolo[1,5-a]pyrimidin-3-carbonsäureethylester | 355.48 | 356,1 |
| 128 | 2,5-Di-tert-buyl-7-(3,4-dimethoxy-phenyl)-tetrahydro-pyrazolo[1,5-a]pyrimidin | 371.52 | 372,2 |
| 127 | 3-Brom-5-tert-butyl-7-(3,4-dimethoxy-phenyl)-2-phenyl-tetrahydro-pyrazolo[1,5-a]pyrimidin | 470.41 | 470,2/472,1 |
| 128 | 2-[3-Cyano-6,7-bis-(4-methoxy-phenyl)-tetrahydro-pyrazolo[1,5-a]pyrimidin-5-yl]-cyclopropancarbonsäureethylester | 472.54 | 473,0 |
| 129 | 3-Cyano-8,7-bis-(4-methoxy-phenyl)-tetrahydro-pyrazolo[1,5-a]pyrimidin-5-carbonsäure | 404.42 | 405,0 |
| 130 | 4-[3-Brom-6-methyl-2-phenyl-5-(4-frifluormethyl-phenyl)-tetrahydro-pyrazolo[1,5-a]pyrimidin-7-yl]-phenol | 528.37 | 528,1 |
| 131 | 7-(4-Hydroxy-phenyl)-6-methyl-2-methylsulfanyl-5-(4-trifluormethyl-phenyl)-tetrahydro-pyrazolo[1,5-a]pyrimidin-3-carbonltril | 444.47 | 445,1 |
| 132 | 7-(4-Hydroxy-phenyl)-6-methyl-5-(4-trifluormathyl-phenyl)-tetrahydro-pyrazolo[1,5-a]pyrimidin-3-carbonitril | 398.38 | 399,1 |
| 140 | 7-Phenyl-3-phenylazo-5-pyridin-2-yl-3,5,6,7-tetrahydro-pyrazolo[15-a]pyrimidin-2-ol | 398.47 | 397,4 |
| 141 | 7-Phenylsulfanyl-5-pyridin-2-yl-3,5,6,7-tetrahydro-pyrazolo[1,5-a]pyrimidine-3-carbonsäureathylester | 380,5 | 381,4 |
| 142 | 3-Phanylazo-7-phenylsulfanyl-5-pyridln-2-yl-3,5,6,7-tetrahydro-pyrazolo[1,5-a]pyrimidin-2-ol | 428,51 | 429,6 |
| 143 | 3-Brom-7-phenylsullanyl-5-pyrldin-2-yl-3,5,6,7-tetrahydro-pyrazolo-[15-a]pyrimidin | 387,3 | 387,2 |
| 144 | 7-Phenysulfanyl-5-pyridin-2-yl-3,5,6,7-tetrahydro-pyrazolo[1,5-a]pyrimidin-3-carbonitril. | 333,41 | 334,2 |
| 146 | 7-(3,4-Dimethoxy-phenyl)-2-methylsulfanyl-5-pyridin-2-yl-tetrahydro-pyrazolo[1,5-a]pyrimidin-3-carbonitril | 407,0 | 409,5/409,9 |
| 147 | 3-Brom-7-(3,4-dimethoxy-phenyl)-2-phenyl-5-pyridin-2-yl-tetrahydro-pyrazolo[1,5-a]pyrimidin | 490,9 | 492,6/494,4 |
| 148 | 3-Brom-7-(3,4-dimethoxy-phenyl)-2-phenyl-tetrahydro-pyrazolo[1,5-a]pyrimidin-5-carbonsäureethylester | 485,9 | 486,5/488,4 |
| 149 | 3-Brom-7-(3,4-dimethoxy-phenyl)-5-(5-nitro-furan-2-yl)-2-phenyl-tetrahydro-pyrazolo[1,5-a]pyrimidin | 524,9 | 525,4/527,1 |
| 150 | 3-Cyano-7-(3,4-dimethoxy-phenyl)-2-methylsulfanyl-tetrahydro-pyrazolo[1,5-a]pyrimidin-5-carbonsäureethylester | 402,0 | 402,5 |
| 151 | 3-Cyano-7-(3,4-dimethoxy-phenyl)-tetrahydro-pyrazolo[1,5-a]pyrimidin-5-carbonsäureethylester | 356,0 | 357,2 |
| 153 | 7-(3,4-Dimethoxy-phenyl)-2-methylsulfanyl-5-(5-nitro-furan-2-yl)-tetrahydro-pyrazolo[1,5-a]pyrimidine-3-carbonitril | 441,5 | 442,1 |
| 154 | 7-(3,4-Dimethoxy-phenyl)-5-pyridin-2-yl-4,5,6,7-tetrahydro-pyrazolo[1,5-a]pyrimidine-3-carbonsäureethylester | 408,5 | 409,5 |

### Beispiel 145

### 3-Brom-7-(3,4-dimethoxy-phenyl)-2-phenyl-5-pyridin-2-yl-4,5,6,7-tetrahydro-pyrazolo[1,5-a]pyrimidin-Dihydrochlorid

In einem 100 ml Einhalskolben wurden 1,5 g 5-Amino-4-brom-3-phenylpyrazol in Acetonitril vorgelegt, 1,55 g 3,4-Dimethoxystyrol, 0,88 g 2-Pyridylcarbaldehyd und 0,72 ml Trifluoressigsäure zugegeben und über Nacht bei Raumtemperatur gerührt. Die Reaktionslösung wurde komplett eingeengt. Das Rohprodukt wurde mit Reversed-Phase-HPLC aufgereinigi. HPLC-Säule, Macherey-Nagel, VP 100/21 Nucleosil 100-3 C18 HD (Ser.No. 0115186 Batch 23710123);
Gradient Methanol (Riedel-deHaen, Chromasolv) / Wasser: Gradient (4
Stufen) von 60 bis 100 % Methanol in 37,5 min (Flow 10 ml/min, Injection volume: 1ml)
HPLC: Beckman SYSTEM GOLD, (Detector 166, Injector
Endurance/SPARK, 125P Solvent Module, Fraktionssammler:
Foxy200/ISCO)

Zur Hydrochloridbildung wurden 139 mg 3-Brom-7-(3,4-dimethoxy-phenyl)-2-phenyl-5-pyridin-2-yl-4,5,6,7-tetrahydro-pyrazolo[1,5-a]pyrimidin in 1,1 ml Methylethylketon gelöst und dann mit 6 µl H₂O und 7 µl TMSCI versetzt. Nach einiger Zeit fiel ein Feststoff aus. Nach Absaugen und waschen mit Ether erhielt man gelbe Kristalle, die im Vakuum getrocknet wurden.
¹H-NMR-Daten (600 MHz; DMSO-d6):
δ = 8.68 (m, 1H), 8.27 (m, 1 H), 7.92 (d, 1H, J = 8.1 Hz), 7.74-7.67 (m, 3H), 7.39 (dd, 2H, J = 7.5, 7.5 Hz), 7.33 (t, 1 H, J = 7.2 Hz), 6.83 (d, 1 H, J = 7.7 Nz), 6.78 (s, 1H), 6.66 (d, 1H, J = 9.0 Hz), 5.47 (m, 1H), 5.03 (m, 1H), 3.71 (s, 3H), 3.69 (s, 3H), 2.67 (m, 1H), 2.59 (m, 1H).

### Beispiel 147

3-Brom-7-(3,4-dimethoxy-phenyl)-2-phenyl-5-pyridin-2-yl-tetrahydro-pyrazolo-[1,5-a]pyrimidin, hergestellt nach dem oben angegebenen Verfahren
¹H-NMR-Daten (600 MHz; DMSO-d6):
δ = 8.34 (br. s, 1H), 7.93 (d, 1 H, J = 3.8 Hz), 7.64 (s, 1H), 7.14 (d, 1 H, J = 4.5 Hz), 6.81 (d, 1H, J = 8.3 Hz), 6.71 (s, 1H), 6.60 (d, 1H, J = 8.3 Hz), 5.40 (dd, 1H, J = 4.5, 8.3 Hz), 5.09 (br. d, 1H, J = 8.3 Hz), 3.70 (s. 3H), 3.68 (s, 3H), 2.59 (br. d, 1 H, J = 13.6 Hz), 2.50 (m, 1 H).

### Beispiel 152

### 7-(3,4-Dimethoxy-phenyl)-5-pyridin-2-yl-4,5,6,7-tetrahydro-pyrazolo[1,5-a]pyrimidine-3-carbonitril-Dihydrochlorid

1 g (9,3 mmol) 3-Aminopyrazol-4-carbonitril wurden in 28 ml Acetonitril vorgelegt, 2,28 g (13,9 mmol) 3,4-Dimethoxystyrol, 1,29 g (12 mmol) Pyridin-2-carbaldehyd und 1,05 g (9,3 mmol) Trifluoeressigsäure zugegeben und Ober Nacht bei Raumtemperatur gerührt Die dunkelbraune Lösung wurde komplett einrotiert und getrocknet. Das Produkt wurde über HPLC (Bedingungen wie oben angegeben) gereinigt.

Die so erhaltene Base (270 mg) wurde in Methanol suspendiert und dann mit 15µl H₂O und 208 µl TMSCI versetzt. Es entstand eine klare Lösung, die am Rotavapor eingeengt wurde. Die entstandenen gelbe Kristalle wurden im Vakuum getrocknet.
¹H-NMR-Daten (600 MHz; DMSO-d6):
δ = 8.65 (d, 1 H, J = 4.8 Hz), 8,23 (t, 1H, J = 7.2 Hz), 8.16 (m, 1H), 7.86 (d, 1H), 7.70-7.65 (m, 2H), 6.77 (d, 1H, J = 8.3 Hz), 6.68 (s, 1H), 6.53 (d, 1H, J = 8.3 Hz), 5.43 (dd, 1H, J = 4.5, 7.5 Hz), 5.08 (m, 1H), 3.70 (s, 3H), 3.68 (s, 3H), 2.73 (m, 1H), 2.63 (br. d, 1H, J = 14.3 Hz).

### Beispiel 56 (Dihydrochlorid)

In zu den Beispielen 145 und 152 analoger Weise wurde das Dihydrochlorid von 7-(3,4-Dimethoxy-phenyl)-5-(5-nitro-thiophen-2-yl)-tetrahydro-pyrazolo[1,5-a]pyrimidin-3-carbonitril hergestellt.
¹H-NMR-Daten (600 MHz; DMSO-d6):
δ 8.65 (d, 1H, J = 4.8 Hz), 8.23 (t, 1H, J = 7.2 Hz), 8.16 (m, 1H), 7,86 (d, 1H), 7.70-7.65 (m, 2H), 6.77 (d, 1H, J = 8.3 Hz), 6.68 (s, 1H), 6.53 (d, 1H, J = 8.3 Hz), 5,43 (dd, 1H, J = 4.5, 7.5 Hz), 5.08 (m, 1H), 3.70 (s, 3H), 3.68 (s, 3H), 2.73 (m, 1H), 2.63 (br. d, 1H, J = 14.3 Hz).

### Pharmakologische Untersuchungen:

Die Untersuchungen zur Bestimmung der NMDA-antagonistischen Wirkung der erfindungsgemäßen Verbindungen wurden an Himmembranhomogenaten (Homogenat von Rattenhim ohne Cerebellum, Pons und Medulla oblongata von männlichen Wistar-Ratten (Charies River, Sulzfeld, Deutschland)) durchgeführt.

Hierzu wurden frisch präparierte Rattengehime nach Abtrennen von Cerebellum, Pons und Medulla oblongata in 50 mmol/l Tris/HCl (pH 7,7) mit einem Polytron-Homogenisator (Modell PT3000, Kinematika AG, Littau, Schweiz) bei 6.000 Umdrehungen pro Minute (UPM) für 1 Minute unter Eiskühlung aufgeschlossen und anschließend für 15 Minuten bei 4 °C und 60.000 g zentrifugiert, Nach Dekantieren und Verwerfen des Überstandes, erneutem Aufnehmen in 50 mmol/l Tris/HGI (pH 7,7) und Aufschluß des Membranpellets mit einem Homogenisator bei 2.000 UPM für 1 Minute wurde erneut für 15 Minuten bei 4 °C und 60.000 g zentrifugiert. Der Überstand wurde wiederum verworfen und das Membranpellet in 50 mmol/l Tris/HCl (pH 7,7) homogenisiert (2.000 UPM für 1 Minute) und atiquotiert bei -70 °C eingefroren.

Für den Razeptorbindungstest wurden jeweils Aliquote aufgetaut und anschließend für 15 Minuten bei 4 °C und 60.000 g zentrifugiert. Nach Dekantieren und Verwerfen des Überstandes wurde das Membranpellet für den Bindungstest mit Bindungstest-Puffer aufgenommen und homogenisiert (2.000 UPM für 1 Minute). Als Bindungstest-Puffer wurden 5 mmol/l Tris/HCl (pH 7,7) supplementiert mit 30 µmol/l Glycin und 100 µmol/l Glutaminsäure verwendet.

Als radioaktiv markierter Ligand wurde 1 nmol/l (³H)-(+)-MK801 ((5R,10S)-(+)-5-Methyl-10,11-dihydro-5H-dibenzo(a,d)cyclohepten-5,10-imin (NET-972, NEN, Köln, Deutschland)) zugegeben. Der Anteil an unspezifischer Bindung wurde in Anwesenheit von 10 µmol/l nicht radioaktiv markiertem (+)-MK801 (RBI/Sigma, Deisenhofen, Deutschland) bestimmt. In weiteren Ansätzen wurden die jeweiligen erfindungsgemäßen Verbindungen in Konzentrationsreihen zugegeben und die Verdrängung des radioaktiven Liganden aus seiner spezifischen Bindung am NMDA-Rezeptor ermittelt.

Die Ansätze wurden jeweils für 40 Minuten bei 25 °C inkubiert und anschließend zur Bestimmung des an das Hirnmembranhomogenat gebundenen radioaktiven Liganden mittels Filtration geerntet. Die durch die Filter zurückgehaltene Radioaktivität wurde nach Zugabe von Szintillator (Szintillator "Ready Protein", Beckmann Coulter GmbH, Krefeld, Deutschland) im β-Counter (Packard TRI-CARB Liquid Szintillation Analyzer 2000CA, Packard Instrument, Meriden, CT 06450, USA) vermessen.

Die resultierende prozentuale Hemmung der spezifischen Bindung des Liganden (³H)-(+)-MK801 in Gegenwart von je 10 µmol/l der jeweiligen erfindungsgemäßen Verbindung dient als Maß für die Affinität dieser Verbindung zu der (+)-MK801-Bindungsstelle des ionotropen NMDA-Rezeptors. Die Affinität sind in Tabelle 2 als Mittetwerte von Doppelbestimmungen an ausgewählten Beispielen angegeben:

**Tabelle 2**

| **Beispiel** | **% Hemmung** |
|---|---|
| 140 | 44 |
| 141 | 45 |
| 142 | 75 |
| 143 | 49 |
| 144 | 45 |

Für die Bestimmung der Hemmung des Nucleosid-Transport-Proteins wurden folgende Versuchsbedingungen gewählt:
100µl der Substanzlösung in wäßriger Lösung mit DMSO als Lösungsvermittler wurden mit 100µl [³H]NBI (N⁶-Benzyladenosin)1.5 nM, 100µl Puffer (50 mM Tris.HCl,pH 7.4) und 100µl wäßriger Suspension von Erythrocytenmembran 30 min bei 25°C inkubiert. Nach der Inkubation wurde das Testgemisch abfiltriert (Whatman GF/C Filter, mit 50 mM Tris.HCl nachgewaschen). Die Filter wurden in Röhrchen überführt, mit 3,5 ml Scintillations-Fluid versetzt und nach zwei Stunden im β-Counter gemessen. Die Meßergebnisse (K_{I}-Wert bei 10 µM bzw. % Verdrängung bei 10 µM) sind in Tabelle 3 wiedergegeben.

**Tabelle 3**

| **Beispiel Nr.** | **Ki-Wert [µM]** | **% Verdrängung bei 10 µM** |
|---|---|---|
| **39** | | 40 |
| **53** | 0.6 | |
| **54** | 1,3 | |
| **55** | 0,4 | |
| **56** | | 43 |
| **64** | 0,6 | |
| **111** | 0.3 | |
| **145** | 0.4 | |
| **153** | | 50 |
| **154** | | 41 |

Das Dihydrochlorid von Beispiel 147 (hergestellt in zu den Beispielen 145 und 152 analoger Weise aus Beispiel 147) wurde auf seine Affinität gegenüber dem rekombinanten Human-Adensosin-A₃-Rezeptor in einem Verdrängungsassay (C. A. Salvatore et al., *Proc. Natl. Acad. Sci. USA* (1993), Vol. 90, 10365-10369) untersucht. Bei einer Ligandenkonzentration von 0,1 nM [¹²⁵I]AB-MECA (N⁶-(4-Amino-3-[¹²⁵I]iodbenzyladenosin) betrug die Verdrängung bei einer Konzentration von 3 µM 93 %.

### Pharmazeutische Formulierung eines erfindungsgemäßen Arzneimittels

1 g des Hydrochlorids von 3-Phenylazo-7-phenylsulfanyl-5-pyridin-2-yl-3,5,6,7-tetrahydro-pyrazolo[1,5-a]pyrimidin-2-ol wurde in 1 I Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur (I A) oder (I B), 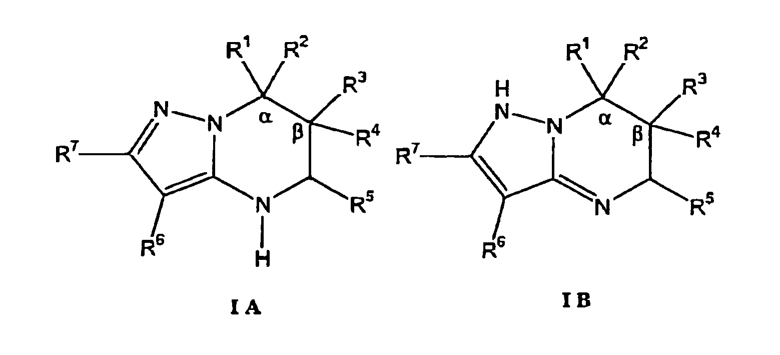 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl', -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴
W bildet, wobei W α'-CH=CH-CH₂-β', α'-CH=CH-CH₂₋CH₂-β',α'-O-(CH₂)ₘ-β' mit m = 2, 3, 4 oder 5, 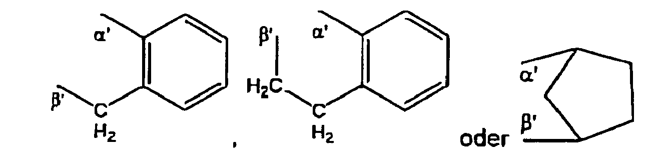 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnete** Ende von W mit dem mit β **gekennzeichneten** Atom der Verbindung der allgemeinen Struktur (I A) oder (I B) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Buiyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, CI, Br, I, -CN, -NC, -NH₂, -NO₂, -SH, -OH, -CO₂H und -CO₂Alkyl, Aryl' oder
-(CH₂)ₖ-Aryl', wobei k =1,2,3 oder 4 ist, Heterocyclyl oder C(=O)R¹¹ bedeutet;
R⁶ H, Methyl, Ethyl, -CN, Fluor, Chlor, Brom, lod, -C(=O)R¹⁷ oder -N=N-Aryl¹ bedeutet;
R⁷ H, Aryl¹, OR¹⁸, S(O)_{q}R¹⁹, wobei q = 0, 1 oder 2, oder unsubstituiertes Methyl, Ethyl, n-Propyf, 2-Propyl, n-Butyl, iso-Butyl, sek.-Butyl, tert-Butyl, n-Amyl, iso-Amyl, sek.-Amyl, n-Hexyl, iso-Hexyl oder sek.-Hexyl bedeutet,
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁷ OR²⁶ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁶ H, Methyl, Ethyl, n-Prvpyl, 2-Prvpyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet; und
Heterocyclyl Furan-2-yl, Furan-3-yl, Thien-2-yl, Thien-3-yl, Pyridin-2-yl-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 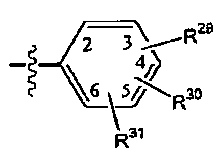 steht:
Aryl² für 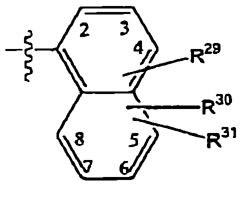 steht;
Aryl³ für 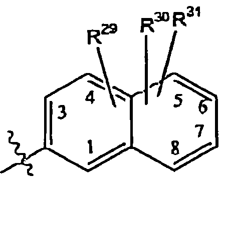 steht;
R²⁹, R³⁰ und R³¹ unabhängig voneinander H, C₁₋₆-[Alk], C₃₋₈₋[Cycloalk], -(C₁₋₆-Alkyl)-C₃₋₈-[Cycloalk], [Ar], -(C₁₋₆₋Alkyl)-[Ar], [Het], -(C₁₋₆-Alkyl)-[Het], F, Cl, Br, I, -CN, -NC, -OR³², -SR³³, -NO, -NO₂, NH₂, NHR³⁴, NR³⁵R³⁶, -N-OH, -N-OC₁₋₆-[Alk], -NHNH₂, -N=N-[Ar], -(C=O)R³⁷, 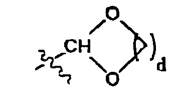 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)_{g}- mit g = 4 oder 5 stehen;
R³⁷ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar] bedeutet; und
R³⁹ H, C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht,
wobei die Verbindungen
4,5,6,7-Tetrahydro-2-methyl-5,7-diphenyl-pyrazolo[1,5-a]pyrimidin,
4,5,6,7-Tetrahydro-2,5-dimethyl-7-phenyl-pyrazolv[1,5-a]pyrimidin,
4.5,6,7-Tetrahydro-5, 7-dimethyl-3-phenyl-pyrazolo[1,5-a]pyrimidin,
4,5,6,7-Tetrahydro-2,5,7-trimethyl-pyrazolo[1,5-a]pyrimidin,
4,5,6,7-Tetrahydro-5,7-dimethyl-2-phenyl-pyrazolo[1,5-a]pyrimidin,
4,5,6,7-Tetrahydro-2-methyl-5,7-di-n-propyl-pyrazolo[1,5-a]pyrimidin-3-carbonitril,
4,5,6,7-Tetrahydro-5-methyl-7-[3-(trifluormethyl)-phenyl]pyrazolo[1,5-a]pyrimidin-3-carbonitril,
7-[4-(Chlor)-phenyl]-4,5,6,7-tetrahydro-5-methyl-pyrazolo[1,5-a]pyrimidin-3-carbonitril,
7-[3-(Chlor)-phenyl]-4,5,6,7-tetrahydro-5-methyl-pyrazolo[1,5-a]pyrimidin-3-carbonitril,
ausgenommen sind.

2. Verbindung nach Anspruch 1, **dadurch gekennzeichnet, daß**
R¹ und R² unabhängig voneinander H, O-R⁹, S-R¹⁰, unsubstituiertes oder einfach substituiertes oder mehrfach gleich oder verschieden substituiertes Methyl, Ethyl, n-Propyl, 2-Propyl, n-Butyl, tert.-Butyl oder n-Hexyl, wobei die Substituenten ausgewählt sind aus der Gruppe bestehend aus F, Cl, Br, I, -CN, -NC, -NH₂, -NO₂, -SH und -OH; Aryl' oder -CH₂-Aryl', wobei die Aryl'-Substituenten R²⁹, R³⁰ und R³¹ unabhängig voneinander H, Methyl, Ethyl, 2-Propyl, n-Butyl, tert.-Butyl, n-Hexyl, F, Cl, Br, I, OH, O-Methyl, O-Ethyl sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder Methyl, Ethyl, n-Propyl, 2-Propyl, n-Butyl, tert.-Butyl oder n-Hexyl bedeutet,
R³ und R⁴ H, Methyl oder Aryl¹ bedeutet, wobei die Aryl¹⁻Substiuenten R²⁹, R³⁰ und R³¹ unabhängig voneinander H, Methyl oder O-Methyl sind,
wobei mindestens einer der Reste R³ und R⁴ H ist,
oder
einer der Reste R¹ und R² zusammen mit einem der Reste R³ und R⁴
W bildet, wobei W α'-CH=CH-CH₂-β', α'-CH=CH-CH₂₋CH₂-β', α'-O-(CH₂)ₘ-β' mit m = 2, 3, 4 oder 5, 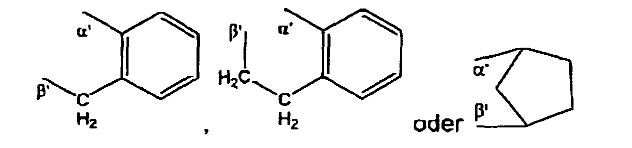 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnet**e Ende von W mit dem mit β **gekennzeichneten** Atom der Verbindung der allgemeinen Struktur (I A) oder (I B) verbunden ist, der andere Rest von R¹ und R² und der andere Rest von R³ und R⁴ jeweils H bedeuten;
R⁵ Methyl, Ethyl, n-Propyl, 2-Propyl, n-Butyl, iso-Butyl, sek.-Butyl, tert.-Butyl, -(CH₂)₄-OH, Cyclopropyl, wobei Cyclopropyl unsubstituiert oder einfach mit C(=O)OH, C(=O)O-Methyl oder C(=O)O-Ethyl substituiert ist, Cyclopentyl, Cyclohexyl, Aryl¹ oder -(CH₂)ₖ-Aryl¹, wobei die Aryl'-Substituenten R²⁹, R³⁰ und R³¹ unabhängig voneinander H, -OH, -O-Methyl, O-C₆H₅, CH₃, CF₃ oder C(=O)OH sind und k = 1 oder 2 ist, Heterocyclyl oder C(=O)R¹¹ bedeutet,
R⁶ H, -CN, Brom, -C(=O)R¹⁷ oder -N=N-Phenyl bedeutet;
R⁷ H, Aryl¹ mit R²⁹, R³⁰ und R³¹ gleich H, OH, S(O)qR¹⁹,
wobei q = 0 oder 2 ist, oder Methyl, Ethyl, n-Propyl, 2-Propyl, n-Butyl, iso-Butyl, sek.-Butyl oder tert.-Butyl bedeutet,
R⁹ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclopentyl oder Cyclohexyl bedeutet oder -[(CH₂)ᵣ-O]ₛ-H mit r = 1, 2 oder 3 und s = 1 oder 2 bedeutet;
R¹⁰ Aryl¹ bedeutet;
R¹¹ Aryl¹ mit R²⁹, R³⁰ und R³¹ gleich H oder OR²⁵ bedeutet;
R¹⁷ OR²⁶ bedeutet;
R¹⁹ Methyl oder Aryl¹ bedeutet, wobei einer der Aryl¹⁻Substiuenten R²⁹, R³⁰ und R³¹ gleich H oder -NO₂ ist und die beiden anderen Aryl¹-Substituenten von R²⁹, R³⁰ und R³¹ H sind,
R²⁵ H, Methyl oder Ethyl bedeutet, wobei R²⁵ nicht H bedeutet, wenn zugleich R¹ Aryl und R² Alkyl bedeuten;
R²⁶ H, Methyl oder Ethyl bedeutet; und
Heterocyclyl Furan-2-yl, Furan-3-yl, Thien-2-yl, Thien-3-yl, Pyridin-2-y-, Pyridin-3-yl oder Pyridin-4-yl bedeutet, wobei Furanyl, Thienyl und Pyridinyl jeweils unsubstituiert oder einfach substituiert oder mehrfach gleich oder verschieden mit -NO₂, -CH₃ oder C(=O)OH substituiert sind.

3. Verbindung nach Anspruch 1 oder 2, **dadurch gekennzeichnet, daß**
R¹ und R² unabhängig voneinander H, O-CH₂-CH₂-OH, O-Cyclohexyl, S-Phenyl, Methyl, Phenyl, 3-Fluor-phenyl, 3-Brom-phenyl, 4-Brom-phenyl, 4-Chlor-phenyl, 4-Fluor-phenyl, 3-Methyl-phenyl, 4-Hydroxy-phenyl, 4-Methoxy-phenyl, 2,4-Dimethyl-phenyl, 3,4-Dimethoxy-phenyl, 2,3,4-Trimethoxyphenyl, 2-Naphthyl oder -CH₂₋Phenyl bedeuten,
R³ und R⁴ H, Methyl oder 4-Methoxy-phenyl bedeuten, wobei mindestens einer der Reste R³ und R⁴ H ist,
oder
einer der Reste R¹ und R² zusammen mit einem der Reste R³ und R⁴
W bildet, wobei W α'-CH=CH-CH₂-β', α'-CH=CH-CH₂₋CH₂-β', α'-O-(CH₂)ₘ-β' mit m = 2, 3, 4 oder 5, 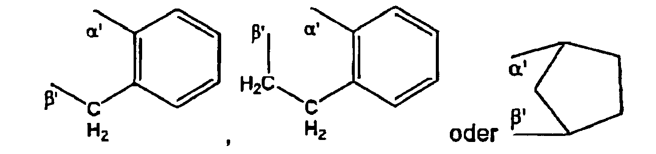 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnete** Ende von W mit dem mit β **gekennzeichneten** Atom der Verbindung der allgemeinen Struktur (I A) oder (I B) verbunden ist, der andere Rest von R¹ und R² und der andere Rest von R³ und R⁴ jeweils H bedeuten;
R⁵ n-Propyl, n-Butyl, tert.-Butyl, -(CH₂)₄-OH, Cyclopropyl, Cycloprop-2-yl-1-carbonsäureethylester, Cyclohexyl, 4-Trifluorphenyl, 4-Phenoxy-phenyl, 2-Hydroxy-3- methoxy-phenyl, 4-Hydroxy-3-methoxy-phenyl, 3-Carboxy-2-hydroxy-phenyl, -(CH₂)₂-Phenyl, 5-Carboxyfuran-2-yl, 5-Methyl-furan-2-yl, 5-Nitro-furan-2-yl, 5-Nitro-thien-2-yl, Pyridin-2-yl, Pyridin-3-yl, C(=O)Phenyl, C(=O)OH oder C(=O)OEthyl bedeutet, wobei R⁵ nicht C(=O)OH bedeutet, wenn zugleich R¹ Aryl und R² Alkyl bedeuten;
R⁶ H, -CN, Brom, -C(=O)OH, -C(=O)OEthyl oder -N=N-Phenyl bedeutet; und
R⁷ H, Phenyl, OH, -S-Methyl, -SO₂-(4-nitrophenyl) oder tert.-Butyl bedeutet.

4. Verbindung nach Anspruch 1, **dadurch gekennzeichnet, daß** die Verbindung ausgewählt ist aus:
• 3-Brom-5-(5-nitro-furan-2-yl)-7-m-tolyl-tetrahydro-pyrazolo[1,5-a]pyrimidin
• 3-Brom-7-(4-fluor-phenyl)-7-methyl-5-(5-nitro-furan-2-yl)-tetrahydro-pyrazolo[1,5-a]pyrimidin
• 3-Brom-7-naphthalin-2-yl-5-(5-nitro-furan-2-yl)-tetrahydro-pyrazolo[1,5-a]pyrimidin
• 2-(3-Brom-7-m-tolyl-tetrahydro-pyrazolo[1,5-a]pyrimidin-5-yl)-cyclopropancarbonsäureethylester
• 2-[3-Brom-7-(4-brom-phenyl)-tetrahydro-pyrazolo[1,5-a]pyrimidin-5-yl]-cyclopropancarbonsäureethylester
• 2-(3-Brom-7-naphthalin-2-yl-tetrahydro-pyrazolo[1,5-a]pyrimidin-5-yl)-cyclopropancarbonsäureethylester
• 3-Brom-7-(4-fluor-phenyl)-7-methyl-5-(5-methyl-furan-2-yl)-tetrahydro-pyrazolo[1,5-a]pyrimidin
• 3-Brom-7-(3,4-dimethoxy-phenyl)-tetrahydro-pyrazolo[1,5-a]pyrimidin-5-carbonsäureethylester
• 3-Brom-7-(4-methoxy-phenyl)-tetrahydro-pyrazolo[1,5-a]pyrimidin-5-carbonsäureethylester
• 3-Brom-7-(4-methoxy-phenyl)-tetrahydro-pyrazolo[1,5-a]pyrimidin-5-carbonsäure
• 3-Brom-7-(2,4-dimethyl-phenyl)-5-(5-nitro-furan-2-yl)-tetrahydro-pyrazolo[1,5-a]pyrimidin
• 3-Brom-7-(4-methoxy-phenyl)-5-(5-nitro-furan-2-yl)-tetrahydro-pyrazolo[1.5-a]pyrimidin
• 5,5a,6,8a-Tetrahydro-3H-1,4,8b-triaza-as-indacen-3,5-dicarbonsäurediethylester, 5,5a,6,8a-Tetrahydro-4H-1,4,8b-triaza-as-indacen-3,5-dicarbonsäurediethylester;
• 2-Hydroxy-3-phenylazo-5,5a,6,8a-tetrahydro-3H-1,4,8b-triaza-as-indacen-5-carbonsäureethylester; 2-Hydroxy-3-phenylazo-5,5a,6,8a-tetrahydro-4H-1,4,8b-triaza-as-indacen-5-carbonsäureethylester
• 2-tert-Butyl-5,5a,6,8a-tetrahydro-3H-1,4,8b-triaza-as-indacen-5-carbonsäureethylester; 2-tert-Butyl-5,5a,6,8a-tetrahydro-3H-1,4,8b-triaza-as-indacen-5-carbonsäureethylester
• 3-Brom-2-phenyl-5,5a,6,8a-tetrahydro-3H-1,4,8b-triaza-as-indacen-5-carbonsäureethylester; 3-Brom-2-phenyl-5,5a,6,8a-tetrahydro-4H-1,4,8b-triaza-as-indacen-5-carbonsäureethylester
• 7-(2,3,4-Trimethoxy-phenyl)-tetrahydro-pyrazolo[1,5-a]pyrimidin-3,5-dicarbonsäurediethylester
• 3-Cyano-2-methylsulfanyl-7-(2,3,4-trimethoxy-phenyl)-tetrahydro-pyrazolo[1,5-a]pyrimidin-5-carbonsäureethylester
• 2-Hydroxy-7-(4-hydroxy-phenyl)-6-methyl-3-phenylazo-tetrahydro-pyrazolo[1,5-a]pyrimidin-5-carbonsäureethylester
• 3-Brom-7-(4-hydroxy-phenyl)-6-methyl-2-phenyl-tetrahydro-pyrazolo[1,5-a]pyrimidin-5-carbonsäureethylester
• 5,5a,6,10b-Tetrahydro-3H-1,4,10c-triaza-cyclopenta[c]fluoren-3,5-dicarbonsäurediethylester; 5,5a,6,10b-Tetrahydro-4H-1,4,10c-triaza-cyclopenta[c]fluoren-3,5-dicarbonsäurediethylester
• 2-Hydroxy-3-phenylazo-5,5a,6,10b-tetrahydro-3H-1,4,10c-triaza-cyclopenta[c]fluoren-5-carbonsäureethylester; 2-Hydroxy-3-phenylazo-5,5a,6,10b-tetrahydro-4H-1,4,10c-triaza-cyclopenta[c]fluoren-5-carbonsäureethylester
• 7-Phenylsulfanyl-tetrahydro-pyrazolo[1,5-a]pyrimidin-3,5-dicarbonsäurediethyl ester
• 3-Cyano-2-methylsulfanyl-7-phenylsulfanyl-tetrahydro-pyrazolo[1,5-a]pyrimidin-5-carbonsäureethylester
• 3-Cyano-2-methylsulfanyl-7-(2,3,4-trimethoxy-phenyl)-tetrahydro-pyrazolo[1,5-a]pyrimidin-5-carbonsäure
• 7-Phenylsulfanyl-tetrahydro-pyrazolo[1,5-a]pyrimidin-3,5-dicarbonsäure3-ethyl ester
• 3-Cyano-7-(2,4-dimethyl-phenyl)-2-methylsulfanyl-tetrahydro-pyrazolo[1,5-a]pyrimidin-5-carbonsäureethylester
• 3-Cyano-7-(2,4-dimethyl-phenyl)-tetrahydro-pyrazolo[1,5-a]pyrimidin-5-carbonsäureethylester
• 7-(2,4-Dimethyl-phenyl)-tetrahydro-pyrazolo[1,5-a]pyrimidin-3,5-dicarbonsäure3-ethyl ester
• 3-Brom-7-(2,4-dimethyl-phenyl)-2-phenyl-tetrahydro-pyrazolo[1,5-a]pyrimidin-5-carbonsäure
• 3-Cyano-7-(2,4-dimethyl-phenyl)-2-methylsulfanyl-tetrahydro-pyrazolo[1,5-a]pyrimidin-5-carbonsäure
• 3-Cyano-7-(2,4-dimethyl-phenyl)-tetrahydro-pyrazolo[1,5-a]pyrimidin-5-carbonsäure
• 3-Cyano-7-(3,4-dimethoxy-phenyl)-2-methylsulfanyl-tetrahydro-pyrazolo[1,5-a]pyrimidin-5-carbonsäure
• 7-(2,4-Dimethyl-phenyl)-5-(5-nitro-furan-2-yl)-tetrahydro-pyrazolo[1,5-a]pyrimidin-3-carbonsäureethylester
• 7-(2,4-Dimethyl-phenyi)-5-(5-nitro-furan-2-yl)-3-phenylazo-tetrahydro-pyrazolo[1,5-a]pyrimidin-2-ol
• 3-Brom-7-(2,4-dimethyl-phenyl)-5-(5-nitro-furan-2-yl)-2-phenyl-tetrahydro-pyrazolo[1,5-a]pyrimidin
• 7-(2,4-Dimethyl-phenyl)-2-methylsulfanyl-5-(5-nitro-furan-2-yl)-tetrahydro-pyrazolo[1,5-a]pyrimidin-3-carbonitril
• 7-(2,4-Dimethyl-phenyl)-5-(5-nitro-furan-2-yl)-tetrahydro-pyrazolo[1,5-a]pyrimidin-3-carbonitril
• 3-Brom-7-(3,4-dimethoxy-phenyl)-5-(5-nitro-furan-2-yl)-2-phenyl-tetrahydro-pyrazolo[1,5-a]pyrimidin
• 7-(4-Methoxy-phenyl)-2-methylsulfanyl-5-(5-nitro-furan-2-yl)-tetrahydro-pyrazolo[1,5-a]pyrimidin-3-carbonitril
• 7-(2,4-Dimethyl-phenyl)-5-(2-ethoxycarbonyl-cyclopropyl)-tetrahydro-pyrazolo[1,5-a]pyrimidin-3-carbonsäureethylester
• 2-[7-(2,4-Dimethyl-phenyl)-2-hydroxy-3-phenylazo-tetrahydro-pyrazolo[1,5-a]pyrimidin-5-yl]-cyclopropancarbonsäureethylester
• 2-[2-tert-Butyl-7-(2,4-dimethyl-phenyl)-tetrahydro-pyrazolo[1,5-a]pyrimidin-5-yl]-cyclopropancarbonsäureethylester
• 2-[3-Brom-7-(2,4-dimethyl-phenyl)-2-phenyl-tetrahydro-pyrazolo[1,5-a]pyrimidin-5-yl]-cyclopropancarbonsäureethylester
• 2-[3-Cyano-7-(2,4-dimethyl-phenyl)-2-methylsulfanyl-tetrahydro-pyrazolo[1,5-a]pyrimidin-5-yl]-cyclopropancarbonsäureethylester
• 5-(2-Ethoxycarbonyl-cyclopropyl)-7-(3-fluor-phenyl)-tetrahydro-pyrazolo[1,5-a]pyrimidin-3-carbonsäureethylester
• 2-[3-Brom-7-(3-brom-phenyl)-2-phenyl-tetrahydro-pyrazolo[1,5-a]pyrimidin-5-yl]-cyclopropancarbonsäureethylester
• 2-[7-(3-Brom-phenyl)-3-cyano-2-methylsulfanyl-tetrahydro-pyrazolo[1,5-a]pyrimidin-5-yl]-cyclopropancarbonsäureethylester
• 7-(2,4-Dimethyl-phenyl)-5-(5-nitro-thiophen-2-yl)-3-phenylazo-tetrahydro-pyrazolo[1,5-a]pyrimidin-2-ol
• 7-(2,4-Dimethyl-phenyl)-2-methylsulfanyl-5-(5-nitro-thiophen-2-yl)-tetrahydro-pyrazolo[1,5-a]pyrimidin-3-carbonitril
• 7-(2,4-Dimethyl-phenyl)-5-(5-nitro-thiophen-2-yl)-tetrahydro-pyrazolo[1,5-a]pyrimidin-3-carbonitril
• 7-(3,4-Dimethoxy-phenyl)-5-(5-nitro-thiophen-2-yl)-tetrahydro-pyrazolo[1,5-a]pyrimidin-3-carbonsäureethylester
• 7-(3,4-Dimethoxy-phenyl)-5-(5-nitro-thiophen-2-yl)-3-phenylazo-tetrahydro-pyrazolo[1,5-a]pyrimidin-2-ol
• 3-Brom-7-(3,4-dimethoxy-phenyl)-5-(5-nitro-thiophen-2-yl)-2-phenyl-tetrahydro-pyrazolo[1,5-a]pyrimidin
• 7-(3,4-Dimethoxy-phenyl)-2-methylsulfanyl-5-(5-nitro-thiophen-2-yl)-tetrahydro-pyrazolo[1,5-a]pyrimidin-3-carbonitril
• 7-(3,4-Dimethoxy-phenyl)-5-(5-nitro-thiophen-2-yl)-tetrahydro-pyrazolo[1,5-a]pyrimidin-3-carbonitril
• 7-(4-Methoxy-phenyl)-5-(5-nitro-thiophen-2-yl)-tetrahydro-pyrazolo[1,5-a]pyrimid in-3-carbonsäureethylester
• 5-[3-Brom-7-(4-methoxy-phenyl)-2-phenyl-tetrahydro-pyrazolo[1,5-a]pyrimidin-5-yl]-furan-2-carbonsäure
• 5-Benzoyl-7-(2,4-dimethyl-phenyl)-tetrahydro-pyrazolo[1,5-a]pyrimidin-3-carbonsäureethylester
• 5-Benzoyl-7-(2,4-dimethyl-phenyl)-2-methylsulfanyl-tetrahydro-pyrazolo[1,5-a]pyrimidin-3-carbonitril
• 5-Benzoyl-7-(2,4-dimethyl-phenyl)-tetrahydro-pyrazolo[1,5-a]pyrimidin-3-carbonitril
• 5-Benzoyl-7-(3,4-dimethoxy-phenyl)-tetrahydro-pyrazolo[1,5-a]pyrimidin-3-carbonsäureethylester
• [3-Brom-7-(3,4-dimethoxy-phenyl)-2-phenyl-tetrahydro-pyrazolo[1,5-a]pyrimidin-5-yl]-phenyl-methanon
• 5-Benzoyl-7-(3,4-dimethoxy-phenyl)-2-methylsulfanyl-tetrahydro-pyrazolo[1,5-a]pyrimidin-3-carbonitril
• 5-Benzoyl-7-(3,4-dimethoxy-phenyl)-tetrahydro-pyrazolo[1,5-a]pyrimidin-3-carbonitril
• 5-Benzoyl-7-(4-methoxy phenyl)-tetrahydro-pyrazolo[1,5-a]pyrimidin-3-carbonsäureethylester
• 5-Benzoyl-7-(4-methoxy-phenyl)-2-methylsulfanyl-tetrahydro-pyrazolo[1,5-a]pyrimidin-3-carbonitril
• 5-Benzoyl-7-(4-methoxy-phenyl)-tetrahydro-pyrazolo[1,5-a]pyrimidin-3-carbonitril
• 5-Benzoyl-7-(3-fluor-phenyl)-tetrahydro-pyrazolo[1,5-a]pyrimidin-3-carbonsäureethylester
• [3-Brom-7-(3-fluor-phenyl)-2-phenyl-tetrahydro-pyrazolo[1,5-a]pyrimidin-5-yl]-phenyl-methanon
• [3-Brom-7-(3-brom-phenyl)-2-phenyl-tetrahydro-pyrazolo[1,5-a]pyrimidin-5-yl]-phenyl-methanon
• 7-(2,4-Dimethyl-phenyl)-5-(4-phenoxy-phenyl)-tetrahydro-pyrazolo[1,5-a]pyrimidin-3-carbonsäureethylester
• 3-Brom-7-(2,4-dimethyl-phenyl)-5-(4-phenoxy-phenyl)-2-phenyl-tetrahydro-pyrazolo[1,5-a]pyrimidin
• 7-(2,4-Dimethyl-phenyl)-2-methylsulfanyl-5-(4-phenoxy-phenyl)-tetrahydro-pyrazolo[1,5-a]pyrimidin-3-carbonitril
• 7-(2,4-Dimethyl-phenyl)-5-(4-phenoxy-phenyl)-tetrahydro-pyrazolo[1,5-a]pyrimidin-3-carbonitril
• 7-(3,4-Dimethoxy-phenyl)-5-(4-phenoxy-phenyl)-tetrahydro-pyrazolo[1,5-a]pyrimidin-3-carbonsäureethylester
• 7-(3,4-Dimethoxy-phenyl)-2-methylsulfanyl-5-(4-phenoxy-phenyl)-tetrahydro-pyrazolo[1,5-a]pyrimidin-3-carbonitril
• 3-[3-Cyano-7-(4-hydroxy-phenyl)-6-methyl-tetrahydro-pyrazolo[1,5-a]pyrimidin-5-yl]-2-hydroxy-benzvesäure
• 3-(3-Cyano-5,5a,6,10b-tetrahydro-3H-1,4,10c-triaza-cyclopenta[c]fluoren-5-yl)-2-hydroxy-benzvesäure; 3-(3-Cyano-5,5a,6,10b-tetrahydro-4H-1,4,10c-thaza-cyclopenta[c]fluoren-5-yl)-2-hydruxy-benzoesäure
• 3-(3-Cyano-7-phenylsulfanyl-tetrahydro-pyrazolo[1,5-a]pyrimidin-5-yl)-2-hydroxy-benzoesäure
• 3-[2-tert-Butyl-7-(4-chlor-phenyl)-7-methyl-tetrahydro-pyrazolo[1,5-a]pyrimidin-5-yl]-2-hydroxy-benzoesäure
• 5-(4-Hydroxy-3-methoxy-phenyl)-7-(4-hydroxy-phenyl)-6-methyl-tetrahydro-pyrazolo[1,5-a]pyrimidin-3-carbonsäureethylester
• 5-(4-Hydroxy-3-methoxy-phenyl)-7-(4-hydroxy-phenyl)-6-methyl-tetrahydro-pyrazolo[1,5-a]pyrimidin-3-carbonitril
• 5-(4-Hydroxy-3-methoxy-phenyl)-5,5a,6,10b-tetrahydro-3H-1,4,10c-triaza-cyclopenta[c]fluoren-3-carbonsäureethylester; 5-(4-Hydroxy-3-methoxy-phenyl)-5,5a,6,10b-tetrahydro-4H-1,4,10c-triaza-cyclopenta[c]fluoren-3-carbonsäureethylester
• 4-(2-tert-Butyl-5,5a,6,10b-tetrahydro-3H-1,4,10c-triaza-cyclopenta[c]fluoren-5-yl)-2-methoxy-phenol; 4-(2-tert-Butyl-5,5a,6,10b-tetrahydro-4H-1,4,10c-triaza-cyclopenta[c]fluoren-5-yl)-2-methoxy-phenol
• 5-(4-Hydroxy-3-methoxy-phenyl)-2-methylsulfanyl-5,5a,6,10b-tetrahydro-3H-1,4,10c-triaza-cyclopenta[c]fluoren-3-carbonitril; 5-(4-Hydroxy-3-methoxy-phenyl)-2-methylsulfanyl-5,5a,6,10b-tetrahydro-3H-1,4,10c-triaza-cyclopenta[c]fluoren-3-carbonitril
• 5-(4-Hydroxy-3-methoxy-phenyl)-7-phenylsulfanyl-tetrahydro-pyrazolo[1,5-a]pyrimidin-3-carbonsäureethylester
• 4-(2-tert-Butyl-7-phenylsulfanyl-tetrahydro-pyrazolo[1,5-a]pyrimidin-5-yl)-2-methoxy-phenol
• 4-(3-Brom-2-phenyl-7-phenylsulfanyl-tetrahydro-pyrazolo[1,5-a]pyrimidin-5-yl)-2-methoxy-phenol
• 5-(2-Hydroxy-3-methoxy-phenyl)-7-phenylsulfanyl-tetrahydro-pyrazolo[1,5-a]pyrimidin-3-carbonsäureethylester
• 7-(4-Chlor-phenyl)-5-(2-hydroxy-3-methoxy-phenyl)-7-methyl-tetrahydro-pyrazolo[1,5-a]pyrimidin-3-carbonsäureethylester
• 5-(4-Hydroxy-butyl)-5,5a,6,10b-tetrahydro-3H-1,4,10c-triaza-cyclopenta[c]fluoren-3-carbonitril; 5-(4-Hydroxy-butyl)-5,5a,6,10b-tetrahydro-4H-1,4,10c-triaza-cyclopenta[c]fluoren-3-carbonitril
• 5-(4-Hydroxy-butyl)-2-methylsulfanyl-7-phenylsulfanyl-tetrahydro-pyrazolo[1,5-a]pyrimidin-3-carbonitril
• 5-(4-Hydroxy-butyl)-7-phenylsulfanyl-tetrahydro-pyrazolo[1.5-a]pyrimidin-3-carbonitril
• 7-(4-Chlor-phenyl)-5-(4-hydroxy-butyl)-7-methyl-tetrahydro-pyrazolo[1,5-a]pyrimidin-3-carbonitril
• 5-Butyl-2-methylsulfanyl-5,5a,6,10b-tetrahydro-3H-1,4,10c-triaza-cyclopenta[c]fluoren-3-carbonitril; 5-Butyl-2-methylsulfanyl-5,5a,6,10b-tetrahydro-4H-1,4,10c-triaza-cyclopenta[c]fluoren-3-carbonitril
• 5-Butyl-2-methylsulfanyl-7-phenylsulfanyl-tetrahydro-pyrazolo[1,5-a]pyrimidin-3-carbonitril
• 5-Butyl-7-phenylsulfanyl-tetrahydro-pyrazvlv[1,5-a]pyrimidin-3-carbonitril
• 5-Butyl-7-(4-chlor-phenyl)-7-methyl-2-methyisuffanyl-tetrahydro-pyrazolo[1,5-a]pyrimidin-3-carbonitril
• 5-Cyclopropyl-7-(2,4-dimethyl-phenyl)-3-phenylazo-tetrahydro-pyrazolo[1,5-a]pyrimidin-2-ol
• 2-tert-Butyl-5-cyclopropyl-7-(2,4-dimethyl-phenyl)-tetrahydro-pyrazolo[1,5-a]pyrimidin
• 5-Cyclopropyl-7-(2,4-dimethyl-phenyl)-2-methylsulfanyl-tetrahydro-pyrazolo[1,5-a]pyrimidin-3-carbonitril
• 2-tert-Butyl-5-cyclopropyl-7-(3,4-dimethoxy-phenyl)-tetrahydro-pyrazolo[1,5-a]pyrimidin
• 3-Brom-5-cyclopropyl-7-(3,4-dimethoxy-phenyl)-2-phenyl-tetrahydro-pyrazolo[1,5-a]pyrimidin
• 5-Cyclopropyl-7-(4-methoxy-phenyl)-tetrahydro-pyrazolo[1,5-a]pyrimidin-3-carbonsäureethylester
• 5-Cyclopropyl-3,5,5a,6,7,11b-hexahydro-1,4,11c-triaza-cyclopenta[c]phenanthrene-3-carbonitril
• 7-(2,4-Dimethyl-phenyl)-5-pyridin-2-yl-tetrahydro-pyrazolo[1,5-a]pyrimidin-3-carbonsäureethylester
• 7-(2,4-Dimethyl-phenyl)-3-phenylazo-5-pyridin-2-yl-tetrahydro-pyrazolo[1,5-a]pyrimidin-2-ol
• 3-Brom-7-(2,4-dimethyl-phenyl)-2-phenyl-5-pyridin-2-yl-tetrahydro-pyrazolo[1,5-a]pyrimidin
• 7-(2,4-Dimethyl-phenyl)-2-methylsulfanyl-5-pyridin-2-yl-tetrahydro-pyrazolo[1,5-a]pyrimidin-3-carbonitril
• 7-(3,4-Dimethoxy-pheriyl)-2-methylsuffanyl-5-phenethyl-tetrahydro-pyrazolo[1,5-a]pyrimidin-3-carbonitril
• 7-(3,4-Dimethoxy-phenyl)-5-phenethyl-tetrahydro-pyrazolo[1,5-a]pyrimidin-3-carbonitril
• 5-Cyclopropyl-7-(2-hydroxy-ethoxy)-tetrahydro-pyrazolo[1,5-a]pyrimidin-3-carbonsäureethylester
• 2-(2-tert-Butyl-5-cyclopropyl-tetrahydro-pyrazolo[1,5-a]pyrimidin-7-yloxy)-ethanol
• 5-Cyclopropyl-3,5,5a,6,7,8a-hexahydro-8-oxa-1,4,8b-triaza-as-indacen-3-carbonsäureethylester; 5-Cyclopropyl-4,5,5a,6,7,8a-hexahydro-8-oxa-1,4,8b-triaza-as-indacen-3-carbonsäureethylester
• 5-Cyclopropyl-3-phenylazo-3,5,5a,6,7,8a-hexahydro-8-oxa-1,4,8b-triaza-as-indacen-2-ol; 5-Cyclopropyl-3-phenylazo-4,5,5a,6,7,8a-hexahydro-8-oxa-1,4,8b-triaza-as-indacen-2-ol
• 7-Cyclohexyloxy-5-cyclopropyl-tetrahydro-pyrazolo[1,5-a]pyrimidin-3-carbonsäureethylester
• 7-Cyclohexyloxy-5-cyclopropyl-2-methylsulfanyl-tetrahydro-pyrazolo[1,5-a]pyrimidin-3-carbonitril
• 7-(4-Chlor-phenyl)-5-cyclohexyl-tetrahydro-pyrazolo[1,5-a]pyrimidin-3-carbonitril
• 5-Cyclohexyl-7-(2-hydroxy-ethoxy)-tetrahydro-pyrazolo[1,5-a]pyrimidin-3-carbonsäureethylester
• 5-Cyclohexyl-3,5,5a,6,7,8a-hexahydro-8-oxa-1,4,8b-triaza-as-indacen-3-carbonsäureethylester; 5-Cyclohexyl-4,5,5a,6,7,8a-hexahydro-8-oxa-1,4,8b-triaza-as-indacen-3-carbonsäureethylester
• 5-Cyclohexyl-7-cyclohexyloxy-tetrahydro-pyrazolo[1,5-a]pyrimidin-3-carbonsäureethylester
• 7-(2,4-Dimethyl-phenyl)-3-phenylazo-5-propyl-tetrahydro-pyrazolo[1,5-a]pyrimidin-2-ol
• 7-(2,4-Dimethyl-phenyl)-2-methylsulfanyl-5-propyl-tetrahydro-pyrazolo[1,5-a]pyrimidin-3-carbonitril
• 5-tert-Butyl-7-(2,4-dimethyl-phenyl)-tetrahydro-pyrazolo[1,5-a]pyrimidin-3-carbonsäureethylester
• 2,5-Di-tert-butyl-7-(3,4-dimethoxy-phenyl)-tetrahydro-pyrazolo[1,5-a]pyrimidin
• 3-Brom-5-tert-butyl-7-(3,4-dimethoxy-phenyl)-2-phenyl-tetrahydro-pyrazolo[1,5-a]pyrimidin
• 2-[3-Cyano-6,7-bis-(4-methoxy-phenyl)-tetrahydro-pyrazolo[1,5-a]pyrirnidin-5-yl]-cyclopropancarbonsäureethylester
• 3-Cyano-6,7-bis-(4-methoxy-phenyl)-tetrahydro-pyrazolo[1,5-a]pyrimidin-5-carbonsäure
• 4-[3-Brom-6-methyl-2-phenyl-5-(4-trifluormethyl-phenyl)-tetrahydro-pyrazolo[1,5-a]pyrimidin-7-yl]-phenol
• 7-(4-Hydroxy-phenyl)-6-methyl-2-methylsuffanyl-5-(4-trifluormethyl-phenyl)-tetrahydro-pyrazolo[1,5-a]pyrimidin-3-carbonitril
• 7-(4-Hydroxy-phenyl)-6-methyl-5-(4-trifluormethyl-phenyl)-tetrahydro-pyrazolo[1,5-a]pyrimidin-3-carbonitril
• 7-Phenyl-3-phenylazo-5-pyridin-2-yl-3,5,6,7-tetrahydro-pyrazolo[15-a]pyrimidin-2-ol
• 7-Phenylsulfanyl-5-pyridin-2-yl-3,5,6,7-tetrahydro-pyrazolv[1,5-a]pyrimidin-3-carbonsäureethylester
• 3-Phenylazo-7-phenylsulfanyl-5-pyridin-2-yl-3,5,6,7-tetrahydro-pyrazolo[1,5-a]pyrimidin-2-ol
• 3-Brom-7-phenylsulfanyl-5-pyridin-2-yl-3,5,6,7-tetrahydro-pyrazolo[15-a]pyrimidin
• 7-Phenylsulfanyl-5-pyridin-2-yl-3,5,6,7-tetrahydro-pyrazolo[1,5-a]pyrimidin-3-carbonitril
• 7-(3,4-Dimethoxy-phenyl)-2-methylsulfanyl-5-pyridin-2-yl-tetrahydro-pyrazolo[1,5-a]pyrimidin-3-carbonitril
• 3-Brom-7-(3,4-dimethoxy-phenyl)-2-phenyl-5-pyridin-2-yl-tetrahydro-pyrazolo[1,5-a]pyrimidin
• 3-Brom-7-(3,4-dimethoxy-phenyl)-2-phenyl-tetrahydro-pyrazolo[1,5-a]pyrimidin-5-carbonsäureethylester
• 3-Brom-7-(3,4-dimethoxy-phenyl)-5-(5-nitro-furan-2-yl)-2-phenyl-tetrahydro-pyrazolo[1,5-a]pyrimidin
• 3-Cyano-7-(3,4-dimethoxy-phenyl)-2-methylsulfanyl-tetrahydro-pyrazolo[1,5-a]pyrimidin-5-carbonsäureethylester
• 3-Cyano-7-(3,4-dimethoxy-phenyl)-tetrahydro-pyrazolo[1,5-a]pyrimidin-5-carbonsäureethylester
• 3-Brom-7-(3,4-dimethoxy-phenyl)-2-phenyl-5-pyridin-2-yl-4,5,6,7-tetrahydro-pyrazolo[1,5-a]pyrimidin
• 7-(3,4-Dimethoxy-phenyl)-5-pyridin-2-yl-4,5,6,7-tetrahydro-pyrazolo[1,5-a]pyrimidin-3-carbonitril
• 7-(3,4-Dimethoxy-phenyl)-2-methylsulfanyl-5-(5-nitro-furan-2-yl)-tetrahydro-pyrazolo[1,5-a]pyrimidin-3-carbonitril
• 7-(3,4-Dimethoxy-phenyl)-5-pyridin-2-yl-4,5,6,7-tetrahydro-pyrazolo[1,5-a]pyrimidin-3-carbonsäureethylester.

5. Verfahren zur Herstellung von Verbindungen der allgemeinen Struktur (I A) und/oder (I B) sowie ihrer pharmazeutisch annehmbaren Salze 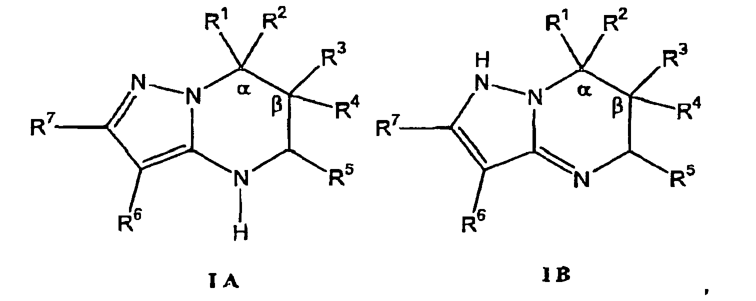 worin
R¹, R², R³, R⁴, R⁵, R⁶ und R⁷ wie in Anspruch 1 definiert sind, **dadurch gekennzeichnet, daß**
ein Pyrazolamin der allgemeinen Struktur (IIIA) oder (IIIB) 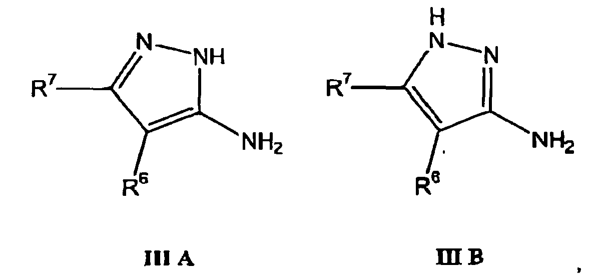 worin
R⁶ und R⁷ wie oben in diesem Anspruch definiert sind,
mit einem Aldehyd der allgemeinen Struktur (IV) 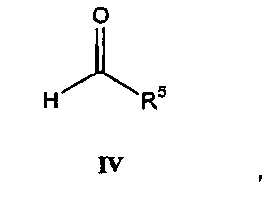 worin
R⁵ wie oben in diesem Anspruch definiert ist,
und einem Olefin der allgemeinen Struktur (V) 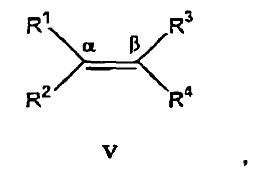 worin
R¹, R², R³ und R⁴ wie oben in diesem Anspruch definiert sind mit der Maßgabe, daß, wenn einer der Reste R¹ und R² zusammen mit einem der Reste R³ und R⁴ W bildet, das mit α' **gekennzeichnet**e Ende von W mit dem α-Kohlenstoffatom des Olefins der allgemeinen Struktur (V) und das mit β' **gekennzeichnet**e Ende von W mit dem β-Kohlenstoffatom des Olefins der allgemeinen Struktur (V) verbunden ist,
in Gegenwart einer Säure umgesetzt wird.

6. Verfahren nach Anspruch 5, **dadurch gekennzeichnet, daß** die Umsetzung des Heterocyclylamins der allgemeinen Struktur (III A) oder (III B) mit dem Aldehyd der allgemeinen Struktur (IV) und dem Olefin der allgemeinen Struktur (V) in einem Eintopf-Verfahren durchgeführt wird.

7. Verfahren nach irgendeinem der Ansprüche 5 oder 6, **dadurch gekennzeichnet, daß** die Säure Trifluoressigsäure ist.

8. Verfahren nach irgendeinem der Ansprüche 5 bis 7, **dadurch gekennzeichnet, daß** die Umsetzung in einem organischen Lösungsmittel bei einer Temperatur von 0 bis 100 °C und einer Reaktionszeit von 0,25 bis 12 h durchgeführt wird.

9. Verfahren nach einem der Ansprüche 5 bis 8, **dadurch gekennzeichnet, daß** die Reaktion bei einer Temperatur von 15 bis 40 °C durchgeführt wird.

10. Arzneimittel, enthaltend mindestens eine Verbindung der allgemeinen Struktur (I A) oder (I B) 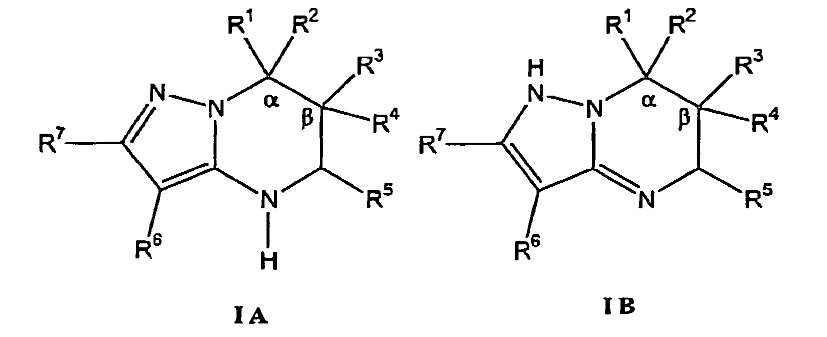 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereo isomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆-[Alk], Aryl' oder -(C₁₋₈-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten, wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl', -CH₂-Aryl' oder unsubstituiertes Methyl, Ethyl. n-Propyl, 2-Propyl, n-Butyl, iso-Butyl, sek.-Butyl, tert.-Butyl, n-Amyl, iso-Amyl, sek. Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴
W bildet, wobei W α'-CH=CH-CH₂-β', α'-CH=CH-CH₂₋CH₂-β',α'-O-(CH₂)ₘ-β' mit m = 2, 3, 4 oder 5, 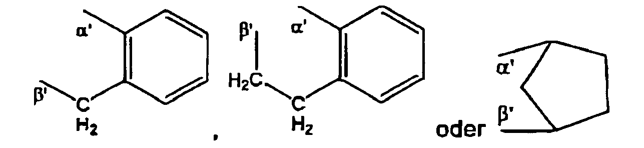 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnete** Ende von W mit dem mit β **gekennzeichneten** Atom der Verbindung der allgemeinen Struktur (I A) oder (I B) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NH₂, -NO₂, -SH, -OH, -CO₂H und -CO₂Alkyl, Aryl' oder
-(CH₂)ₖ-Aryl', wobei k = 1,2,3 oder 4 ist, Heterocyclyl oder C(=O)R¹¹ bedeutet;
R⁶ H, Methyl, Ethyl, -CN, Fluor, Chlor, Brom, lod, -C(=O)R¹⁷ oder -N=N-Aryl¹ bedeutet;
R⁷ H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁷ OR²⁶ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.- Butyl, n-Amyl, iso-Amyl, sek.-Amyl, n-Hexyl, iso-Hexyl oder sek.-Hexyl bedeutet;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁶ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet: und
Heterocyclyl Furan-2-yl, Furan-3-yl, Thien-2-yl, Thien-3-yl, Pyridin-2-yl-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 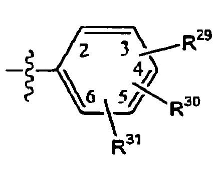 steht;
Aryl² für 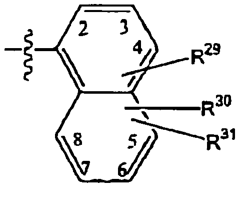 steht;
Aryl³ für 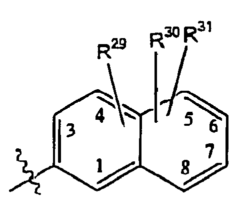 steht;
R²⁹, R³⁰ und R³¹ unabhängig voneinander H, C₁₋₆-[Alk], C₃₋₈₋[Cydoalk], -(C₁₋₆-Alkyl)-C₃₋₈-[Cycloalk], [Ar], -(C₁₋₆₋Alkyl)-[Ar], [Het], -(C₁₋₆-Alkyl)-[Het], F, Cl, Br, I, -CN,-NC, -OR³², -SR³³, -NO, -NO₂, NH₂, NHR³⁴, NR³⁵R³⁶,-N-OH, -N-OC₁₋₆-[Alk], -NHNH₂, -N=N-[Ar], -(C=O)R³⁷, 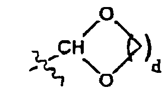 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)₉- mit g = 4 oder 5 stehen;
R³⁷ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het], -(C₁₋₅-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar] bedeutet; und
R³⁹ H, C₁₋₆-[Alk], -C₃₋₈-[Cycloalk], -(C₁₋₈-Alkyl)-C₃₋₈₋[Cycloalk], -[Ar], -(C₁₋₆-Alkyl)-[Ar], -[Het] oder -(C₁₋₆-Alkyl)-[Het] bedeutet.
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO_{2,} -SH, -OH, -CO₂H und -CO₂-Alkyl;
[Ar] für einen unsubstituierten Rest ausgewählt aus der Gruppe bestehend aus Phenyl, Naphthyl, Anthracenyl und Biphenyl steht;
[Het] für einen unsubstituierten Hoterocyclyl-Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht.

11. Verwendung einer Verbindung der allgemeinen Struktur (I A) oder (I B), 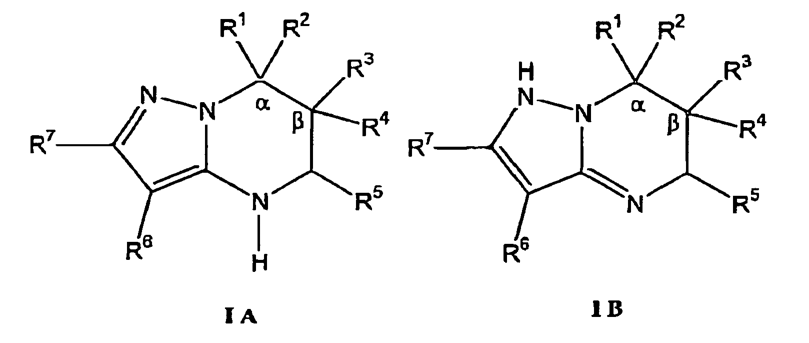 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; worin
R¹ und R² unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-C₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl', -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist,
oder einer der Reste R¹ und R² zusammen mit einem der Reste R³ und R⁴
W bildet, wobei W α'-CH=CH-CH₂-β', α'-CH=CH-CH₂₋CH₂-β',α'-O-(CH₂)ₘ-β' mit m = 2, 3, 4 oder 5, 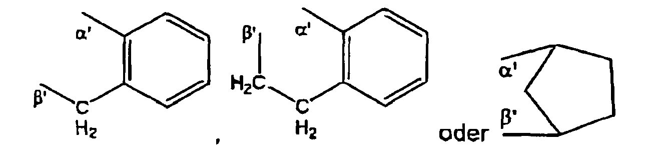 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnete** Ende von W mit dem mit β **gekennzeichneten** Atom der Verbindung der allgemeinen Struktur (I A) oder (I B) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NH₂, -NO₂, -SH, -OH, -CO₂H und -CO₂Alkyl, Aryl' oder -(CH₂)ₖ-Aryl', wobei k = 1,2,3 oder 4 ist, Heterocyclyl oder C(=O)R¹¹ bedeutet;
R⁶ H, Methyl, Ethyl, -CN, Fluor, Chlor, Brom, Iod, -C(=O)R¹⁷ oder -N=N-Aryl¹ bedeutet;
R⁷ H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁷ OR²⁶ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet;
R²⁵ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet, wobei R²⁵ nicht H bedeutet, wenn zugleich R¹ Aryl und R² Alkyl bedeuten;
R²⁶ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet; und
Heterocyclyl Furan-2-yl, Furan-3-yl, Thien-2-yl, Thien-3-yl, Pyridin-2-yl-. Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂, -CO₂H und -CO₂Alkyl, bedeutet;
Aryl' Aryl¹, Aryl² oder Aryl³ bedeutet;
Aryl¹ für 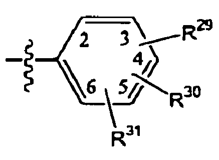 steht;
Aryl² für 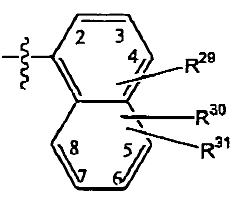 steht;
Aryl³ für 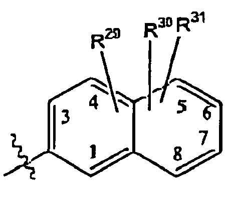 steht;
R²⁹, R³⁰ und R³¹ unabhängig voneinander H, C₁₋₆-[Alk], C₃₋₈₋[Cycloalk], -(C₁₋₆-Alkyl)-C₃₋₈-[Cycloalk], [Ar], -(C₁₋₆₋Alkyl)-[Ar], [Het], -(C₁₋₆-Alkyl)-[Het], F, Cl, Br, I, -CN, -NC, -OR³², -SR³³, -NO, -NO₂, NH₂, NHR³⁴, NR³⁵R³⁶, -N-OH, -N-OC₁₋₆-[Alk], -NHNH₂, -N=N-[Ar], -(C=O)R³⁷, 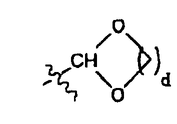 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar], -[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)_{g}- mit g = 4 oder 5 stehen;
R³⁷ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar] bedeutet; und
R³⁹ H, C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Ar] für einen unsubstituierten Rest ausgewählt aus der Gruppe bestehend aus Phenyl, Naphthyl, Anthracenyl und Biphenyl steht:
[Het] für einen unsubstituierten Heterocyclyl-Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht,
zur Herstellung eines Arzneimittels zur Behandlung von Schmerz.

12. Verwendung einer Verbindung der allgemeinen Struktur (I A) oder (I B), 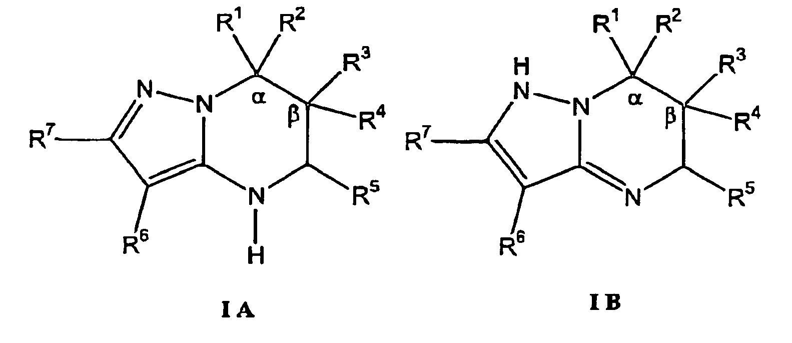 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats; der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin R¹, R², R³, R⁴, R⁵, R⁶ und R⁷ wie in Anspruch 11 definiert sind; zur Herstellung eines Arzneimittels zur Behandlung und/oder Prophylaxe von Epilepsie, Schizophrenie, neurodegenerativen Erkrankungen, insbesondere Morbus Alzheimer, Morbus Huntington und Morbus Parkinson, von cerebralen Ischämien und Infarkten, von Psychosen bedingt durch erhöhten Aminosäurespiegel, Himödemen, Unterversorgungszuständen des zentralen Nervensystems, insbesondere bei Hypoxien und Anoxien, von AIDS-Demens, von Encephalomyelitis, des Tourette-Syndroms, der perinatalen Asphyxie und/oder bei Tinnitus.

13. Verwendung einer Verbindung gemäß Formel (IA) und/oder (IB) 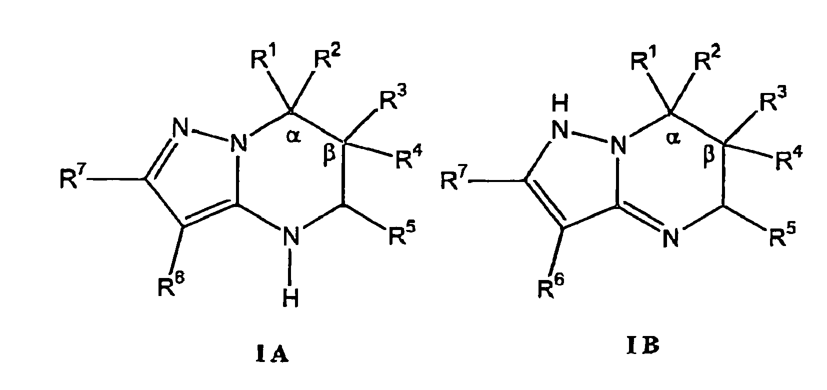 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin
R¹ und R² unabhängig voneinander H, O-R⁹, S-R¹⁰, C₁₋₆-[Alk], Aryl' oder -(C₁₋₆-Alkyl)-Aryl', wobei die Aryl'-Substituenten R²⁹, R³⁰ und R³¹ unabhängig voneinander H, C₁₋₆-[Alk], F, Cl, Br, I, OH, O-G₁₋₆-[Alk], O-Aryl¹ oder O-CH₂-Aryl¹ sind, bedeuten,
wobei einer der Reste R¹ und R² H ist und der andere Rest von R¹ und R² nicht H ist oder für den Fall, daß einer der Reste R¹ und R² Aryl' bedeutet, der andere Rest von R¹ und R² H oder C₁₋₆-Alkyl bedeutet,
R³ und R⁴ H, Aryl', -CH₂-Aryl' oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl, bedeutet,
wobei mindestens einer der Reste R³ und R⁴ H ist, oder einer der Reste R¹ und R² zusammen mit einem der Reste R³ und R⁴
W bildet, wobei W α'-CH=CH-CH₂-β', α'-CH=CH-CH₂₋CH₂-β',α'-O-(CH₂)ₘ-β' mit m = 2, 3, 4 oder 5, 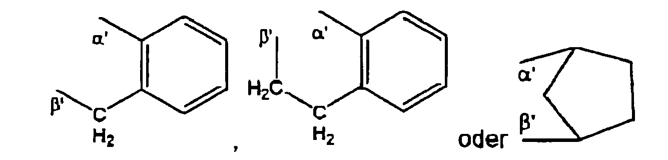 bedeutet, das mit α' **gekennzeichnete** Ende von W mit dem mit α **gekennzeichneten** Atom der Verbindung der allgemeinen Struktur (I A) oder (I B) verbunden ist und das mit β' **gekennzeichnete** Ende von W mit dem mit β **gekennzeichneten** Atom der Verbindung der allgemeinen Struktur (I A) oder (I B) verbunden ist, der andere Rest von R¹ und R² H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hoxyl, iso-Hexyl, oder sek.-Hexyl bedeutet und der andere Rest von R³ und R⁴ H oder Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl bedeutet;
R⁵ Methyl, Ethyl, n-Propyl, 2-Propyl, n-Butyl, iso-Butyl, sek.-Butyl, tert-Butyl, n-Amyl, iso-Amyl, sek.-Amyl, n-Hexyl, iso-Hexyl, sek.-Hexyl, Cyclopropyl, Cyclobutyl, Cyclopentyl, Cyclohexyl oder Cycloheptyl, die jeweils unsubstituiert oder einfach substituiert oder mehrfach gleich oder verschieden substituiert sind mit einem Substituenten ausgewählt aus der Gruppe bestehend aus F, Cl, Br, I, -CN, -NC, -NH₂, -NO₂, -SH, -OH, -CO₂H und CO₂Alkyl, Aryl' oder
-(CH₂)ₖ-Aryl', wobei k = 1,2,3 oder 4 ist, Heterocyclyl oder C(=O)R¹¹ bedeutet;
R⁶ H, Methyl, Ethyl, -CN, Fluor, Chlor, Brom, Iod, -C(=O)R¹⁷ oder -N=N-Aryl¹ bedeutet;
R⁷ H, Aryl¹, OR¹⁸, S(O)_{q}R¹⁹, wobei q = 0, 1 oder 2, oder unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet,
R⁹ unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl, sek.-Hexyl, Cyclopropyl, Cyclobutyl, Cyclopentyl. Cyclohexyl oder Cycloheptyl bedeutet oder -[(CH₂)ᵣ-O]ₛ-H mit r = 1, 2, 3, 4, 5 oder 6 und s = 1, 2, 3, 4, 5 oder 6 bedeutet;
R¹⁰ Aryl' bedeutet;
R¹¹ Aryl' oder OR²⁵ bedeutet;
R¹⁷ OR²⁶ bedeutet;
R¹⁸ H oder Methyl bedeutet;
R¹⁹ H, Aryl¹ oder jeweils unsubstituiertes Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek-Hexyl bedeutet;
R²⁵ H, Methyl, Ethyl, n-Pmpyl, 2-Propyl, n-Butyl, iso-Butyl, sek.-Butyl, tert.-Butyl, n-Amyl, iso-Amyl, sek.-Amyl, n-Hexyl, isa-Hexyl oder sek.-Hexyl bedeutet, wobei R²⁵ nicht H bedeutet, wenn zugleich R¹ Aryl und R² Alkyl bedeuten;
R²⁶ H, Methyl, Ethyl, n-Propyl, 2-Propyl, n-Butyl, iso-Butyl, sek.-Butyl, tert.-Butyl, n-Amyl, iso-Amyl, sek.-Amyl, n-Hexyl, iso-Hexyl oder sek.-Hexyl bedeutet; und
Heterocyclyl Furan-2-yl, Furan-3-yl, Thien-2-yl, Thien-3-yl, Pyridin-2-yl-, Pyridin-3-yl oder Pyridin-4-yl, wobei Furanyl, Thienyl und Pyridinyl jeweils unsubstituiert oder einfach substituiert oder mehrfach gleich oder verschieden substituiert sind mit einem Substituenten ausgewählt aus der Gruppe bestehend aus F, Cl, Br, I, -CN, -NO₂,-CO₂H und -CO₂Alkyl, bedeutet;
Aryl' Aryl¹, Aryl² oder Aryl³ bedeutet;
Aryl¹ für 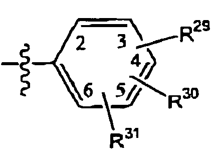 steht;
Aryl² für 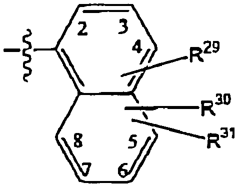 steht;
Aryl³ für 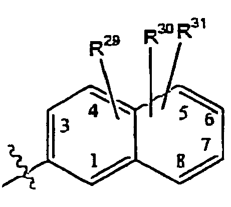 steht;
R²⁹, R³⁰ und R³¹ unabhängig voneinander H, C₁₋₆-[Alk], C₃₋₈₋[Cycloalk], -(C₁₋₆-Alkyl)-C₃₋₈-[Cycloalk], [Ar], -(C₁₋₆₋Alkyl)-[Ar], [Het], -(C₁₋₆-Alkyl)-[Het], F, Cl, Br, I, -CN, -NC, -OR³², -SR³³, -NO, -NO₂, NH₂, NHR³⁴, NR³⁵R³⁶, -N-OH, -N-OC₁₋₆-[Alk], -NHNH₂, -N=N-[Ar], -(C=O)R³⁷, 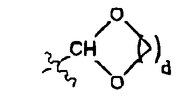 mit d = 1, 2 oder 3, oder -(C=S)R³⁷ bedeuten und in jeder beliebigen Ringposition sein können;
R³² und R³³ unabhängig voneinander H, -C₁₋₆-[Alk], -C₃₋₈-[Cycloalk], -(C₁₋₆-Alkyl)-C₃₋₈-[Cycloalk], -[Ar], -(C₁₋₆-Alkyl)-[Ar],-[Het], -(C₁₋₆-Alkyl)-[Het], (C=O)R³⁸, -[(CH₂)_{w}-O]_{z}-H oder -[(CH₂)_{w}-O]_{z}-C₁₋₆-[Alk] mit w = 1, 2, 3 oder 4 und z = 1, 2, 3, 4 oder 5 bedeuten;
R³⁴ C₁₋₆-[Alk], -CH₂-[Ar] oder -(C=O)O-tert.-Butyl bedeutet;
R³⁵ und R³⁶ unabhängig voneinander C₁₋₆-[Alk] bedeuten oder gemeinsam für -(CH₂)_{g}- mit g = 4 oder 5 stehen;
R³⁷ H, -C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het], -(C₁₋₆-Alkyl)-[Het], -OR³⁹, -NH₂, -NHR³⁴, -NR³⁵R³⁶ bedeutet;
R³⁸ H, -C₁₋₆-[Alk], -C₃₋₈-[Cycloalk], -(C₁₋₆-Alkyl)-C₃₋₈₋[Cycloalk]. -[Ar], -(C₁₋₆-Alkyl)-[Ar] bedeutet; und
R³⁹ H, C₁₋₆-[Alk], -C₃₋₈-[Cycloalk], -(C₁₋₆-Alkyl)-C₃₋₈₋[Cycloalk], -[Ar], -(C₁₋₆-Alkyl)-[Ar], -[Het] oder -(C₁₋₆-Alkyl)-[Het] bedeutet,
[Alk] für einen acyclischen, gesättigten oder ungesättigten, verzweigten oder geradkettigen Kohlenwasserstoff-Rest steht, der unsubstituiert oder einfach substituiert oder mehrfach gleich oder verschieden substituiert sein kann,
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Cycloalk] für einen cyclischen, gesättigten oder ungesättigten Kohlenwasserstoff-Rest steht, der unsubstituiert oder einfach substituiert oder mehrfach gleich oder verschieden substituiert und ggf. benzokondensiert sein kann.
wobei die Substituenten ausgewählt werden können aus der Gruppe bestehend aus F, Cl, Br, I, -CN, -NC, -NH₂, -NO₂, -SH, -OH, -CO₂H und -CO₂-Alkyl;
[Ar] für einen unsubstituierten Rest ausgewählt aus der Gruppe bestehend aus Phenyl, Naphthyl, Anthracenyl und Biphenyl steht;
[Het] für einen unsubstituierten Heterocyclyl-Rest ausgewählt aus der Gruppe bestehend aus Pyrrolidinyl, Tetrahydrofuryl, Piperidinyl, Piperazinyl und Morpholinyl oder für einen unsubstituierten Heteroaryl-Rest ausgewählt aus der Gruppe bestehend aus Pyrrolyl, Pyrazolyl, Imidazolyl, Pyridazinyl, Pyrimidinyl, Pyrazinyl, Furanyl, Thienyl und Pyridinyl steht;
als Ligand von Nucleosid-Transport-Proteinen und/oder der Adenosin-Kinase und/oder der Adenosin-Deaminase und/oder von A₁- und/oder von A₂- und/oder von A₃-Rozeptoren.

14. Verwendung einer Verbindung gemäß Formel (IA) und/oder (IB) 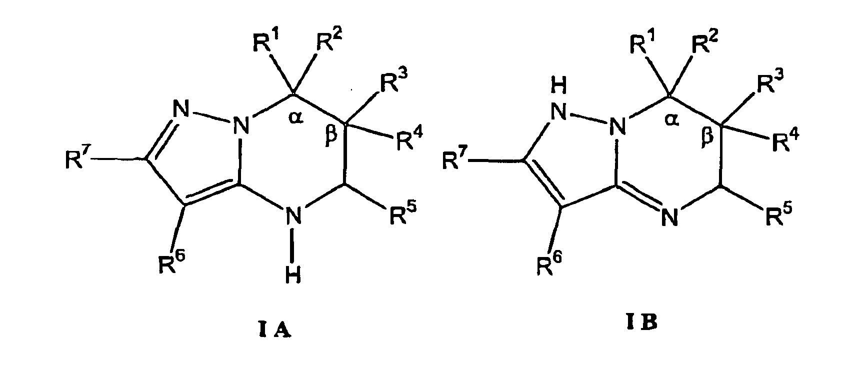 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin R¹, R², R³, R⁴, R⁵, R⁶ und R⁷ wie in Anspruch 13 definiert sind; zur Herstellung eines Medikaments zur Prävention und/oder Behandlung von Zuständen und/oder Krankheiten, die über eine Stimulierung und/oder Inhibierung von Nucleosid-Transport-Proteinen und/oder der Adenosin-Kinase und/oder Adenosin-Deaminase und/oder von A₁- und/oder von A₂- und/oder von A₃₋Rezeptoren beeinflußt werden.

15. Verwendung nach einem der Ansprüche 13 oder 14 zur Herstellung eines Medikaments zur Prävention und/oder Behandlung von Schmerz, neuropathischem Schmerz, Atemwegserkrankungen, Krebs, kardialen Arrythmien, Ischämien, Epilepsie, Morbus Huntigton, Immunstörungen und -erkrankungen, Entzündungszuständen und -erkrankungen, Neugeborenen-Hypoxie, neurodegenerativen Erkrankungen, Morbus Parkinson, Nierenversagen. Schizophrenie, Schlafstörungen, Schlaganfall, Thrombosen, Harninkontinenz, Diabetes, Psoriasis, septischem Schock, Gehimtraumata, Glaukom und/oder Stauungsinsuffizienz.

16. Verwendung nach irgendeinem der Ansprüche 13 bis 15, **dadurch gekennzeichnet, daß**
R¹ und R² unabhängig voneinander H, O-CH₂-CH₂-OH, O-Cyclohexyl, S-Phenyl, Methyl, Phenyl, 3-Fluor-phenyl, 3-Brom-phenyl, 4-Brom-phenyl, 4-Chlor-phenyl, 4-Fluor-phenyl, 3-Methyl-phenyl, 4-Hydroxy-phenyl, 4-Methoxy-phenyl, 2,4-Dimethyl-phenyl, 3,4-Dimethoxy-phenyl, 2,3,4-Trimethoxyphenyl, 2-Naphthyl oder -CH₂₋Phenyl bedeuten,
R³ und R⁴ H, Methyl oder 4-Methoxy-phenyl bedeuten, wobei mindestens einer der Reste R³ und R⁴ H ist,
oder
einer der Reste R¹ und R² zusammen mit einem der Reste R³ und
R⁴ W bildet,
wobei W α'-CH=CH-CH₂-β', α'-CH=CH-CH₂-CH₂-β', α'-O-(CH₂)ₘ-β' mit m = 2, 3, 4 oder 5, 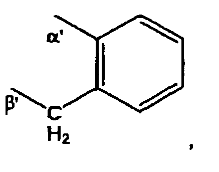 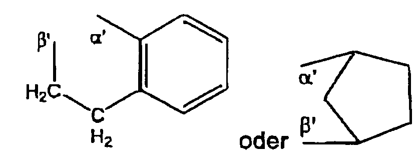 bedeutet, das mit α' **gekennzeichnete** Ende von W mit dem mit α **gekennzeichneten** Atom der Verbindung der allgemeinen Formel (IA) und/oder (IB) verbunden ist, das mit β' **gekennzeichnete** Ende von W mit dem mit β **gekennzeichneten** Atom der Verbindung der allgemeinen Struktur (IA) und/oder (IB) verbunden ist, der andere Rest von R¹ und R² und der andere Rest von R³ und R⁴ jeweils H bedeuten;
R⁵ n-Propyl, n-Butyl, tert-Butyl. -(CH₂)₄-OH, Cyclopropyl, Cycloprop-2-yl-1-carbonsäureethylester, Cyclohexyl, 4-Trifluorphenyl, 4-Phenoxy-phenyl, 2-Hydroxy-3-methoxy-phenyl, 4-Hydroxy-3-methoxy-phenyl, 3-Carboxy-2-hydroxy-phenyl, -(CH₂)₂-Phenyl, 5-Carboxy-furan-2-yl, 5-Methyl-furan-2-yl, 5-Nitro-furan-2-yl, 5-Nitro-thien-2-yl, Pyridin-2-yl, Pyridin-3-yl, C(=O)Phenyl, C(=O)OH oder C(=O)OEthyl bedeutet, wobei R⁵ nicht C(=O)OH bedeutet, wenn zugleich R¹ Aryl und R² Alkyl bedeuten;
R⁶ H, -CN, Brom, -C(=O)OH, -C(=O)OEthyl oder -N=N-Phenyl bedeutet; und
R⁷ H, Phenyl, OH, -S-Methyl, -SO₂-(4-nitrophenyl) oder tert.-Butyl bedeutet.

17. Pharmazeutische Zusammensetzung, die mindestens eine Verbindung der allgemeinen Struktur (I A) oder (I B), 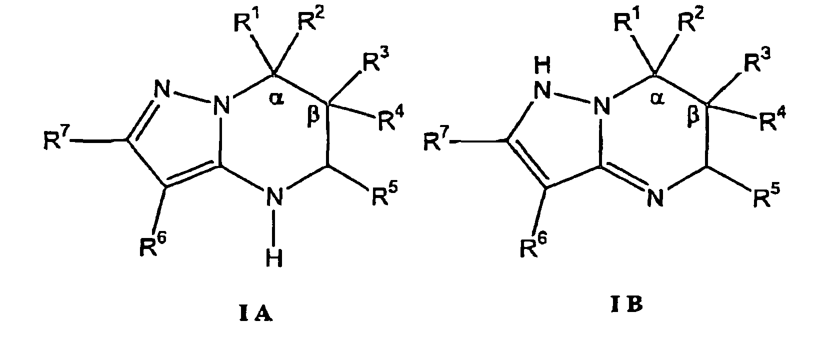 in dargestellter Form oder in Form ihrer Säure(n) oder ihrer Base(n) oder in Form eines ihrer Salze, insbesondere der physiologisch verträglichen Salze, oder in Form eines ihrer Solvate, insbesondere der Hydrate;
in Form ihres Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
worin R¹, R², R³, R⁴, R⁵, R⁶ und R⁷ wie in Anspruch 1 definiert sind; sowie mindestens einen pharmazeutischen Hilfsstoff enthält

## Claims

1. Compound of the general structure (I A) or (I B) 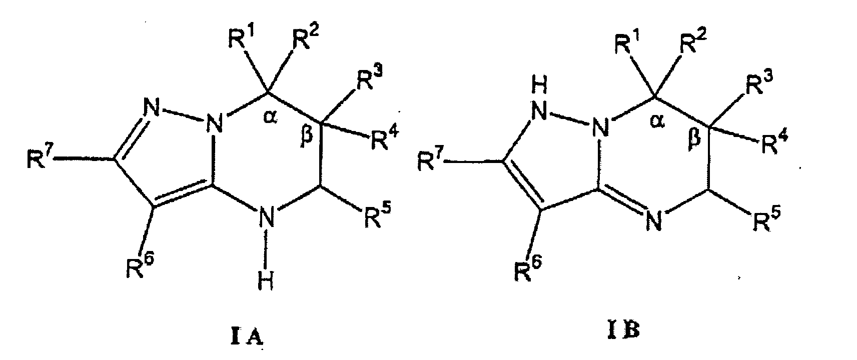 in the illustrated form or in the form of their acid(s) or base(s) or in the form of one of their salts, in particular the physiologically compatible salts, or in the form of one of their solvates, in particular the hydrates;
in the form of their racemate, pure stereoisomers, in particular enantiomers or diastereomers, or in the form of mixtures of the stereoisomers, in particular of the enantiomers or diastereomers, in an arbitrary mixture ratio,
wherein
R¹ and R² independently of one another denote H, O-R⁹, S-R¹⁰, C₁₋₆-[alk], aryl' or -(C₁₋₆-alkyl)-aryl',
wherein the aryl' substituents R²⁹, R³⁰ and R³¹ independently of one another denote H, C₁₋₆₋[alk], F, Cl, Br, I, OH, O-C₁₋₆-[alk], O-aryl¹ or O-CH₂-aryl¹, wherein one of the radicals R¹ and R² is H and the other radical of R¹ and R² is not H, or in the case that one of the radicals R¹ and R² denotes aryl', the other radical of R¹ and R² denotes H or C₁₋₆₋alkyl,
R³ and R⁴ denote H, aryl', -CH₂-aryl' or unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, wherein at least one of the radicals R³ and R⁴ is H, or
one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 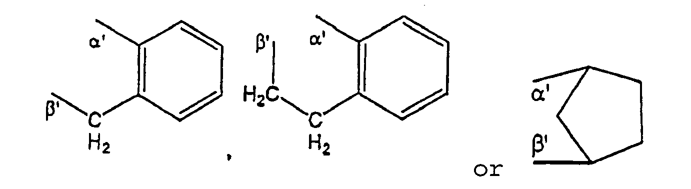 the end of W identified by α' is joined to the atom of the compound of the general structure (I A) or (I B) identified by α, and the end of W identified by β' is joined to the atom of the general structure (I A) or (I B) identified by β, the other radical of R¹ and R² is H or methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl and the other radical of R³ and R⁴ denotes H or methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl;
R⁵ denotes methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl that in each case is unsubstituted or singly substituted or multiply identically or differently substituted with a substituent selected from the group consisting of F, Cl, Br, I, -CN, -NC, -NH₂, -NO₂, -SH, -OH, -CO₂H and aryl', or denotes -(CH₂)ₖ-aryl', where k = 1,2,3 or 4, heterocyclyl or C(=O)R¹¹;
R⁶ denotes H, methyl, ethyl, -CN, fluorine, chlorine, bromine, iodine, -C(=O)R¹⁷ or -N=N-aryl¹;
R⁷ denotes H, aryl¹, OR¹⁸, S(O)_{q}R¹⁹, where q = 0, 1 or 2, or denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl,
R⁹ denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl or denotes-[(CH₂)ᵣ-O]ₛ-H where r = 1, 2, 3, 4, 5 or 6 and s = 1, 2, 3, 4, 5 or 6;
R¹⁰ denotes aryl';
R¹¹ denotes aryl' or OR²⁵;
R¹⁷ denotes OR²⁶;
R¹⁸ denotes H or methyl;
R¹⁹ denotes H, aryl¹, or in each case unsubstituted, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl;
R²⁵ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, where R²⁵ does not denote H if at the same time R¹ denotes aryl and R² denotes alkyl;
R²⁶ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl; and
Heterocyclyl denotes furan-2-yl, furan-3-yl, thien-2-yl, thien-3-yl, pyridin-2-yl, pyridin-3-yl or pyridin-4-yl, where furanyl, thienyl and pyridinyl are in each case unsubstituted or singly substituted or multiply identically or differently substituted with a substituent selected from the group consisting of F, Cl, Br, I, -CN, -NO₂, -CO₂H and -CO₂-alkyl;
Aryl' denotes aryl¹, aryl² or aryl³;
Aryl¹ denotes 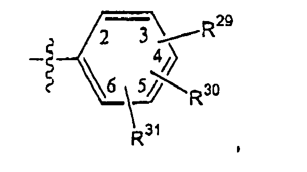
Aryl² denotes 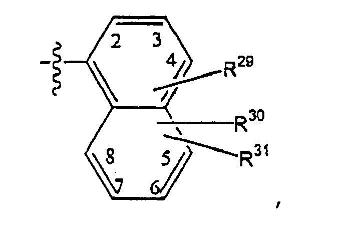
Aryl³ denotes 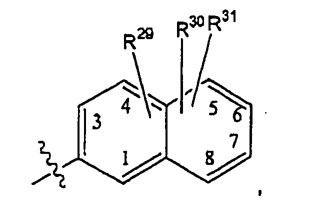
R²⁹, R³⁰ and R³¹ independently of one another denote H, C₁₋₆-[alk], C₃₋₈-cycloalkyl, (C₁₋₆ alkyl) -C₃₋₈₋[cycloalk], [ar], (C₁₋₆-alkyl)-[ar], [het], (C₁₋₆ alkyl)-[het], F, Cl, Br, I, -CN, -NC, -OR³², -SR³³, -NO, -NO₂, NH₂ , NHR³⁴, NR³⁵R³⁶, -N-OH, -N-OC₁₋₆- [alk], -NHNH₂, -N=N- [ar], -(C=O)R³⁷, 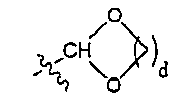 where d = 1, 2 or 3, or denotes -(C=S)R³⁷, and may be in any arbitrary ring position;
R³² and R³³ independently of one another denote H, -C₁₋₆₋[alk], -C₃₋₈- [cycloalk], - (C₁₋₆-alkyl)-C₃₋₈₋[cycloalk], -[ar], -(C₁₋₆-alkyl)-[ar], -[het], -(C₁₋₆-alkyl)-[het], (C=O)R³⁸, - [(CH₂)_{w}-O]_{z}-H or - [(CH₂)_{w}-O]_{z}-C₁₋₆-[alk] where w = 1, 2, 3 or 4 and z = 1, 2, 3, 4 or 5;
R³⁴ denotes C₁₋₆-[alk], -CH₂-[ar] or -(C=O) O-tert.-butyl;
R³⁵ and R³⁶ independently of one another denote C₁₋₆₋[alk] or together denote -(CH₂)-_{g} where g = 4 or 5;
R³⁷ denotes H, -C₁₋₆-[alk], -C₃₋₈-[cycloalk], - (C₁₋₆-alkyl) -C₃₋₈-[cycloalk], -[ar], -(C₁₋₆₋alkyl) - [ar], -[het], -(C₁₋₆-alkyl)-[het], -OR³⁹, -NH₂, -NHR³⁴, -NR³⁵R³⁶;
R³⁸ denotes H, -C₁₋₆-[alk], -C₃₋₈-[cycloalk], -(C₁₋₆-alkyl)-C₃₋₈-[cycloalk], -[ar], -(C₁₋₆₋alkyl)-[ar];
and
R³⁹ denotes H, -C₁₋₆- [alk], -C₃₋₈-[cycloalk], -(C₁₋₆-alkyl) -C₃₋₈- [cycloalk], - [ar], - (C₁₋₆₋alkyl) - [ar], - [het] or - (C₁₋₆-alkyl)-[het] .
[Alk] denotes an acyclic, saturated or unsaturated, branched or straight-chain hydrocarbon radical that may be unsubstituted or singly substituted or multiply identically or differently substituted,
wherein the substituents may be selected from the group consisting of F, Cl, Br, I, -CN, -NC, -NH₂, -NO₂, -SH, -OH, -CO₂H and -CO₂-alkyl;
[Cycloalk] denotes a cyclic, saturated or unsaturated hydrocarbon radical that may be unsubstituted or singly substituted or multiply identically or differently substituted and optionally benzo-condensed,
wherein the substituents may be selected from the group consisting of F, Cl, Br, I, -CN, -NC, -NH₂, -NO₂, -SH, -OH, -CO₂H and -CO₂-alkyl;
[Ar] denotes an unsubstituted radical selected from the group consisting of phenyl, naphthyl, anthracenyl and biphenyl;
and
(Het) denotes an unsubstituted heterocyclyl radical slected from the group consisting of pyrrolidinyl, tetrahydrofuryl, piperidinyl, piperazinyl and morpholinyl or denotes an unsubstituted heteroaryl radical selected from the group consisting of pyrrolyl, pyrazolyl, imidazolyl, pyridazinyl, pyrimidinyl, pyrazinyl, furanyl, thienyl and pyridinyl
with the exception of the compounds
4,5,6,7-tetrahydro-2-methyl-5,7-diphenylpyrazolo-[1,5-a]pyrimidine,
4,5,6,7-tetrahydro-2,5-dimethyl-7-phenylpyrazolo-[1,5-a]pyrimidine,
4,5,6,7-tetrahydro-5,7-dimethyl-3-phenylpyrazolo-[1,5-a]pyrimidine,
4,5,6,7-tetrahydro-2,5,7-trimethylpyrazolo[1,5-a] pyrimidine,
4,5,6,7-tetrahydro-5,7-dimethyl-2-phenylpyrazolo-[1,5-a]pyrimidine,
4,5,6,7-tetrahydro-2-methyl-5,7-di-n-propylpyrazolo[1,5-a]pyrimidine-3-carbonitrile,
4,5,6,7-tetrahydro-5-methyl-7-[3-(trifluoro-methyl)-phenyl]pyrazolo[1,5-a]pyrimidine-3-carbonitrile,
7-[4-(chloro)-phenyl]-4,5,6,7-tetrahydro-5-methylpyrazolo[1,5-a]pyrimidine-3-carbonitrile,
7-[3-(chloro)-phenyl]-4,5,6,7-tetrahydro-5-methylpyrazolo[1,5-a]pyrimidine-3-carbonitrile.

2. Compound according to claim 1, **characterised in that**
R¹ and R² independently of one another denote H, O-R⁹, S-R¹⁰, unsubstituted or singly substituted or multiply identically or differently substituted methyl, ethyl, n-propyl, 2-propyl, n-butyl, tert.-butyl or n-hexyl,
where the substituents are selected from the group consisting of F, Cl, Br, -CN, -NC, -NH₂, -NO₂, -SH and -OH; aryl' or -CH₂-aryl',
where the aryl' substituents R²⁹, R³⁰ and R³¹ independently of one another are H, methyl, ethyl, 2-propyl, n-butyl, tert.-butyl, n-hexyl, F, Cl, Br, I, OH, O-methyl, O-ethyl,
wherein one of the radicals R¹ and R² is H and the other radical of R¹ and R² is not H, or in the case that one of the radicals R¹ and R² denotes aryl', the other radical of R¹ and R² denotes H or methyl, ethyl, n-propyl, 2-propyl, n-butyl, tert.-butyl or n-hexyl,
R³ and R⁴ denote H, methyl or aryl¹, wherein the aryl¹ substituents R²⁹, R³⁰ and R³¹ independently of one another are H, methyl or O-methyl,
wherein at least one of the radicals R³ and R⁴ is H,
or one of the radicals R¹ and R² together with one of the radicals R³ and R⁴ forms W, where W denotes α'-CH=CH-CH₂-β',α'-CH=CH-CH₂-CH₂-β', α'-O-(CH₂)ₘ-β', where m = 2, 3, 4 or 5 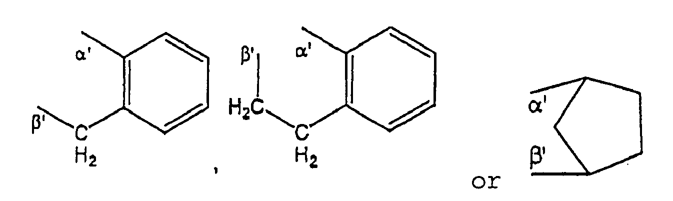 the end of W identified by α' is joined to the atom of the compound of the general structure (I A) or (I B) identified by α, and the end of W identified by β' is joined to the atom of the compound of the general structure (I A) or (I B) identified by β, the other radical of R¹ and R² and the other radical of R³ and R⁴ in each case denotes H;
R⁵ denotes methyl, ethyl, n-propyl, 2-propyl, n-butyl, isobutyl, sec.-butyl, tert.-butyl, -(CH₂)₄-OH, cyclopropyl, where cyclopropyl is unsubstituted or singly substituted by C(=O)OH, C(=O)O-methyl or C(=O)O-ethyl, or denotes cyclopentyl, cyclohexyl, aryl¹ or (CH₂)ₖ-aryl¹ where the aryl¹ substituents R²⁹, R³⁰ and R³¹ independently of one another denote H, -OH, -O-methyl , O-C₆H₅, CH₃, CF₃ or C(=O)OH and k = 1 or 2, or denotes heterocyclyl or C(=O)R¹¹;
R⁶ denotes H, -CN, bromine, -C(=O)R¹⁷ or -N=N-phenyl;
R⁷ denotes H, aryl¹ where R²⁹, R³⁰ and R³¹ are H, OH, S(O)_{q}R¹⁹, where q = 0 or 2, or denotes methyl, ethyl, n-propyl, 2-propyl, n-butyl, isobutyl, sec.-butyl or tert.-butyl;
R⁹ denotes methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclopentyl or cyclohexyl or denotes -[(CH₂)ᵣ-O]ₛ-H where r = 1, 2 or 3 and s = 1 or 2;
R¹⁰ denotes aryl¹;
R¹¹ denotes aryl¹ where R²⁹, R³⁰ and R³¹ denote H or OR²⁵;
R¹⁷ denotes OR²⁶;
R¹⁹ denotes methyl or aryl¹, where one of the aryl¹ substituents R²⁹, R³⁰ and R³¹ is H or -NO₂, and the two other aryl¹ substituents of R²⁹, R³⁰ and R³¹ are H;
R²⁵ denotes H, methyl or ethyl, where R²⁵ does not denote H if at the same time R¹ denotes aryl and R² denotes alkyl;
R²⁶ denotes H, methyl or ethyl; and
Heterocyclyl denotes furan-2-yl, furan-3-yl, thien-2-yl, thien-3-yl, pyridin-2-yl, pyridin-3-yl or pyridin-4-yl, where furanyl, thienyl and pyridinyl are in each case unsubstituted or singly substituted or multiply identically or differently substituted by -NO₂, -CH₃ or C(=O)OH.

3. Compound according to claim 1 or 2, **characterised in that**
R¹ and R² independently of one another denote H,O-CH₂₋CH₂-OH, O-cyclohexyl, S-phenyl, methyl, phenyl, 3-fluorophenyl, 3-bromophenyl, 4-bromophenyl, 4-chlorophenyl, 4-fluorophenyl, 3-methylphenyl, 4-hydroxyphenyl, 4-methoxyphenyl, 2,4-dimethylphenyl, 3,4-dimethoxyphenyl, 2,3,4-trimethoxyphenyl, 2-naphthyl or -CH₂-phenyl,
R³ and R⁴ denote H, methyl or 4-methoxyphenyl, where at least one of the radicals R³ and R⁴ is H, or
one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 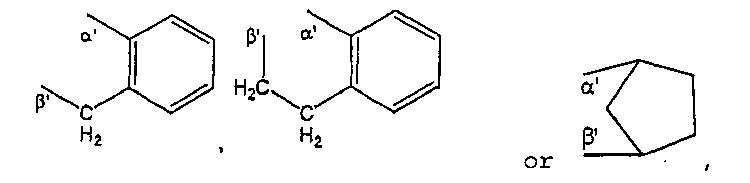 the end of W identified by α' is joined to the atom of the compound of the general structure (I A) or (I B) identified by α, and the end of W identified by β' is joined to the atom of the compound of the general structure (I A) or (I B) identified by β, and the other radical of R¹ and R² and the other radical of R³ and R⁴ in each case denote H;
R⁵ denotes n-propyl, n-butyl, tert.-butyl, -(CH₂)₄-OH, cyclopropyl, cycloprop-2-yl-1-carboxylic acid ethyl ether, cyclohexyl, 4-trifluorophenyl, 4-phenoxyphenyl, 2-hydroxy-3-methoxyphenyl, 4-hydroxy-3-methoxyphenyl, 3-carboxy-2-hydroxy-phenyl, -(CH₂)₂-phenyl, 5-carboxyfuran-2-yl, 5-methylfuran-2-yl, 5-nitrofuran-2-yl, 5-nitro-thien-2-yl, pyridin-2-yl, pyridin-3-yl, C(=O)-phenyl, C(=O)OH or C(=O)Oethyl, where R⁵ does not denote C(=O)OH if at the same time R¹ denotes aryl and R² denotes alkyl;
R⁶ denotes H, -CN, bromo, -C(=O)OH, -C(=O)Oethyl or -N=N-phenyl;
R⁷ denotes H, phenyl, OH, -S-methyl, -SO₂-(4-nitrophenyl) or tert.-butyl.

4. Compound according to claim 1, **characterised in that** the compound is selected from
• 3-bromo-5-(5-nitrofuran-2-yl)-7-m-tolyltetrahydropyrazolo[1,5-a]pyrimidine
• 3-bromo-7-(4-fluorophenyl)-7-methyl-5-(5-nitrofuran-2-yl)-tetrahydropyrazolo[1,5-a]pyrimidine
• 3-bromo-7-naphthalin-2-yl-5-(5-nitrofuran-2-yl)-tetrahydropyrazolo[1,5-a]pyrimidine
• 2-(3-bromo-7-m-tolyltetrahydropyrazolo[1,5-a]pyrimidin-5-yl)-cyclopropanecarboxylic acid ethyl ester
• 2-[3-bromo-7-(4-bromophenyl)-tetrahydropyrazolo[1,5-a]pyrimidin-5-yl]-cyclopropanecarboxylic acid ethyl ester
• 2-(3-bromo-7-naphthalin-2-yl-tetrahydropyrazolo[1,5-a]pyrimidin-5-yl)-cyclopropanecarboxylic acid ethyl ester
• 3-bromo-7-(4-fluorophenyl)-7-methyl-5-(5-methyl-furan-2-yl)-tetrahydropyrazolo[1,5-a]pyrimidine
• 3-bromo-7-(3,4-dimethoxyphenyl)-tetrahydropyrazolo[1,5-a]pyrimidine-5-carboxylic acid ethyl ester
• 3-bromo-7-(4-methoxyphenyl)-tetrahydropyrazolo[1,5-a]pyrimidine-5-carboxylic acid ethyl ester
• 3-bromo-7-(4-methoxyphenyl)-tetrahydropyrazolo[1,5-a]pyrimidine-5-carboxylic acid
• 3-bromo-7-(2,4-dimethylphenyl)-5-(5-nitrofuran-2-yl)-tetrahydropyrazolo[1,5-a]pyrimidine
• 3-bromo-7-(4-methoxyphenyl)-5-(5-nitrofuran-2-yl)-tetrahydropyrazolo[1,5-a]pyrimidine
• 5,5a,6,8a-tetrahydro-3H-1,4,8b-triaza-as-indacene-3,5-dicarboxylic acid diethyl ester; 5,5a,6,8a-tetrahydro-4H-1,4,8b-triaza-as-indacene-3,5-dicarboxylic acid diethyl ester
• 2-hydroxy-3-phenylazo-5,5a,6,8a-tetrahydro-3H-1,4,8b-triaza-as-indacene-5-carboxylic acid ethyl ester; 2-hydroxy-3-phenylazo-5,5a,6,8a-tetrahydro-4H-1,4,8b-triaza-as-indacene-5-carboxylic acid ethyl ester
• 2-tert.-butyl-5,5a,6,8a-tetrahydro-3H-1,4,8b-triaza-as-indacene-5-carboxylic acid ethyl ester; 2-tert.-butyl-5,5a,6,8a-tetrahydro-3H-1,4,8b-triaza-as-indacene-5-carboxylic acid ethyl ester
• 3-bromo-2-phenyl-5,5a,6,8a-tetrahydro-3H-1,4,8b-triaza-as-indacene-5-carboxylic acid ethyl ester; 3-bromo-2-phenyl-5,5a,6,8a-tetrahydro-4H-1,4,8b-triaza-as-indacene-5-carboxylic acid ethyl ester
• 7-(2,3,4-trimethoxyphenyl)-tetrahydropyrazolo[1,5-a]pyrimidine-3,5-dicarboxylic acid diethyl ester
• 3-cyano-2-methylsulfanyl-7-(2,3,4-trimethoxyphenyl)-tetrahydropyrazolo[1,5-a]pyrimidine-5-carboxylic acid ethyl ester
• 2-hydroxy-7-(4-hydroxyphenyl)-6-methyl-3-phenylazo-tetrahydropyrazolo[1,5-a]pyrimidine-5-carboxylic acid ethyl ester
• 3-bromo-7-(4-hydroxyphenyl)-6-methyl-2-phenyltetra-hydropyrazolo[1,5-a]pyrimidine-5-carboxylic acid ethyl ester
• 5,5a,6,10b-tetrahydro-3H-1,4,10c-triazacyclopenta[c]fluorene-3,5-dicarboxylic acid diethyl ester; 5,5a,6,10b-tetrahydro-4H-1,4,10c-triazacyclopenta[c]fluorene-3,5-dicarboxylic acid diethyl ester
• 2-hydroxy-3-phenylazo-5,5a,6,10b-tetrahydro-3H-1,4,10c-triazacyclopenta[c]fluorene-5-carboxylic acid ethyl ester; 2-hydroxy-3-phenylazo-5,5a,6,10b-tetrahydro-4H-1,4,10c-triazacyclopenta[c]fluorene-5-carboxylic acid ethyl ester
• 7-phenylsulfanyltetrahydropyrazolo[1,5-a]pyrimidine-3,5-dicarboxylic acid diethyl ester
• 3-cyano-2-methylsulfanyl-7-phenylsulfanyltetrahydro-pyrazolo[1,5-a]pyrimidine-5-carboxylic acid ethyl ester
• 3-cyano-2-methylsulfanyl-7-(2,3,4-trimethoxyphenyl)-tetrahydropyrazolo[1,5-a]pyrimidine-5-carboxylic acid
• 7-phenylsulfanyltetrahydropyrazolo[1,5-a]pyrimidine-3,5-dicarboxylic acid-3-ethyl ester
• 3-cyano-7-(2,4-dimethylphenyl)-2-methylsulfanyl-tetrahydropyrazolo[1,5-a]pyrimidine-5-carboxylic acid ethyl ester
• 3-cyano-7-(2,4-dimethylphenyl)-tetrahydropyrazolo [1,5-a]pyrimidine-5-carboxylic acid ethyl ester
• 7-(2,4-dimethylphenyl)-tetrahydropyrazolo-[1,5-a]pyrimidine-3,5-dicarboxylic acid-3-ethyl ester
• 3-bromo-7-(2,4-dimethylphenyl)-2-phenyltetrahydro-pyrazolo-[1,5-a]pyrimidine-5-carboxylic acid
• 3-cyano-7-(2,4-dimethylphenyl)-2-methylsulfanyl-tetrahydropyrazolo[1,5-a]pyrimidine-5-carboxylic acid
• 3-cyano-7-(2,4-dimethylphenyl)-tetrahydropyrazolo-[1,5-a]pyrimidine-5-carboxylic acid
• 3-cyano-7-(3,4-dimethoxyphenyl)-2-methylsulfanyl-tetrahydropyrazolo[1,5-a]pyrimidine-5-carboxylic, acid
• 7-(2,4-dimethylphenyl)-5-(5-nitrofuran-2-yl)-tetrahydropyrazolo[1,5-a]pyrimidine-3-carboxylic acid ethyl ester
• 7-(2,4-dimethylphenyl)-5-(5-nitrofuran-2-yl)-3-phenylazotetrahydropyrazolo[1,5-a]pyrimidin-2-ol
• 3-bromo-7-(2,4-dimethylphenyl)-5-(5-nitrofuran-2-yl)-2-phenylazotetrahydropyrazolo[1,5-a]pyrimidine
• 7-(2,4-dimethylphenyl)-2-methylsulfanyl-5-(5-nitrofuran-2-yl)-tetrahydropyrazolo[1,5-a]pyrimidine-3-carbonitrile
• 7-(2,4-dimethylphenyl)-5-(5-nitrofuran-2-yl)-tetrahydropyrazolo[1,5-a]pyrimidine-3-carbonitrile
• 3-bromo-7-(3,4-dimethoxyphenyl)-5-(5-nitrofuran-2-yl)-2-phenyltetrahydropyrazolo[1,5-a]pyrimidine
• 7-(4-methoxyphenyl)-2-methylsulfanyl-5-(5-nitro-furan-2-yl)-tetrahydropyrazolo[1,5-a]pyrimidine-3-carbonitrile
• 7-(2,4-dimethylphenyl)-5-(2-ethoxycarbonylcyclo-propyl)-tetrahydropyrazolo[1,5-a]pyrimidine-3-carboxylic acid ethyl ester
• 2-[7-(2,4-dimethylphenyl)-2-hydroxy-3-phenylazo-tetrahydropyrazolo[1,5-a]pyrimidin-5-yl]-cyclopropanecarboxylic acid ethyl ester
• 2-[2-tert.-butyl-7-(2,4-dimethylphenyl)tetrahydro-pyrazolo[1,5-a]pyrimidin-5-yl]cyclopropane-carboxylic acid ethyl ester
• 2-[3-bromo-7-(2,4-dimethylphenyl)-2-phenyltetra-hydropyrazolo[1,5-a]pyrimidin-5-yl]cyclopropanecarboxylic acid ethyl ester
• 2-[3-cyano-7-(2,4-dimethylphenyl)-2-methylsulfanyl-tetrahydropyrazolo[1,5-a]pyrimidin-5-yl]cyclopropanecarboxylic acid ethyl ester
• 5-(2-ethoxycarbonylcyclopropyl)-7-(3-fluorophenyl)-tetrahydropyrazolo[1,5-a]pyrimidine-3-carboxylic acid ethyl ester
• 2-[3-bromo-7-(3-bromophenyl)-2-phenyltetrahydro-pyrazolo[1,5-a]pyrimidin-5-yl]cyclopropane-carboxylic acid ethyl ester
• 2-[7-(3-bromophenyl)-3-cyano-2-methylsulfanyl-tetrahydropyrazolo[1,5-a]pyrimidin-5-yl]cyclopropanecarboxylic acid ethyl ester
• 7-(2,4-dimethylphenyl)-5-(5-nitrothiophen-2-yl)-3-phenylazotetrahydropyrazolo[1,5-a]pyrimidin-2-ol
• 7-(2,4-dimethylphenyl)-2-methylsulfanyl-5-(5-nitrothiophen-2-yl)-tetrahydropyrazolo[1,5-a]pyrimidine-3-carbonitrile
• 7-(2,4-dimethylphenyl)-5-(5-nitrothiophen-2-yl)-tetrahydropyrazolo[1,5-a]pyrimidine-3-carbonitrile
• 7-(3,4-dimethoxyphenyl)-5-(5-nitrothiophen-2-yl)-tetrahydropyrazolo[1,5-a]pyrimidine-3-carboxylic acid ethyl ester
• 7-(3,4-dimethoxyphenyl)-5-(5-nitrothiophen-2-yl)-3-phenylazotetrahydropyrazolo[1,5-a]pyrimidin-2-ol
• 3-bromo-7-(3,4-dimethoxyphenyl)-5-(5-nitrothiophen-2-yl)-2-phenyltetrahydropyrazolo[1,5-a]pyrimidine
• 7-(3,4-dimethoxyphenyl)-2-methylsulfanyl-5-(5-nitrothiophen-2-yl)-tetrahydropyrazolo[1,5-a]pyrimidine-3-carbonitrile
• 7-(3,4-dimethoxyphenyl)-5-(5-nitrothiophen-2-yl)-tetrahydropyrazolo[1,5-a]pyrimidine-3-carbonitrile
• 7-(4-methoxyphenyl)-5-(5-nitrothiophen-2-yl)-tetrahydropyrazolo[1,5-a]pyrimidine-3-carboxylic, acid ethyl ester
• 5-[3-bromo-7-(4-methoxyphenyl)-2-phenyltetrahydro-pyrazolo[1,5-a]pyrimidin-5-yl]-furan-2-carboxylic acid
• 5-benzoyl-7-(2,4-dimethylphenyl)-tetrahydropyrazolo-[1,5-a]pyrimidine-3-carboxylic acid ethyl ester
• 5-benzoyl-7-(2,4-dimethylphenyl)-2-methylsulfanyl-tetrahydropyrazolo[1,5-a]pyrimidine-3-carbonitrile
• 5-benzoyl-7-(2,4-dimethylphenyl)-tetrahydropyrazolo-[1,5-a]pyrimidine-3-carbonitrile
• 5-benzoyl-7-(3,4-dimethoxyphenyl)-tetrahydro-pyrazolo[1,5-a]pyrimidine-3-carboxylic acid ethyl ester
• [3-bromo-7-(3,4-dimethoxyphenyl)-2-phenyltetrahydro-pyrazolo[1,5-a]pyrimidin-5-yl]-phenylmethanone
• 5-benzoyl-7-(3,4-dimethoxyphenyl)-2-methylsulfanyl-tetrahydropyrazolo[1,5-a]pyrimidine-3-carbonitrile
• 5-benzoyl-7-(3,4-dimethoxyphenyl)tetrahydro-pyrazolo[1,5-a]pyrimidine-3-carbonitrile
• 5-benzoyl-7-(4-methoxyphenyl)-tetrahydropyrazolo-[1,5-a]pyrimidine-3-carboxylic acid ethyl ester
• 5-benzoyl-7-(4-methoxyphenyl)-2-methylsulfanyl-tetrahydropyrazolo[1,5-a]pyrimidine-3-carbonitrile
• 5-benzoyl-7-(4-methoxyphenyl)tetrahydropyrazolo-[1,5-a]pyrimidine-3-carbonitrile
• 5-benzoyl-7-(3-fluorophenyl)-tetrahydropyrazolo-[1,5-a]pyrimidine-3-carboxylic acid ethyl ester
• [3-bromo-7-(3-fluorophenyl)-2-phenyltetrahydro-pyrazolo[1,5-a]pyrimidin-5-yl]-phenylmethanone
• [3-bromo-7-(3-bromophenyl)-2-phenyltetrahydro-pyrazolo[1,5-a]pyrimidin-5-yl]-phenylmethanone
• 7-(2,4-dimethylphenyl)-5-(4-phenoxyphenyltetra-hydropyrazolo[1,5-a]pyrimidine-3-carboxylic acid ethyl ester
• 3-bromo-7-(2,4-dimethylphenyl)-5-(4-phenoxyphenyl)-2-phenyltetrahydropyrazolo[1,5-a]pyrimidine
• 7-(2,4-dimethylphenyl)-2-methylsulfanyl-5-(4-phenoxyphenyl)-tetrahydropyrazolo[1,5-a]pyrimidine-3-carbonitrile
• 7-(2,4-dimethylphenyl)-5-(4-phenoxyphenyl)-tetrahydropyrazolo[1,5-a]pyrimidine-3-carbonitrile
• 7-(3,4-dimethoxyphenyl)-5-(4-phenoxyphenyl)-tetrahydropyrazolo[1,5-a]pyrimidine-3-carboxylic acid ethyl ester
• 7-(3,4-dimethoxyphenyl)-2-methylsulfanyl-5-(4-phenoxyphenyl)-tetrahydropyrazolo[1,5-a]pyrimidine-3-carbonitrile
• 3-[3-cyano-7-(4-hydroxyphenyl)-6-methyltetrahydro-pyrazolo[1,5-a]pyrimidin-5-yl]-2-hydroxybenzoic acid
• 3-(3-cyano-5,5a,6,10b-tetrahydro-3H-1,4,10c-triazacyclopenta[c]fluoren-5-yl)-2-hydroxybenzoic acid; 3-(3-cyano-5,5a,6,10b-tetrahydro-4H-1,4,10c-triazacyclopenta[c]fluoren-5-yl)-2-hydroxybenzoic acid
• 3-(3-cyano-7-phenylsulfanyltetrahydropyrazolo[1,5-a]pyrimidin-5-yl]-2-hydroxybenzoic acid
• 3-[2-tert.-butyl-7-(4-chlorophenyl)-7-methyltetrahydropyrazolo[1,5-a]pyrimidin-5-yl]-2-hydroxybenzoic acid
• 5-(4-hydroxy-3-methoxyphenyl)-7-(4-hydroxyphenyl)-6-methyltetrahydropyrazolo[1,5-a]pyrimidine-3-carboxylic acid ethyl ester
• 5-(4-hydroxy-3-methoxyphenyl)-7-(4-hydroxyphenyl)-6-methyltetrahydropyrazolo[1,5-a]pyrimidine-3-carbonitrile
• 5-(4-hydroxy-3-methoxyphenyl)-5,5a,6,10b-tetrahydro-3H-1,4,10c-triazacyclopenta[c]fluorene-3-carboxylic acid ethyl ester; 5-(4-hydroxy-3-methoxyphenyl)-5,5a,6,10b-tetrahydro-4H-1,4,10c-triazacyclopenta-[c]fluorene-3-carboxylic acid ethyl ester
• 4-(2-tert.-butyl-5,5a,6,10b-tetrahydro-3H-1,4,10c-triazacyclopenta[c]fluoren-5-yl)-2-methoxyphenol; 4-(2-tert.-butyl-5,5a,6,10b-tetrahydro-4H-1,4,10c-triazacyclopenta[c]fluoren-5-yl)-2-methoxyphenol
• 5-(4-hydroxy-3-methoxyphenyl)-2-methylsulfanyl-5,5a,6,10b-tetrahydro-3H-1,4,10c-triazacyclo-penta[c]fluorene-3-carbonitrile; 5-(4-hydroxy-3-methoxyphenyl)-2-methylsulfanyl-5,5a,6,10b-tetrahydro-3H-1,4,10c-triazacyclopenta[c]fluorene-3-carbonitrile
• 5-(4-hydroxy-3-methoxyphenyl)-7-phenylsulfanyl-tetrahydropyrazolo[1,5-a]pyrimidine-3-carboxylic acid ethyl ester
• 4-(2-tert.-butyl-7-phenylsulfanyl-tetrahydro-pyrazolo[1,5-a]pyrimidin-5-yl)-2-methoxyphenol
• 4-(3-bromo-2-phenyl-7-phenylsulfanyl-tetrahydro-pyrazolo[1,5-a]pyrimidin-5-yl)-2-methoxyphenol
• 5-(2-hydroxy-3-methoxyphenyl)-7-phenylsulfanyl-tetrahydropyrazolo[1,5-a]pyrimidine-3-carboxylic acid ethyl ester
• 7-(4-chlorophenyl)-5-(2-hydroxy-3-methoxyphenyl)-7-methyltetrahydropyrazolo[1,5-a]pyrimidine-3-carboxylic acid ethyl ester
• 5-(4-hydroxybutyl)-5,5a,6,10b-tetrahydro-3H-1,4,10c-triazacyclopenta[c]fluorene-3-carbonitrile; 5-(4-hydroxybutyl)-5,5a,6,10b-tetrahydro-4H-1,4,10c-triazacyclopenta[c]fluorene-3-carbonitrile
• 5-(4-hydroxybutyl)-2-methylsulfanyl-7-phenyl-sulfanyltetrahydropyrazolo[1,5-a]pyrimidine-3-carbonitrile
• 5-(4-hydroxybutyl)-7-phenylsulfanyltetrahydro-pyrazolo[1,5-a]pyrimidine-3-carbonitrile
• 7-(4-chlorophenyl)-5-(4-hydroxybutyl)-7-methyltetrahydropyrazolo[1,5-a]pyrimidine-3-carbonitrile
• 5-butyl-2-methylsulfanyl-5,5a,6,10b-tetrahydro-3H-1,4,10c-triazacyclopenta[c]fluorene-3-carbonitrile; 5-butyl-2-methylsulfanyl-5,5a,6,10b-tetrahydro-4H-1,4,10c-triazacyclopenta[c]fluorene-3-carbonitrile
• 5-butyl-2-methylsulfanyl-7-phenylsulfanyltetrahydro-pyrazolo[1,5-a]pyrimidine-3-carbonitrile
• 5-butyl-7-phenylsulfanyl-tetrahydropyrazolo[1,5-a]pyrimidine-3-carbonitrile
• 5-butyl-7-(4-chlorophenyl)-7-methylsulfanyltetra-hydropyrazolo[1,5-a]pyrimidine-3-carbonitrile
• 5-cyclopropyl-7-(2,4-dimethylphenyl)-3-phenylazo-tetrahydropyrazolo[1,5-a]pyrimidin-2-ol
• 2-tert.-butyl-5-cyclopropyl-7-(2,4-dimethylphenyl)-tetrahydropyrazolo[1,5-a]pyrimidine
• 5-cyclopropyl-7-(2,4-dimethylphenyl)-2-methyl-sulfanyltetrahydro-pyrazolo[1,5-a]pyrimidine-3-carbonitrile
• 2-tert.-butyl-5-cyclopropyl-7-(3,4-dimethoxyphenyl)-tetrahydropyrazolo[1,5-a]pyrimidine
• 3-bromo-5-cyclopropyl-7-(3,4-dimethoxyphenyl)-2-phenyltetrahydropyrazolo[1,5-a]pyrimidine
• 5-cyclopropyl-7-(4-methoxyphenol)-tetrahydro-pyrazolo[1,5-a]pyrimidine-3-carboxylic acid ethyl ester
• 5-cyclopropyl-3,5,5a,6,7,11b-hexahydro-1,4,11c-triazacyclopenta[c]phenanthrene-3-carbonitrile
• 7-(2,4-dimethylphenyl)-5-pyridin-2-yl-tetrahydro-pyrazolo[1,5-a]pyrimidine-3-carboxylic acid ethyl ester
• 7-(2,4-dimethylphenyl)-3-phenylazo-5-pyridin-2-yl-tetrahydropyrazolo[1,5-a]pyrimidin-2-ol
• 3-bromo-7-(2,4-dimethylphenyl)-2-phenyl-5-pyridin-2-yl-tetrahydropyrazolo[1,5-a]pyrimidine
• 7-(2,4-dimethylphenyl)-2-methylsulfanyl-5-pyridin-2-yl-tetrahydropyrazolo[1,5-a]pyrimidine-3-carbonitrile
• 7-(3,4-dimethoxyphenyl)-2-methylsulfanyl-5-phenethyl-tetrahydropyrazolo[1,5-a]pyrimidine-3-carbonitrile
• 7-(3,4-dimethoxyphenyl)-5-phenethyl-tetrahydro-pyrazolo[1,5-a]pyrimidine-3-carbonitrile
• 5-cyclopropyl-7-(2-hydroxyethoxy)-tetrahydro-pyrazolo[1,5-a]pyrimidine-3-carboxylic acid ethyl ester
• 2-(2-tert.-butyl-5-cyclopropyltetrahydropyrazolo-[1,5-a]pyrimidin-7-yloxy)-ethanol
• 5-cyclopropyl-3,5,5a,6,7,8a-hexahydro-8-oxa-1,4,8b-triaza-as-indacene-3-carboxylic acid ethyl ester; 5-cyclopropyl-4,5,5a,6,7,8a-hexahydro-8-oxa-1,4,8b-triaza-as-indacene-3-carboxylic acid ethyl ester
• 5-cyclopropyl-3-phenylazo-3,5,5a,6,7,8a-hexahydro-8-oxa-1,4,8b-triaza-as-indacen-2-ol; 5-cyclopropyl-3-phenylazo-4,5,5a,6,7,8a-hexahydro-8-oxa-1,4,8b-triaza-as-indacen-2-ol
• 7-cyclohexyloxy-5-cyclopropyltetrahydropyrazolo-[1,5-a]pyrimidin-3-carboxylic acid ethyl ester
• 7-cyclohexyloxy-5-cyclopropyl-2-methylsulfanyl-tetrahydropyrazolo[1,5-a]pyrimidin-3-carbonitrile
• 7-(4-chlorophenyl)-5-cyclohexyltetrahydropyrazolo-[1,5-a]pyrimidin-3-carbonitrile
• 5-cyclohexyl-7-(2-hydroxyethoxy)-tetrahydro-pyrazolo[1,5-a]pyrimidine-3-carboxylic acid ethyl ester
• 5-cyclohexyl-3,5,5a,6,7,8a-hexahydro-8-oxa-1,4,8b-triaza-as-indacene-3-carboxylic acid ethyl ester; 5-cyclohexyl-4,5,5a,6,7,8a-hexahydro-8-oxa-1,4,8b-triaza-as-indacene-3-carboxylic acid ethyl ester
• 5-cyclohexyl-7-cyclohexyloxytetrahydropyrazolo [1,5-a]pyrimidine-3-carboxylic acid ethyl ester
• 7-(2,4-dimethylphenyl)-3-phenylazo-5-propyl-tetrahydropyrazolo[1,5-a]pyrimidin-2-ol
• 7-(2,4-dimethylphenyl)-2-methylsulfanyl-5-propyl-tetrahydropyrazolo[1,5-a]pyrimidine-3-carbonitrile
• 5-tert.-butyl-7-(2,4-dimethylphenyl)-tetrahydro-pyrazolo[1,5-a]pyrimidine-3-carboxylic acid ethyl ester
• 2,5-di-tert.-butyl-7-(3,4-dimethoxyphenyl)-tetrahydropyrazolo[1,5-a]pyrimidine
• 3-bromo-5-tert.-butyl-7-(3,4-dimethoxyphenyl)-2-phenyltetrahydropyrazolo[1,5-a]pyrimidine
• 2-[3-cyano-6,7-bis-(4-methoxyphenyl)-tetrahydropyrazolo[1,5-a]pyrimidin-5-yl]-cyclopropanecarboxylic acid ethyl ester
• 3-cyano-6,7-bis-(4-methoxyphenyl)-tetrahydro-pyrazolo[1,5-a]pyrimidine-5-carboxylic acid
• 4-[3-bromo-6-methyl-2-phenyl-5-(4-trifluoro-methylphenyl)-tetrahydropyrazolo[1,5-a]pyrimidin-7-yl]-phenol
• 7-(4-hydroxyphenyl)-6-methyl-2-methylsulfanyl-5-(4-trifluoro-methylphenyl)-tetrahydropyrazolo[1,5-a]pyrimidine-3-carbonitrile
• 7-(4-hydroxyphenyl)-6-methyl-5-(4-trifluoromethyl-phenyl)-tetrahydropyrazolo[1,5-a]pyrimidine-3-carbonitrile
• 7-phenyl-3-phenylazo-5-pyridin-2-yl-3,5,6,7-tetrahydropyrazolo[1,5-a]pyrimidin-2-ol
• 7-phenylsulfanyl-5-pyridin-2-yl-3,5,6,7-tetrahydropyrazolo[1,5-a]pyrimidine-3-carboxylic acid ethyl ester
• 3-phenylazo-7-phenylsulfanyl-5-pyridin-2-yl-3,5,6,7-tetrahydro-pyrazolo[1,5-a]pyrimidin-2-ol
• 3-bromo-7-phenylsulfanyl-5-pyridin-2-yl-3,5,6,7-tetrahydropyrazolo-[1,5-a]pyrimidine
• 7-phenylsulfanyl-5-pyridin-2-yl-3,5,6,7-tetrahydropyrazolo[1,5-a]-pyrimidine-3-carbonitrile
• 7-(3,4-dimethoxyphenyl)-2-methyl-sulfanyl-5-pyridin-2-yl-tetra-hydropyrazolo[1,5-a]pyrimidine-3-carbonitrile
• 3-bromo-7-(3,4-dimethoxyphenyl)-2-phenyl-5-pyridin-2-yl-tetrahydro-pyrazolo[1,5-a]pyrimidine
• 3-bromo-7-(3,4-dimethoxyphenyl)-2-phenyltetrahydro-pyrazolo[1,5-a]pyrimidine-5-carboxylic acid ethyl ester
• 3-bromo-7-(3,4-dimethoxyphenyl)-5-(5-nitrofuran-2-yl)-2-phenyl-tetrahydropyrazolo[1,5-a]pyrimidine
• 3-cyano-7-(3,4-dimethoxyphenyl)-2-methylsulfanyltetrahydropyrazolo[1,5-a]pyrimidine-5-carboxylic acid ethyl ester
• 3-cyano-7-(3,4-dimethoxyphenyl)-tetrahydropyrazolo-[1,5-a]-pyrimidine-5-carboxylic acid ethyl ester
• 7-(3,4-dimethoxyphenyl)-2-methylsulfanyl-5-(5-nitrofuran-2-yl)tetrahydropyrazolo[1,5-a]-pyrimidine-3-carbonitrile
• 7-(3,4-dimethoxyphenyl)-5-pyridin-2-yl-4,5,6,7-tetrahydropyrazolo-[1,5-a]pyrimidine-3-carboxylic acid ethyl ester

5. Process for the preparation of compounds of the general structure (I A) and/or (I B) as well as their pharmaceutically acceptable salts 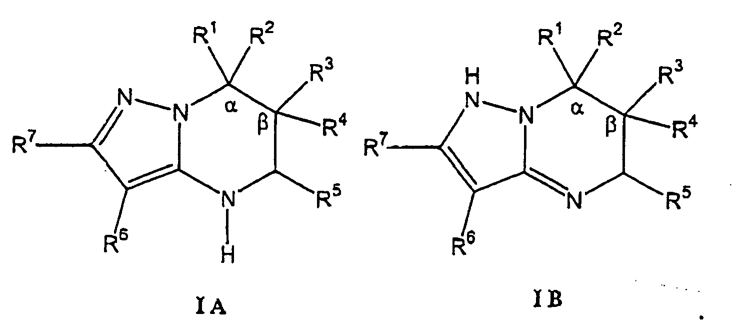 wherein R¹, R², R³, R⁴, R⁵, R⁶ and R⁷ are as defined in claim 1, **characterised in that**
a pyrazolamine of the general structure (IIIA) or (IIIB) 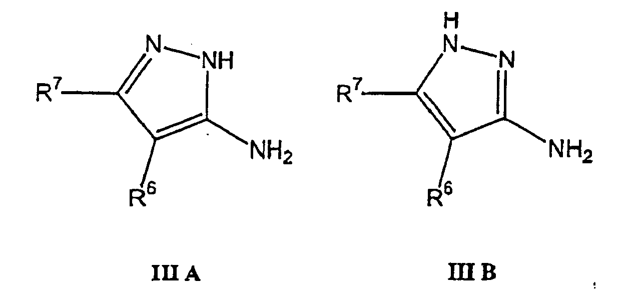 wherein R⁶ and R⁷ are as defined in this claim,
is reacted in the presence of an acid with an aldehyde of the general structure (IV) 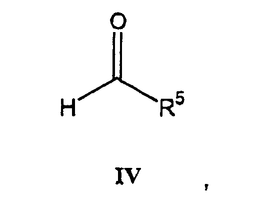 wherein
R⁵ is as defined in this claim,
and with an olefin of the general structure (V) 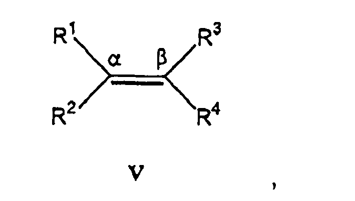 wherein R¹, R², R³ and R⁴ are as defined in this claim, with the proviso that if one of the radicals R¹ and R² together with one of the radicals R³ and R⁴ forms W; the end of W identified by α' is joined to the α-carbon atom of the olefin of the general structure (V), and the end of W identified by β' is joined to the β-carbon atom of the olefin of the general structure (V).

6. Process according to claim 5, **characterised in that** the reaction of the heterocyclylamine of the general structure (IIIA) or (IIIB) with the aldehyde of the general structure (IV) and the olefin of the general structure (V) is carried out in a one-pot process.

7. Process according to either one of claims 5 and 6, **characterised in that** the acid is trifluoroacetic acid.

8. Process according to any one of claims 5 to 7, **characterised in that** the reaction is carried out in an organic solvent at a temperature of 0° to 100°C and at a reaction time of 0.25 to 12 hours.

9. Process according to one of claims 5 to 8, **characterised in that** the reaction is carried out at a temperature of 15° to 40°C.

10. Medicament containing at least one compound of the general structure (I A) or (I B) 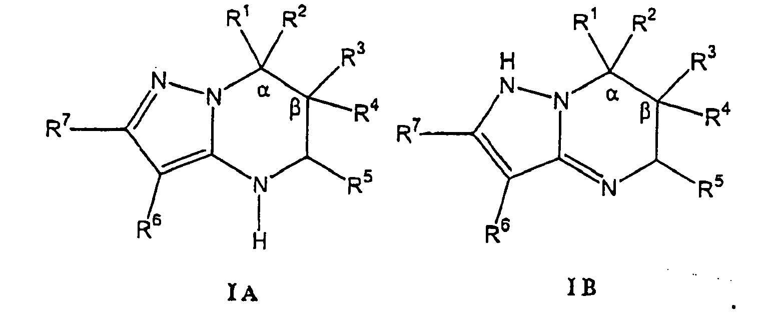 in the illustrated form or in the form of their acid(s) or their base(s) or in the form of one of their salts, in particular physiologically compatible salts, or in the form of one of their solvates, in particular the hydrate;
in the form of their racemate, pure stereoisomers, in particular enantiomers or diastereomers, or in the form of mixtures of the stereoisomers, in particular of the enantiomers or diastereomers, in an arbitrary mixture ratio;
wherein
R¹ and R² independently of one another denote H, O-R⁹, S-R¹⁰, C₁₋₆-[alk], aryl' or -(C₁₋₆-alkyl)-aryl', wherein the aryl' substituents R²⁹, R³⁰ and R³¹ independently of one another denote H, C₁₋₆₋[alk], F, Cl, Br, I, OH, O-C₁₋₆-[alk], O-aryl¹ or O-CH₂-aryl¹, wherein one of the radicals R¹ and R² is H and the other radical of R¹ and R² is not H, or in the case that one of the radicals R¹ and R² denotes aryl', the other radical of R¹ and R² denotes H or C₁₋₆₋alkyl,
R³ and R⁴ denote H, aryl', -CH₂-aryl' or unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, wherein at least one of the radicals R³ and R⁴ is H, or one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 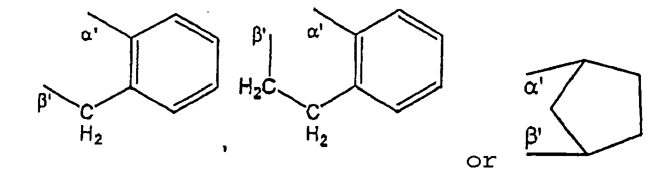 the end of W identified by α' is joined to the atom of the compound of the general structure (I A) or (I B) identified by α, and the end of W identified by P' is joined to the atom of the general structure (I A) or (I B) identified by β, the other radical of R¹ and R² is H or methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl and the other radical of R³ and R⁴ denotes H or methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl;
R⁵ denotes methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl that in each case is unsubstituted or singly substituted or multiply identically or differently substituted with a substituent selected from the group consisting of F, Cl, Br, I, -CN, -NC, -NH₂, -NO₂, -SH, -OH, -CO₂H and aryl', or denotes -(CH₂)ₖ-aryl', where k = 1,2,3 or 4, heterocyclyl or C(=O)R¹¹;
R⁶ denotes H, methyl, ethyl, -CN, fluorine, chlorine, bromine, iodine, -C(=O)R¹⁷ or -N=N-aryl¹;
R⁷ denotes H, aryl¹, OR¹⁸, S(O)_{q}R¹⁹, where q = 0, 1 or 2, or denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl,
R⁹ denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl or denotes -[(CH₂)ᵣ-O]ₛ-H where r = 1, 2, 3, 4, 5 or 6 and s = 1, 2, 3, 4, 5 or 6;
R¹⁰ denotes aryl';
R¹¹ denotes aryl' or OR²⁵;
R¹⁷ denotes OR²⁶;
R¹⁸ denotes H or methyl;
R¹⁹ denotes H, aryl¹, or in each case unsubstituted, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl;
R²⁵ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, where R²⁵ does not denote H if at the same time R¹ denotes aryl and R² denotes alkyl;
R²⁶ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl; and
Heterocyclyl denotes furan-2-yl, furan-3-yl, thien-2-yl, thien-3-yl, pyridin-2-yl, pyridin-3-yl or pyridin-4-yl, where furanyl, thienyl and pyridinyl are in each case unsubstituted or singly substituted or multiply identically or differently substituted with a substituent selected from the group consisting of F, Cl, Br, I, -CN, -NO₂, -CO₂H and -CO₂₋alkyl;
Aryl' denotes aryl¹, aryl² or aryl³;
Aryl¹ denotes 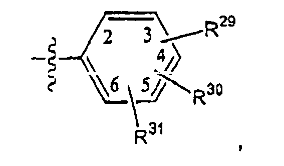
Aryl² denotes 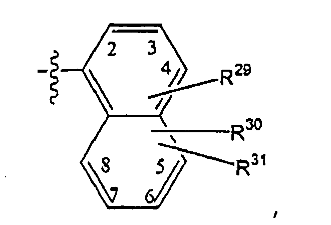
Aryl³ denotes 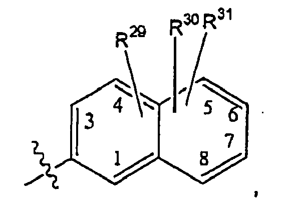
R²⁹, R³⁰ and R³¹ independently of one another denote H, C₁₋₆-[alk], C₃₋₈-cycloalkyl, (C₁₋₆ alkyl)-C₃₋₈₋[cycloalk], [ar], (C₁₋₆-alkyl)-[ar], [het], (C₁₋₆ alkyl)-[het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N-[ar], -(C=O)R³⁷, 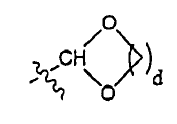 where d = 1, 2 or 3, or denotes -(C=S)R³⁷, and may be in any arbitrary ring position;
R³² and R³³ independently of one another denote H, -C₁₋₆₋[alk], -C₃₋₈- [cycloalk], -(C₁₋₆-alkyl)-C₃₋₈₋[cycloalk], -[ar], - (C₁₋₆-alkyl) - [ar], -[het], -(C₁₋₆-alkyl)-[het], (C=O)R³⁸, -[(CH₂)_{w}-O]_{z}-H or -[(CH₂)_{w}-O]_{z}-C₁₋₆-[alk] where w = 1, 2, 3 or 4 and z = 1, 2, 3, 4 or 5;
R³⁴ denotes C₁₋₆-[alk], -CH₂-[ar] or -(C=O)O-tert.-butyl;
R³⁵ and R³⁶ independently of one another denote C₁₋₆₋[alk] or together denote -(CH₂)-_{g} where g = 4 or 5;
R³⁷ denotes H, -C₁₋₆-[alk], -C₃₋₈-[cycloalk], - (C₁₋₆-alkyl)-C₃₋₈-[cycloalk], -[ar], -(C₁₋₆₋alkyl)-[ar], -[het], -(C₁₋₆-alkyl)-[het],-OR³⁹, -NH₂, -NHR³⁴, -NR³⁵R³⁶;
R³⁸ denotes H, -C₁₋₆-[alk], -C₃₋₈-[cycloalk], - (C₁₋₆-alkyl)-C₃₋₈-[cycloalk], -[ar], -(C₁₋₆₋alkyl)-[ar];
and
R³⁹ denotes H, -C₁₋₆-[alk], -C₃₋₈-[cycloalk], - (C₁₋₆-alkyl) -C₃₋₈- [cycloalk], - [ar], - (C₁₋₆₋alkyl)-[ar], -[het] or -(C₁₋₆-alkyl)-[het] .
[Alk] denotes an acyclic, saturated or unsaturated, branched or straight-chain hydrocarbon radical that may be unsubstituted or singly substituted or multiply identically or differently substituted,
wherein the substituents may be selected from the group consisting of F, Cl, Br, I, -CN, -NC, -NH₂, -NO₂, -SH, -OH, -CO₂H and -CO₂-alkyl;
[Cycloalk] denotes a cyclic, saturated or unsaturated hydrocarbon radical that may be unsubstituted or singly substituted or multiply identically or differently substituted and optionally benzo-condensed,
wherein the substituents may be selected from the group consisting of F, Cl, Br, I, -CN, -NC, -NH₂, -NO₂, -SH, -OH, -CO₂H and -CO₂-alkyl;
[Ar] denotes an unsubstituted radical selected from the group consisting of phenyl, naphthyl, anthracenyl and biphenyl;
and
(Het) denotes an unsubstituted heterocyclyl radical slected from the group consisting of pyrrolidinyl, tetrahydrofuryl, piperidinyl, piperazinyl and morpholinyl or denotes an unsubstituted heteroaryl radical selected from the group consisting of pyrrolyl, pyrazolyl, imidazolyl, pyridazinyl, pyrimidinyl, pyrazinyl, furanyl, thienyl and pyridinyl.

11. Use of a compound of the general structure (I A) or (I B) 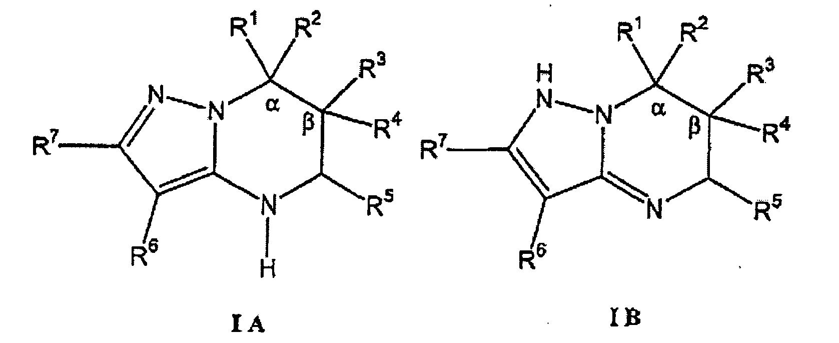 in the illustrated form or in the form of their acid(s) or their base(s) or in the form of one of their salts, in particular physiologically compatible salts, or in the form of one of their solvates, in particular the hydrate;
in the form of their racemate, pure stereoisomers, in particular enantiomers or diastereomers, or in the form of mixtures of the stereoisomers, in particular of the enantiomers or diastereomers, in an arbitrary mixture ratio;
wherein
R¹ and R² independently of one another denote H, O-R⁹, S-R¹⁰, C₁₋₆-[alk], aryl' or - (C₁₋₆-alkyl) -aryl', wherein the aryl' substituents R²⁹, R³⁰ and R³¹ independently of one another denote H, C₁₋₆₋[alk], F, Cl, Br, I, OH, O-C₁₋₆-[alk], O-aryl¹ or O-CH₂-aryl¹, wherein one of the radicals R¹ and R² is H and the other radical of R¹ and R² is not H, or in the case that one of the radicals R¹ and R² denotes aryl', the other radical of R¹ and R² denotes H or C₁₋₆₋alkyl,
R³ and R⁴ denote H, aryl', -CH₂-aryl' or unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, wherein at least one of the radicals R³ and R⁴ is H, or
one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 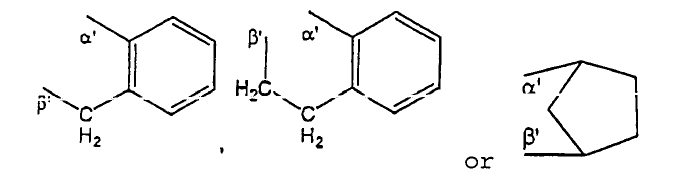 the end of W identified by α' is joined to the atom of the compound of the general structure (I A) or (I B) identified by α, and the end of W identified by β' is joined to the atom of the general structure (I A) or (I B) identified by β, the other radical of R¹ and R² is H or methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl and the other radical of R³ and R⁴ denotes H or methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl;
R⁵ denotes methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl that in each case is unsubstituted or singly substituted or multiply identically or differently substituted with a substituent selected from the group consisting of F, Cl, Br, I, -CN, -NC, -NH₂, -NO₂, -SH, -OH, -CO₂H and -CO₂alkyl, aryl', or denotes -(CH₂)ₖ-aryl', where k = 1,2,3 or 4, heterocyclyl or C(=O)R¹¹;
R⁶ denotes H, methyl, ethyl, -CN, fluorine, chlorine, bromine, iodine, -C(=O)R¹⁷ or -N=N-aryl¹;
R⁷ denotes H, aryl¹, OR¹⁸, S(O)_{q}R¹⁹, where q = 0, 1 or 2, or denotes unsubstituted methyl, ethyl, n-propyl, 2-propyl, n-butyl, isobutyl, sec.-butyl, tert.-butyl, n-amyl,
R⁹ isoamyl, sec.-amyl, n-hexyl, isohexyl or sec.-hexyl, denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl or denotes -[(CH₂)ᵣ-O]ₛ-H where r = 1, 2, 3, 4, 5 or 6 and s = 1, 2, 3, 4, 5 or 6;
R¹⁰ denotes aryl';
R¹¹ denotes aryl' or OR²⁵;
R¹⁷ denotes OR²⁶;
R¹⁸ denotes H or methyl;
R¹⁹ denotes H, aryl¹, or in each case unsubstituted, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl;
R²⁵ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, where R²⁵ does not denote H if at the same time R¹ denotes aryl and R² denotes alkyl;
R²⁶ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl; and
Heterocyclyl denotes furan-2-yl, furan-3-yl, thien-2-yl, thien-3-yl, pyridin-2-yl, pyridin-3-yl or pyridin-4-yl, where furanyl, thienyl and pyridinyl are in each case unsubstituted or singly substituted or multiply identically or differently substituted with a substituent selected from the group consisting of F, Cl, Br, I, -CN, -NO₂, -CO₂H and -CO₂₋alkyl;
Aryl' denotes aryl¹, aryl² or aryl³;
Aryl¹ denotes 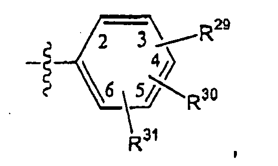
Aryl² denotes 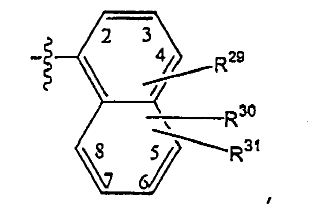
Aryl³ denotes 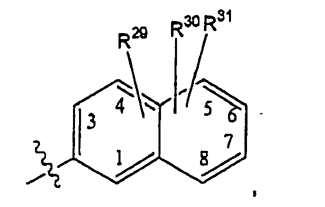
R²⁹, R³⁰ and R³¹ independently of one another denote H, C₁₋₆-[alk], C₃₋₈-cycloalkyl, (C₁₋₆ alkyl)-C₃₋₈₋[cycloalk], [ar], (C₁₋₆-alkyl)-[ar], [het], (C₁₋₆ alkyl)-[het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N-[ar], -(C=O)R³⁷, 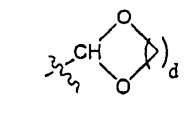 where d = 1, 2 or 3, or denotes -(C=S)R³⁷, and may be in any arbitrary ring position;
R³² and R³³ independently of one another denote H, -C₁₋₆₋[alk], -C₃₋₈-[cycloalk], -(C₁₋₆-alkyl)-C₃₋₈₋[cycloalk], -[ar], -(C₁₋₆-alkyl)-[ar], -[het], -(C₁₋₆-alkyl)-[het], (C=O)R³⁸, - [(CH₂)_{w}-O]_{z}-H or - [(CH₂)_{w}-O]_{z}-C₁₋₆-[alk] where w = 1, 2, 3 or 4 and z = 1, 2, 3, 4 or 5;
R³⁴ denotes C₁₋₆-[alk], -CH₂-[ar] or -(C=O)O-tert.-butyl;
R³⁵ and R³⁶ independently of one another denote C₁₋₆₋[alk] or together denote -(CH₂)-_{g} where g = 4 or 5;
R³⁷ denotes H, -C₁₋₆-[alk], -C₃₋₈-[cycloalk], -(C₁₋₆-alkyl)-C₃₋₈-[cycloalk], -[ar], -(C₁₋₆-alkyl)-[ar], -[het], - (C₁₋₆-alkyl)-[het], -OR³⁹, -NH₂, -NHR³⁴, -NR³⁵R³⁶;
R³⁸ denotes H, -C₁₋₆-[alk], -C₃₋₈- [cycloalk], -(C₁₋₆-alkyl) -C₃₋₈- [cycloalk], -[ar], -(C₁₋₆-alkyl)-[ar] ; and
R³⁹ denotes H, -C₁₋₆-[alk], -C₃₋₈-[cycloalk], -(C₁₋₆-alkyl)-C₃₋₈-[cycloalk], -[ar], -(C₁₋₆-alkyl)-[ar], -[het] or -(C₁₋₆-alkyl)-[het] .
[Alk] denotes an acyclic, saturated or unsaturated, branched or straight-chain hydrocarbon radical that may be unsubstituted or singly substituted or multiply identically or differently substituted, wherein the substituents may be selected from the group consisting of F, Cl, Br, I, -CN, -NC, -NH₂, -NO₂, -SH, -OH, -CO₂H and -CO₂-alkyl;
[Cycloalk] denotes a cyclic, saturated or unsaturated hydrocarbon radical that may be unsubstituted or singly substituted or multiply identically or differently substituted and optionally benzo-condensed,
wherein the substituents may be selected from the group consisting of F, Cl, Br, I, -CN, -NC, -NH₂, -NO₂, -SH, -OH, -CO₂H and -CO₂-alkyl;
[Ar] denotes an unsubstituted radical selected from the group consisting of phenyl, naphthyl, anthracenyl and biphenyl; and
(Het) denotes an unsubstituted heterocyclyl radical slected from the group consisting of pyrrolidinyl, tetrahydrofuryl, piperidinyl, piperazinyl and morpholinyl or denotes an unsubstituted heteroaryl radical selected from the group consisting of pyrrolyl, pyrazolyl, imidazolyl, pyridazinyl, pyrimidinyl, pyrazinyl, furanyl, thienyl and pyridinyl,
for the production of a medicament for pain relief.

12. Use of a compound of the general structure (I A) or (I B), 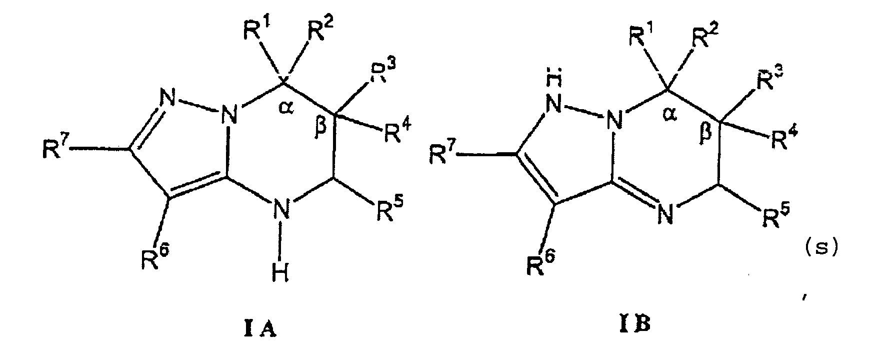 in particular physiologically compatible salts, or in the form of one of its solvates, in particular hydrates;
in the form of its racemate, of the pure stereoisomers, in particular enantiomers or diastereomers, or in the form of mixtures of the stereoisomers, in particular of the enantiomers or diastereomers, in an arbitrary mixture ratio;
wherein R¹, R², R³, R⁴, R⁵, R⁶, R⁷ and R⁸ are as defined in claim 11;
for the production of a medicament for the treatment and/or prophylaxis of epilepsy, schizophrenia, neurodegenerative conditions, in particular Alzheimer's disease, Huntington's disease and Parkinson's disease, cerebral ischaemias and infarcts, psychoses due to raised amino acid levels, cerebral oedemas, insufficiency states of the central nervous system, in particular in hypoxias and anoxias, AIDS dementia, encephalomyelitis, Tourette's syndrome, perinatal asphyxia and/or tinnitus.

13. Use of a compound according to formula (I A) and/or (I B) 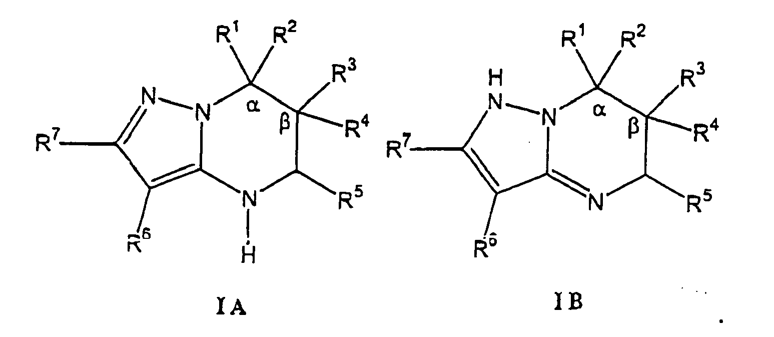 in the illustrated form or in the form of its acid(s) or its base(s) or in the form of one of its salts, in particular physiologically compatible salts, or in the form of one of its solvates, in particular hydrates; in the form of its racemate, of the pure stereoisomers, in particular enantiomers or diastereomers, or in the form of mixtures of the stereoisomers, in particular of the enantiomers or diastereomers, in an arbitrary mixture ratio;
wherein
R¹ and R² independently of one another denote H, O-R⁹, S-R¹⁰, C₁₋₆-[alk], aryl' or -(C₁₋₆-alkyl)-aryl', wherein the aryl' substituents R²⁹, R³⁰ and R³¹ independently of one another denote H, C₁₋₆₋[alk], F, Cl, Br, I, OH, O-C₁₋₆-alkyl, O-aryl¹ or O-CH₂-aryl¹, wherein one of the radicals R¹ and R² is H and the other radical of R¹ and R² is not H, or in the case that one of the radicals R¹ and R² denotes aryl', the other radical of R¹ and R² denotes H or C₁₋₆₋alkyl,
R³ and R⁴ denote H, aryl', -CH₂-aryl' or unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, wherein at least one of the radicals R³ and R⁴ is H, or
one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 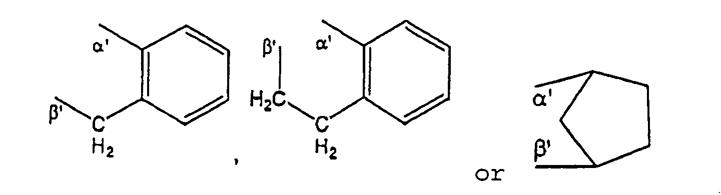 the end of W identified by α' is joined to the atom of the compound of the general structure (I A) or (I B) identified by α, and the end of W identified by β' is joined to the atom of the general structure (I A) or (I B) identified by β, the other radical of R¹ and R² is H or methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl and the other radical of R³ and R⁴ denotes H or methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl;
R⁵ denotes methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl that in each case is unsubstituted or singly substituted or multiply identically or differently substituted with a substituent selected from the group consisting of F, Cl, Br, I, -CN, -NC, -NH₂, -NO₂, -SH, -OH, -CO₂H and aryl', or denotes - (CH₂)ₖ-aryl', where k = 1, 2, 3 or 4, heterocyclyl or C(=O)R¹¹;
R⁶ denotes H, methyl, ethyl, -CN, fluorine, chlorine, bromine, iodine, -C(=O)R¹⁷ or -N=N-aryl¹;
R⁷ denotes H, aryl¹, OR¹⁸, S(O)_{q}R¹⁹, where q = 0, 1 or 2, or denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl,
R⁹ denotes unsubstituted methyl, ethyl, n-propyl, 2-propyl, n-butyl, isobutyl, sec.-butyl, tert.-butyl, n-amyl, isoamyl, sec.-amyl, n-hexyl, isohexyl, sec.-hexyl, cyclopropyl, cyclobutyl, cyclopentyl, cyclohexyl or cycloheptyl or denotes-[(CH₂)ᵣ-O]ₛ-H where r = 1, 2 , 3, 4, 5 or 6 and s = 1, 2, 3, 4, 5 or 6;
R¹⁰ denotes aryl';
R¹¹ denotes aryl' or OR²⁵;
R¹⁷ denotes OR²⁶;
R¹⁸ denotes H or methyl;
R¹⁹ denotes H, aryl¹, or in each case unsubstituted, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl;
R²⁵ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl, where R²⁵ does not denote H if at the same time R¹ denotes aryl and R² denotes alkyl;
R²⁶ denotes H, methyl, ethyl, n-propyl, 2-propyl, n-butyl, isobutyl, sec.-butyl, tert.-butyl, n-amyl, isoamyl, sec.-amyl, n-hexyl, isohexyl or sec.-hexyl; and
Heterocyclyl denotes furan-2-yl, furan-3-yl, thien-2-yl, thien-3-yl, pyridin-2-yl, pyridin-3-yl or pyridin-4-yl, where furanyl, thienyl and pyridinyl are in each case unsubstituted or singly substituted or multiply identically or differently substituted with a substituent selected from the group consisting of F, Cl, Br, I, -CN, -NO₂, -CO₂H and -CO₂-alkyl;
Aryl' denotes aryl¹, aryl² or aryl³;
Aryl¹ denotes 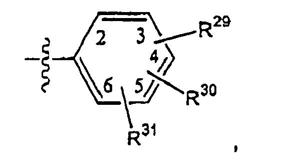
Aryl² denotes 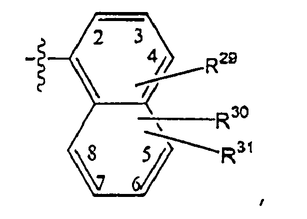
Aryl³ denotes 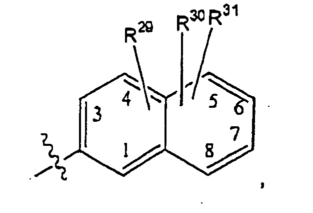
R²⁹, R³⁰ and R³¹ independently of one another denote H, C₁₋₆-[alk], C₃₋₈-cycloalkyl, (C₁₋₆ alkyl)-C₃₋₈₋[cycloalk], [ar], (C₁₋₆-alkyl) - [ar], [het], (C₁₋₆ alkyl) - [het], F, Cl, Br, I, -CN, -NC, -OR³², -SR³³, -NO, -NO₂, NH₂, NHR³⁴, NR³⁵R³⁶, -N-OH, -N-OC₁₋₆-[alk], -NHNH₂, -N=N-[ar], -(C=O)R³⁷, 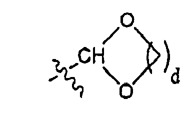 where d = 1, 2 or 3, or denotes -(C=S)R³⁷, and may be in any arbitrary ring position;
R³² and R³³ independently of one another denote H,-C₁₋₆- [alk], -C₃₋₈- [cycloalk], - (C₁₋₆-alkyl) -C₃₋₈-[cycloalk], - [ar], - (C₁₋₆-alkyl) - [ar], - [het], - (C₁₋₆-alkyl)-[het], (C=O)R³⁸, - [(CH₂) _{w}-O] _{z}-H or - [(CH₂)_{w}-O]_{z}-C₁₋₆- [alk] where w = 1, 2, 3 or 4 and z = 1, 2, 3, 4 or 5;
R³⁴ denotes C₁₋₆-[alk], -CH₂-[ar] or -(C=O) O-tert.-butyl;
R³⁵ and R³⁶ independently of one another denote C₁₋₆₋[alk] or together denote -(CH₂)-_{g} where g = 4 or 5;
R³⁷ denotes H, -C₁₋₆-[alk], -C₃₋₈- [cycloalk], - (C₁₋₆-alkyl)-C₃₋₈-[cycloalk], -[ar], - (C₁₋₆₋alkyl)-[ar], -[het], - (C₁₋₆-alkyl)-[het], -OR³⁹, -NH₂, -NHR³⁴, -NR³⁵R³⁶;
R³⁸ denotes H, -C₁₋₆-[alk], -C₃₋₈-[cycloalk], - (C₁₋₆-alkyl)-C₃₋₈-[cycloalk], -[ar], - (C₁₋₆₋alkyl)-[ar]; and
R³⁹ denotes H, -C₁₋₆-[alk], -C₃₋₈-[cycloalk], - (C₁₋₆-alkyl)-C₃₋₈-[cycloalk], -[ar], -(C₁₋₆₋alkyl)-[ar], -[het] or -(C₁₋₆-alkyl)-[het].
[Alk] denotes an acyclic, saturated or unsaturated, branched or straight-chain hydrocarbon radical that may be unsubstituted or singly substituted or multiply identically or differently substituted,
wherein the substituents may be selected from the group consisting of F, Cl, Br, I, -CN, -NC, -NH₂, -NO₂, -SH, -OH, -CO₂H and -CO₂-alkyl;
[Cycloalk] denotes a cyclic, saturated or unsaturated hydrocarbon radical that may be unsubstituted or singly substituted or multiply identically or differently substituted and optionally benzo-condensed,
wherein the substituents may be selected from the group consisting of F, Cl, Br, I, -CN, -NC, -NH₂, -NO₂, -SH, -OH, -CO₂H and -CO₂-alkyl;
[Ar] denotes an unsubstituted radical selected from the group consisting of phenyl, naphthyl, anthracenyl and biphenyl;
and
(Het) denotes an unsubstituted heterocyclyl radical sleeted from the group consisting of pyrrolidinyl, tetrahydrofuryl, piperidinyl, piperazinyl and morpholinyl or denotes an unsubstituted heteroaryl radical selected from the group consisting of pyrrolyl, pyrazolyl, imidazolyl, pyridazinyl, pyrimidinyl, pyrazinyl, furanyl, thienyl and pyridinyl,
as a ligand of nucleoside transport proteins and/or of adenosine kinase and/or of adenosine diaminase and/or of A₁ and/or A₂ and/or A₃ receptors.

14. Use of a compound according to formula (I A) and/or (I B) 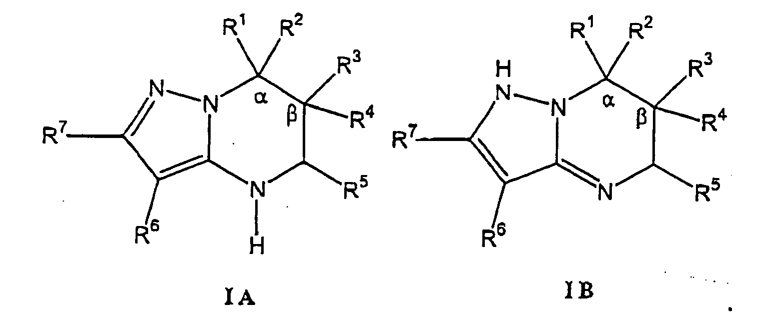 in the illustrated form or in the form of its acid(s) or its base(s) or in the form of one of its salts, in particular physiologically compatible salts, or in the form of one of its solvates, in particular hydrates; in the form of its racemate, of the pure stereoisomers, in particular enantiomers or diastereomers, or in the form of mixtures of the stereoisomers, in particular of the enantiomers or diastereomers, in an arbitrary mixture ratio;
wherein R¹, R², R³, R⁴, R⁵, R⁶ and R⁷ are as defined in claim 13; for the production of a medicament for the prevention and/or treatment of conditions and/or diseases that are influenced by a stimulation and/or inhibition of nucleoside transport proteins and/or adenosine kinase and/or adenosine deaminase and/or A₁ and/or A₂ and/or A₃ receptors.

15. Use according to one of claims 13 and 14 for the production of a medicament for the prevention and/or treatment of pain, neuropathic pain, respiratory pathway conditions, cancer, cardiac arrhythmias, ischaemias, epilepsy, Huntington's disease, malfunctions and diseases of the immune system, inflammatory conditions and diseases, neonatal hypoxia, neurodegenerative conditions, Parkinson's disease, kidney failure, schizophrenia, sleep disturbances, strokes, thromboses, urinary incontinence, diabetes, psoriasis, septic shock, cerebral trauma, glaucoma and/or congestive insufficiency.

16. Use according to any one of claims 13 to 15, **characterised in that**
R¹ and R² independently of one another denote H,O-CH₂₋CH₂-OH, O-cyclohexyl, S-phenyl, methyl, phenyl, 3-fluorophenyl, 3-bromophenyl, 4-bromophenyl, 4-chlorophenyl, 4-fluorophenyl, 3-methylphenyl, 4-hydroxyphenyl, 4-methoxyphenyl, 2,4-dimethyl-phenyl, 3,4-dimethoxyphenyl, 2,3,4-trimethoxy-phenyl, 2-naphthyl or -CH₂-phenyl,
R³ and R⁴ denote H, methyl or 4-methoxyphenyl, where at least one of the radicals R³ and R⁴ is H,
or
one of the radicals R¹ and R² together with one of the radicals R³ and R⁴ form W, where W denotes α'-CH=CH-CH₂-β', α'-CH=CH-CH₂-CH₂-β', α'-O-(CH₂)ₘ-β' where m = 2, 3, 4 or 5, 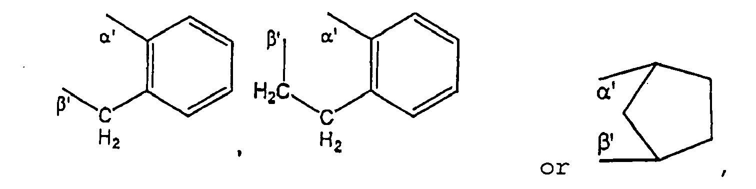 the end of W identified by α' is joined to the atom of the compound of the general formula (I A) and/or (I B) identified by α, and the end of W identified by β' is joined to the atom of the compound of the general structure (I A) and/or (I B) identified by β, and the other radical of R¹ and R² and the other radical of R³ and R⁴ in each case denote H;
R⁵ denotes n-propyl, n-butyl, tert.-butyl, -(CH₂)₄-OH, cyclopropyl, cycloprop-2-yl-1-carboxylic acid ethyl ether, cyclohexyl, 4-trifluorophenyl, 4-phenoxyphenyl, 2-hydroxy-3-methoxyphenyl, 4-hydroxy-3-methoxyphenyl, 3-carboxy-2-hydroxy-phenyl, -(CH₂)₂-phenyl, 5-carboxyfuran-2-yl, 5-methylfuran-2-yl, 5-nitrofuran-2-yl, 5-nitro-thien-2-yl, pyridin-2-yl, pyridin-3-yl, C(=O)-phenyl, C(=O)OH or C(=O)-ethyl, where R⁵ does not denote C(=O)OH if at the same time R¹ denotes aryl and R² denotes alkyl;
R⁶ denotes H, -CN, bromo, -C(=O)OH, -C(=O)Oethyl or -N=N-phenyl;
R⁷ denotes H, phenyl, OH, -S-methyl, -SO₂₋(4-nitrophenyl) or tert.-butyl.

17. Pharmaceutical composition that contains at least compound of the general structure (I A) or (I B), 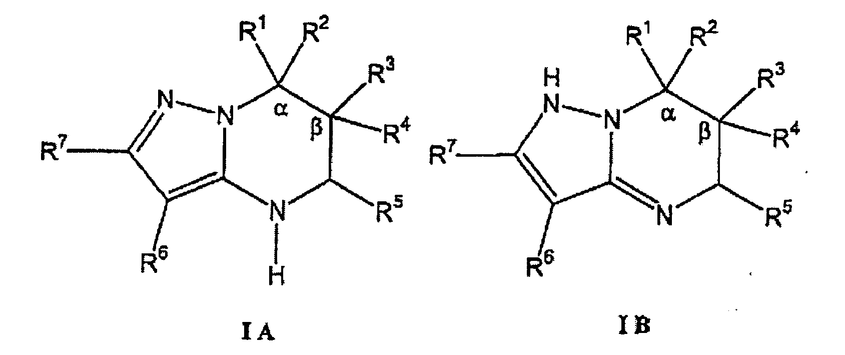 in the illustrated form or in the form of their acid(s) or their base(s) or in the form of one of their salts, in particular physiologically compatible salts, or in the form of one of their solvates, in particular the hydrate;
in the form of their racemate, pure stereoisomers, in particular enantiomers or diastereomers, or in the form of mixtures of the stereoisomers, in particular of the enantiomers or diastereomers, in an arbitrary mixture ratio,
wherein R¹, R², R³ and R⁴ are as defined in claim 1, as well as at least one pharmaceutical auxiliary substance.

## Revendications

1. Composé de formule générale (IA) ou (IB), 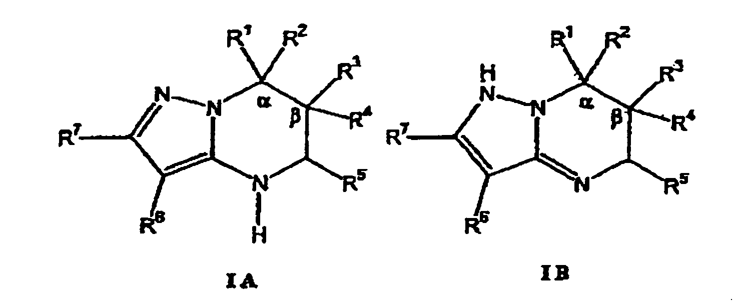 sous la forme représentée ou sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
formule dans laquelle
R¹ et R² représentent, indépendamment l'un de l'autre, H, un groupe O-R⁹, S-R¹⁰, un reste [Alk en C₁ à C₆], aryle' ou -(alkyle en C₁ à C₈)-aryle', les substituants R²⁹, R³⁰ et R³¹ du reste aryle' étant, indépendamment les uns des autres, H, [Alk en C₁ à C₆], F, Cl, Br, I, OH, O-[Alk en C₁ à C₆], O-aryle¹ ou O-CH₂-aryle¹, l'un des restes R¹ et R² représentant H et l'autre de ces deux restes ne représentant pas H ou bien, au cas où l'un des restes R¹ et R² désigne un reste aryle', l'autre de ces deux restes représente H ou un reste alkyle en C₁ à C₆,
R³ et R⁴ représentent H, un reste aryle', -CH₂-aryle' ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle non substitué,
l'un au moins des restes R³ et R⁴ représentant H, ou bien l'un des restes R¹ et R² forme un groupe W conjointement avec l'un des restes R³ et R⁴,
W étant un groupe α'-CH=CH-CH₂-β', α'-CH=CH-CH₂-CH₂-β', α'-O-(CH₂)ₘ-β' où m a la valeur 2, 3, 4 ou 5, 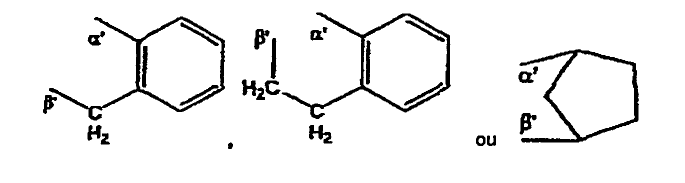 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² désigne H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre reste de R³ et R⁴ désigne H ou un reste méthyle, éthyle, n-propyle, 2-propyle, n-butyle, isobutyle, sec.-butyle, tertio-butyle, n-amyle, isoamyle, sec.-amyle, n-hexyle, isohexyle, sec.-hexyle ;
R⁵ est un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclo-propyle, cyclobutyle, cyclopentyle, cyclo-hexyle ou cycloheptyle, chacun de ces restes étant non substitué ou portant un ou plusieurs substituants identiques ou différents choisis dans le groupe comprenant F, Cl, Br, I, -CN, -NC, -NH₂, -NO₂, -SH, -OH, -CO₂H et -CO₂alkyle, aryle' ou -(CH₂)ₖ₋aryle', où k a la valeur 1, 2, 3 ou 4, hétérocyclyle ou C(=O)R¹¹ ;
R⁶ représente H, un reste méthyle, éthyle, -CN, le fluor, le chlore, le brome, l'iode, un groupe -C(=O)R¹⁷ ou -N=N-aryle¹ ;
R⁷ représente H, un reste aryle¹, un groupe OR¹⁸, S(O)_{q}R¹⁹, où q a la valeur 0, 1 ou 2, ou un reste méthyle non substitué, éthyle, n-propyle, 2-propyle, n-butyle, isobutyle, sec.-butyle, tertiobutyle, n-amyle, iso-amyle, sec.-amyle, n-hexyle, isohexyle ou sec.-hexyle,
R⁹ désigne un reste méthyle non substitué, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]ₛ-H dans lequel r a la valeur 1, 2, 3, 4, 5 ou 6 et s a la valeur 1, 2, 3, 4, 5 ou 6 ;
R¹⁰ est un reste aryle' ;
R¹¹ est un reste aryle' ou un groupe OR²⁵ ;
R¹⁷ est un groupe OR²⁶ ;
R¹⁸ représente H ou un groupe méthyle ;
R¹⁹ représente H, un reste aryle¹ ou un reste méthyle, éthyle, n-propyle, 2-propyle, n-butyle, isobutyle, sec.-butyle, tertio-butyle, n-amyle, isoamyle, sec.-amyle, n-hexyle, isohexyle ou sec.-hexyle chacun non substitué ;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁶ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ; et
Hétérocyclyle désigne un groupe furanne-2-yle, furanne-3-yle, thiène-2-yle, thiène-3-yle, pyridine-2-yle, pyridine-3-yle ou pyridine-4-yle, les groupes furannyle, thiényle et pyridinyle étant chacun non substitués ou portant chacun un ou plusieurs substituants identiques ou différents choisis dans le groupe comprenant F, Cl, Br, I, -CN, -NO₂, -CO₂H et -CO₂alkyle ;
Aryle' désigne un reste aryle¹, aryle² ou aryle³ ;
aryle¹ représente 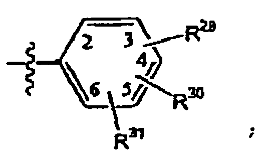
aryle² représente 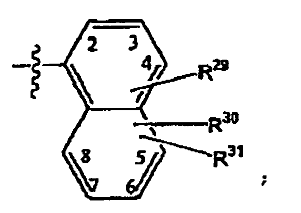
aryle³ représente 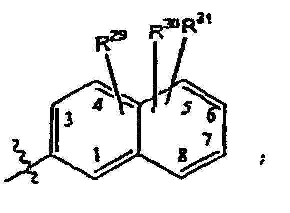
R²⁹, R³⁰ et R³¹ représentent, indépendamment les uns des autres, H, un reste [Alk en C₁ à C₆], [cycloalk en C₃ à C₈], -(alkyle en C₁ à C₆) - [cycloalk en C₃ à C₈], [Ar], - (alkyle en C₁ à C₆)-[Ar], [Het], - (alkyle en C₁ à C₆)-[Het], F, Cl, Br, I, -CN, -NC, -OR³², -SR³³, -NO, -NO₂, NH₂, NHR³⁴, NR³⁵R³⁶, -N-OH, -N-O[Alk en C₁ à C₆], -NHNH₂, -N=N-[Ar], - (C=O)R³⁷, 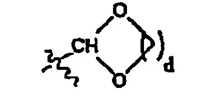 où d a la valeur 1, 2 ou 3, ou un groupe -(C=S)R³⁷, et peuvent occuper n'importe quelle position du noyau,
R³² et R³³ représentent, indépendamment l'un de l'autre, H, un reste [Alk en C₁ à C₆], [cycloalk en C₃ à C₈], -(alkyle en C₁ à C₆)-[cycloalk en C₃ à C₈], -[Ar], - (alkyle en C₁ à C₆)-[Ar], -[Het], -(alkyle en C₁ à C₆)-[Het], (C=O)R³⁸, -[(CH₂)_{w}-O]_{z}-H ou -[(CH₂)_{w}- O]_{z}-[Alk en C₁ à C₆], avec w = 1, 2, 3 ou 4 et z = 1, 2, 3, 4 ou 5 ;
R³⁴ est un reste [Alk en C₁ à C₆], -CH₂-[Ar] ou -(C=O)O-tertiobutyle ;
R³⁵ et R³⁶ représentent, indépendamment l'un de l'autre, un reste [Alk en C₁ à C₆] ou forment ensemble un reste -(CH₂)_{g}- où g a la valeur 4 ou 5 ;
R³⁷ représente H, un reste -[Alk en C₁ à C₆], - [cycloalk en C₃ à C₈], -(alkyle en C₁ à C₆)-[cycloalk en C₃ à C₈], -[Ar], -(alkyle en C₁ à C₆)-[Ar], -[Het], - (alkyle en C₁ à C₆)-[Het], -OR³⁹, -NH₂, -NHR³⁴, -NR³⁵R³⁶ ;
R³⁸ représente H, un reste -[Alk en C₁ à C₆], - [cycloalk en C₃ à C₈], -(alkyle en C₁ à C₆)-[cycloalk en C₃ à C₈], -[Ar], -(alkyle en C₁ à C₆)-[Ar] ;
et
R³⁹ représente H, un reste [Alk en C₁ à C₆], - [cycloalk en C₃ à C₈], -(alkyle en C₁ à C₆)-[cycloalk en C₃ à C₈], -[Ar], - (alkyle en C₁ à C₆)-[Ar], - [Het] ou - (alkyle en C₁ à C₆)-[Het],
[Alk] désigne un reste hydrocarboné acyclique, saturé
ou non saturé, ramifié ou à chaîne droite, qui est non substitué ou qui peut être substitué une ou plusieurs fois identiques ou différentes,
les substituants pouvant être choisis dans le groupe des substituants F, Cl, Br, I, -CN, -NC, -NH₂, -NO₂, -SH, -OH, -CO₂H et -CO₂-alkyle ;
[cycloalk] désigne un reste hydrocarboné cyclique, saturé ou non saturé qui est non substitué ou qui peut être substitué une ou plusieurs fois identiques ou différentes et, le cas échéant, condensé au benzène,
les substituants pouvant être choisis dans le groupe comprenant F, Cl, Br, I, -CN, -NC, -NH₂, -NO₂, -SH, -OH, -CO₂H et -CO₂-alkyle ;
[Ar] désigne un reste non substitué choisi dans le groupe constitué de phényle, naphtyle, anthracényle et biphényle ;
[Het] désigne un reste hétérocyclyle non substitué choisi dans le groupe comprenant les restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle,
en excluant les composés :
4,5,6,7-tétrahydro-2-méthyl-5,7-diphénylpyrazolo[1,5-a]-pyrimidine,
4,5,6,7-tétrahydro-2,5-diméthyl-7-phénylpyrazolo[1,5-a]-pyrimidine,
4,5,6,7-tétrahydro-5,7-diméthyl-3-phénylpyrazolo[1,5-a]-pyrimidine,
4,5,6,7-tétrahydro-2,5,7-triméthylpyrazolo[1,5-a]-pyrimidine,
4,5,6,7-tétrahydro-5,7-diméthyl-2-phénylpyrazolo[1,5-a]-pyrimidine,
4,5,6,7-tétrahydro-2-méthyl-5,7-di-n-propylpyrazolo-[1,5-a]-pyrimidine-3-carbonitrile,
4,5,6,7-tétrahydro-5-méthyl-7-[3-(trifluorométhyl)-phényl]-pyrazolo[1,5-a]pyrimidine-3-carbonitrile,
7-[4-(chloro)-phényl]-4,5,6,7-tétrahydro-5-méthyl-pyrazolo[1,5-a]pyrimidine-3-carbonitrile,
7-[3-(chloro)-phényl]-4,5,6,7-tétrahydro-5-méthyl-pyrazolo[1,5-a]pyrimidine-3-carbonitrile.

2. Composé suivant la revendication 1, **caractérisé en ce que**
R¹ et R² représentent, indépendamment l'un de l'autre, H, O-R⁹, S-R¹⁰, un reste méthyle non substitué ou portant un ou plusieurs substituants identiques ou différents, éthyle, n-propyle, 2-propyle, n-butyle, tertiobutyle ou n-hexyle, les substituants étant choisis dans le groupe comprenant F, Cl, Br, I, -CN, -NC, -NH₂, -NO₂, -SH et -OH ; un reste aryle' ou -CH₂-aryle', les substituants R²⁹, R³⁰ et R³¹ de aryle' étant, indépendamment les uns des autres, H, méthyle, éthyle, 2-propyle, n-butyle, tertiobutyle, n-hexyle, F, Cl, Br, I, OH, O-méthyle, O-éthyle,
l'un des restes R¹ et R² représentant H et l'autre reste de R¹ et R² ne représentant pas H ou bien, au cas où l'un des restes R¹ et R² désigne un reste aryle', l'autre reste de R¹ et R² représente H ou un reste méthyle, éthyle, n-propyle, 2-propyle, n-butyle, tertiobutyle ou n-hexyle ;
R³ et R⁴ représentent H, un reste méthyle ou aryle¹, les substituants R²⁹, R³⁰ et R³¹ du reste aryle¹ étant, indépendamment les uns des autres, H, un reste méthyle ou O-méthyle, l'un au moins des restes R³ et R⁴ représentant H,
ou bien
l'un des restes R¹ et R² forme un groupe W conjointement avec l'un des restes R³ et R⁴,
W étant un groupe α'-CH=CH-CH₂-β', α'-CH=CH-CH₂-CH₂-β', α'-O-(CH₂)ₘ-β' avec m = 2, 3, 4 ou 5, 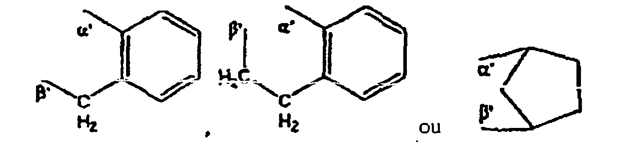 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² et l'autre reste de R³ et R⁴ représentant chacun H ;
R⁵ est un reste méthyle, éthyle, n-propyle, 2-propyle, n-butyle, isobutyle, sec.-butyle, tertiobutyle, - (CH₂)₄-OH, cyclopropyle, le reste cyclopropyle étant non substitué ou substitué une fois avec C(=O)OH, C(=O)O-méthyle ou C(=O)O-éthyle, un reste cyclopentyle, cyclohexyle, aryle¹ ou -(CH₂)ₖ₋aryle¹, les substituants R²⁹, R³⁰ et R³¹ du reste aryle¹ représentant, indépendamment les uns des autres, H, -OH, -O-méthyle, O-C₆H₅, CH₃, CF₃ ou C(=O)OH et k ayant la valeur 1 ou 2, un reste hétérocyclyle ou C(=O)R¹¹ ;
R⁶ représente H, un groupe -CN, le brome, un groupe -C(=O)R¹⁷ ou -N=N-phényle ;
R⁷ représente H, un reste aryle¹ pour lequel R²⁹, R³⁰ et R³¹ représentent H, OH, S(O)_{q}R¹⁹, q ayant la valeur de 0 ou 2, ou méthyle, éthyle, n-propyle, 2-propyle, n-butyle, isobutyle, sec.-butyle ou tertiobutyle,
R⁹ est un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclo-propyle, cyclopentyle ou cyclohexyle ou un groupe -[(CH₂)ᵣ-O]ₛ-H avec r = 1, 2 ou 3, et s = 1 ou 2 ;
R¹⁰ est un reste aryle¹ ;
R¹¹ est un reste aryle¹ pour lequel R²⁹, R³⁰ et R³¹ représentent H ou OR²⁵ ;
R¹⁷ représente OR²⁶ ;
R¹⁹ est un reste méthyle ou un reste aryle¹, l'un des substituants R²⁹, R³⁰ et R³¹ du reste aryle¹ représentant H ou -NO₂ et les deux autres des substituants R²⁹, R³⁰ et R³¹ de aryle¹ représentant H ;
R²⁵ représente H, un reste méthyle ou éthyle, R²⁵ n'étant pas H lorsque R¹ est un reste aryle en même temps que R² est un reste alkyle ;
R²⁶ représente H, un reste méthyle ou éthyle ; et
hétérocyclyle représente un groupe furanne-2-yle, furanne-3-yle, thiène-2-yle, thiène-3-yle, pyridine-2-yle, pyridine-3-yle ou pyridine-4-yle, chacun des groupes furannyle, thiényle et pyridinyle étant non substitué ou substitué une ou plusieurs fois identiques ou différentes avec un reste -NO₂, -CH₃ ou C(=O)OH.

3. Composé suivant la revendication 1 ou 2, **caractérisé en ce que**
R¹ et R² représentent, indépendamment l'un de l'autre, H, un groupe O-CH₂-CH₂-OH, un reste O-cyclohexyle, S-phényle, méthyle, phényle, 3-fluorophényle, 3-bromophényle, 4-bromophényle, 4-chlorophényle, 4-fluorophényle, 3-méthylphényle, 4-hydroxy-phényle, 4-méthoxyphényle, 2,4-diméthylphényle, 3,4-diméthoxyphényle, 2,3,4-triméthoxyphényle, 2-naphtyle ou -CH₂-phényle,
R³ et R⁴ représentent H, un reste méthyle ou 4-méthoxy-phényle, l'un au moins des restes R³ et R⁴ représentant H,
ou bien
l'un des restes R¹ et R² forme un groupe w conjointement avec l'un des restes R³ et R⁴, W étant un groupe α'-CH=CH-CH₂-β', α'-CH=CH-CH₂-CH₂-β', α'-O-(CH₂)ₘ-β' avec m = 2, 3, 4 ou 5, 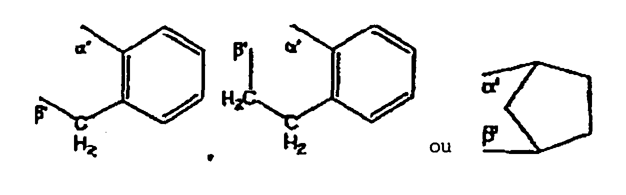 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² et l'autre reste de R³ et R⁴ désignant chacun H ;
R⁵ est un reste n-propyle, n-butyle, tertiobutyle, -(CH₂)₄-OH, cyclopropyle, ester éthylique d'acide cycloprop-2-yl-1-carboxylique, cyclohexyle, 4-trifluorophényle, 4-phénoxyphényle, 2-hydroxy-3-méthoxyphényle, 4-hydroxy-3-méthoxyphényle, 3-carboxy-2-hydroxyphényle, -(CH₂)₂-phényle, 5-carboxyfuranne-2-yle, 5-méthylfuranne-2-yle, 5-nitrofuranne-2-yle, 5-nitrothiène-2-yle, pyridine-2-yle, pyridine-3-yle, C(=O)phényle, C(=O)OH ou C(=O)O-éthyle, R⁵ ne désignant pas un groupe C(=O)OH lorsque R¹ est un reste aryle en même temps que R² est un reste alkyle ;
R⁶ représente H, -CN, le brome, un groupe -C(=O)OH, -C(=O)O-éthyle ou -N=N-phényle ; et
R⁷ représente H, un reste phényle, OH, un reste -S-méthyle, -SO₂-(4-nitrophényle) ou tertio-butyle.

4. Composé suivant la revendication 1, **caractérisé en ce qu'**il est choisi entre les composés suivant :
• 3-bromo-5-(5-nitrofuranne-2-yl)-7-m-tolyl-tétrahydropyrazolo[1,5-a]pyrimidine
• 3-bromo-7-(4-fluorophényl)-7-méthyl-5-(5-nitro-furanne-2-yl)-tétrahydropyrazolo[1,5-a]pyrimidine
• 3-bromo-7-naphtalène-2-yl-5-(5-nitrofuranne-2-yl)-tétrahydropyrazolo[1,5-a]pyrimidine
• ester éthylique d'acide 2-(3-bromo-7-m-tolyl-tétrahydropyrazolo[1,5-a]pyrimidine-5-yl)-cyclopropane-carboxylique
• ester éthylique d'acide 2-[3-bromo-7-(4-bromo-phényl)-tétrahydropyrazolo[1,5-a]pyrimidine-5-yl]-cyclopropanecarboxylique
• ester éthylique d'acide 2-(3-bromo-7-naphtalène-2-yl-tétrahydropyrazolo[1,5-a]pyrimidine-5-yl)-cyclo-propanecarboxylique
• 3-bromo-7-(4-fluorophényl)-7-méthyl-5-(5-méthyl-furanne-2-yl)-tétrahydropyrazolo[1,5-a]pyrimidine
• ester éthylique d'acide 3-bromo-7-(3,4-diméthoxy-phényl)-tétrahydropyrazolo[1,5-a]pyrimidine-5-carboxylique
• ester éthylique d'acide 3-bromo-7-(4-méthoxy-phényl)-tétrahydropyrazolo[1,5-a]pyrimidine-5-carboxylique
• acide 3-bromo-7-(4-méthoxyphényl)-tétrahydro-pyrazolo[1,5-a]pyrimidine-5-carboxylique
• 3-bromo-7-(2,4-diméthylphényl)-5-(5-nitro-furanne-2-yl)-tétrahydropyrazolo[1,5-a]pyrimidine
• 3-bromo-7-(4-méthoxyphényl)-5-(5-nitrofuranne-2-yl)-tétrahydropyrazolo[1,5-a]pyrimidine
• ester diéthylique d'acide 5,5a,6,8a-tétrahydro-3H-1,4,8b-triaza-as-indacène-3,5-dicarboxylique ; ester diéthylique d'acide 5,5a,6,8a-tétrahydro-4H-1,4,8b-triaza-as-indacène-3,5-dicarboxylique
• ester éthylique d'acide 2-hydroxy-3-phénylazo-5,5a,6,8a-tétrahydro-3H-1,4,8b-triaza-as-indacène-5-carboxyliquc ; ester éthylique d'acide 2-hydroxy-3-phényl-azo-5,5a,6,8a-tétrahydro-4H-1,4,8b-triaza-as-indacène-5-carboxylique
• ester éthylique d'acide 2-tertiobutyl-5,5a,6,8a-tétrahydro-3H-1,4,8b-triaza-as-indacène-5-carboxylique ; ester éthylique d'acide 2-tertiobutyl-5,5a-6,8a-tétrahydro-3H-1,4,8b-triaza-as-indacène-5-carboxylique
• ester éthylique d'acide 3-bromo-2-phényl-5,5a,6,8a-tétrahydro-3H-1,4,8b-triaza-as-indacène-5-carboxylique ; ester éthylique d'acide 3-bromo-2-phényl-5,5a,6,8a-tétrahydro-4H-1,4,8b-triaza-as-indacène-5-carboxylique
• ester diéthylique d'acide 7-(2,3,4-triméthoxy-phényl)-tétrahydropyrazolo[1,5-a]pyrimidine-3,5-dicarboxy-lique
• ester éthylique d'acide 3-cyano-2-méthylsulfanyl-7-(2,3,4-triméthoxyphényl)-tétrahydropyrazolo[1,5-a]-pyrimidine-5-carboxylique
• ester éthylique d'acide 2-hydroxy-7-(4-hydroxy-phényl)-6-méthyl-3-phénylazotétrahydropyrazolo[1,5-a]-pyrimidine-5-carboxylique
• ester éthylique d'acide 3-bromo-7-(4-hydroxyphényl)-6-méthyl-2-phényltétrahydropyrazolo[1,5-a]-pyrimidine-5-carboxylique
• ester diéthylique d'acide 5,5a,6,10b-tétrahydro-3H-1,4,10c-triazacyclopenta[c]fluorène-3,5-dicarboxylique ; ester diéthylique d'acide 5,5a,6,10b-tétrahydro-4H-1,4,10c-triazacyclopenta[c]fluorène-3,5-dicarboxylique
• ester éthylique d'acide 2-hydroxy-3-phénylazo-5,5a,6,10b-tétrahydro-3H-1,4,10c-triazacyclopenta[c]-fluorène-5-carboxylique ; ester éthylique d'acide 2-hydroxy-3-phénylazo-5,5a,6,10b-tétrahydro-4H-1,4,10c-triazacyclopenta[c]fluorène-5-carboxylique
• ester diéthylique d'acide 7-phénylsulfanyl-tétrahydropyrazolo[1,5-a]pyrimidine-3,5-dicarboxylique
• ester éthylique d'acide 3-cyano-2-méthylsulfanyl-7-phénylsulfanyltétrahydropyrazolo[1,5-a]pyrimidine-5-carboxylique
• acide 3-cyano-2-méthylsulfanyl-7-(2,3,4-trimétho-xyphényl)-tétrahydropyrazolo[1,5-a]pyrimidine-5-carboxy-lique
• ester 3-éthylique d'acide 7-phénylsulfanyl-tétrahydropyrazolo[1,5-a]pyrimidine-3,5-dicarboxylique
• ester éthylique d'acide 3-cyano-7-(2,4-diméthyl-phényl)-2-méthylsulfanyltétrahydropyrazolo[1,5-a]-pyrimidine-5-carboxylique
• ester éthylique d'acide 3-cyano-7-(2,4-diméthyl-phényl)-tétrahydropyrazolo[1,5-a]pyrimidine-5-carboxylique
• ester 3-éthylique d'acide 7-(2,4-diméthylphényl)-tétrahydropyrazolo[1,5-a]pyrimidine-3,5-dicarboxylique
• acide 3-bromo-7-(2,4-diméthylphényl)-2-phényl-tétrahydropyrazolo[1,5-a]pyrimidine-5-carboxylique
• acide 3-cyano-7-(2,4-diméthylphényl)-2-méthyl-sulfanyltétrahydropyrazolo[1,5-a]pyrimidine-5-carboxylique
• acide 3-cyano-7-(2,4-diméthylphényl)-tétrahydro-pyrazolo[1,5-a]pyrimidine-5-carboxylique
• acide 3-cyano-7-(3,4-diméthoxyphényl)-2-méthyl-sulfanyltétrahydropyrazolo[1,5-a]pyrimidine-5-carboxylique
• ester éthylique d'acide 7-(2,4-diméthylphényl)-5-(5-nitrofuranne-2-yl)-tétrahydropyrazolo[1,5-a]pyrimidine-3-carboxylique
• 7-(2,4-diméthylphényl)-5-(5-nitrofuranne-2-yl)-3-phénylazotétrahydropyrazolo[1,5-a]-pyrimidine-2-ol
• 3-bromo-7-(2,4-diméthylphényl)-5-(5-nitrofuranne-2-yl)-2-phényltétrahydropyrazolo[1,5-a]pyrimidine
• 7-(2,4-diméthylphényl)-2-méthylsulfanyl-5-(5-nitrofuranne-2-yl)-tétrahydropyrazolo[1,5-a]pyrimidine-3-carbonitrile
• 7-(2,4-diméthylphényl)-5-(5-nitrofuranne-2-yl)-tétrahydropyrazolo[1,5-a]pyrimidine-3-carbonitrile
• 3-bromo-7-(3,4-diméthoxyphényl)-5-(5-nitro-furanne-2-yl)-2-phényltétrahydropyrazolo[1,5-a]pyrimidine
• 7-(4-méthoxyphényl)-2-méthylsulfanyl-5-(5-nitro-furanne-2-yl)-tétrahydropyrazolo[1,5-a]pyrimidine-3-carbonitrile
• ester éthylique d'acide 7-(2,4-diméthylphényl)-5-(2-éthoxycarbonylcyclopropyl)-tétrahydropyrazolo[1,5-a]-pyrimidine-3-carboxylique
• ester éthylique d'acide 2-[7-(2,4-diméthyl-phényl)-2-hydroxy-3-phénylazotétrahydropyrazolo[1,5-a]-pyrimidine-5-yl]-cyclopropanecarboxylique
• ester éthylique d'acide 2-[2-tertiobutyl-7-(2,4-diméthylphényl)-tétrahydropyrazolo[1,5-a]pyrimidine-5-yl]-cyclopropanecarboxylique
• ester éthylique d'acide 2-[3-bromo-7-(2,4-diméthylphényl)-2-phényltétrahydropyrazolo[1,5-a]-pyrimidine-5-yl]-cyclopropanecarboxylique
• ester éthylique d'acide 2-[3-cyano-7-(2,4-diméthylphényl)-2-méthylsulfanyltétrahydropyrazolo[1,5-a]-pyrimidine-5-yl]-cyclopropanecarboxylique
• ester éthylique d'acide 5-(2-éthoxycarbonyl-cyclopropyl)-7-(3-fluorophényl)-tétrahydropyrazolo[1,5-a]-pyrimidine-3-carboxylique
• ester éthylique d'acide 2-[3-bromo-7-(3-bromo-phényl)-2-phényltétrahydropyrazolo[1,5-a]pyrimidine-5-yl]-cyclopropanecarboxylique
• ester éthylique d'acide 2-[7-(3-bromophényl)-3-cyano-2-méthylsulfanyltétrahydropyrazolo[1,5-a]pyrimidine-5-yl]-cyclopropanecarboxylique
• 7-(2,4-diméthylphényl)-5-(5-nitrothiophène-2-yl)-3-phénylazotétrahydropyrazolo[1,5-a]pyrimidine-2-ol
• 7-(2,4-diméthylphényl)-2-méthylsulfanyl-5-(5-nitrothiophène-2-yl)-tétrahydropyrazolo[1,5-a]pyrimidine-3-carbonitrile
• 7-(2,4-diméthylphényl)-5-(5-nitrothiophène-2-yl)-tétrahydropyrazolo[1,5-a]pyrimidine-3-carbonitrile
• 7-(3,4-diméthoxyphényl)-5-(5-nitrothiophène-2-yl)-tétrahydropyrazolo[1,5-a]pyrimidine-3-carboxylique
• 7-(3,4-diméthoxyphényl)-5-(5-nitrothiophène-2-yl)-3-phénylazotétrahydropyrazolo[1,5-a]pyrimidine-2-ol
• 3-bromo-7-(3,4-diméthoxyphényl)-5-(5-nitro-thiophène-2-yl)-2-phényltétrahydropyrazolo[1,5-a]-pyri-midine
• 7-(3,4-diméthoxyphényl)-2-méthylsulfanyl-5-(5-nitrothiophène-2-yl)-tétrahydropyrazolo[1,5-a]pyrimidine-3-carbonitrile
• 7-(3,4-diméthoxyphényl)-5-(5-nitrothiophène-2-yl)-tétrahydropyrazolo[1,5-a]pyrimidine-3-carbonitrile
• ester éthylique d'acide 7-(4-méthoxyphényl)-5-(5-nitrothiophène-2-yl)-tétrahydropyrazolo[1,5-a]pyrimidine-3-carboxylique
• acide 5-[3-bromo-7-(4-méthoxyphényl)-2-phényl-tétrahydropyrazolo[1,5-a]pyrimidine-5-yl]-furanne-2-carboxylique
• ester éthylique d'acide 5-benzoyl-7-(2,4-diméthylphényl)-tétrahydropyrazolo[1,5-a]pyrimidine-3-carboxylique
• 5-benzoyl-7-(2,4-diméthylphényl)-2-méthyl-sulfanyltétrahydropyrazolo[1,5-a]pyrimidine-3-carbonitrile
• 5-benzoyl-7-(2,4-diméthylphényl)-tétrahydro-pyrazolo[1,5-a]pyrimidine-3-carbonitrile
• ester éthylique d'acide 5-benzoyl-7-(3,4-diméthoxyphényl)-tétrahydropyrazolo[1,5-a]pyrimidine-3-carboxylique
• [3-bromo-7-(3,4-diméthoxyphényl)-2-phényl-tétrahydropyrazolo[1,5-a]pyrimidine-5-yl]-phénylméthanone
• 5-benzoyl-7-(3,4-diméthoxyphényl)-2-méthyl-sulfanyltétrahydropyrazolo[1,5-a]pyrimidine-3-carbonitrile
• 5-benzoyl-7-(3,4-diméthoxyphényl)-tétrahydro-pyrazolo[1,5-a]pyrimidine-3-carbonitrile
• ester éthylique d'acide 5-benzoyl-7-(4-méthoxyphényl)-tétrahydropyrazolo[1,5-a]pyrimidine-3-carboxylique
• 5-benzoyl-7-(4-méthoxyphényl)-2-méthylsulfanyl-tétrahydropyrazolo[1,5-a]pyrimidine-3-carbonitrile
• 5-benzoyl-7-(4-méthoxyphényl)-tétrahydro-pyrazolo[1,5-a]pyrimidine-3-carbonitrile
• ester éthylique d'acide 5-benzoyl-7-(3-fluoro-phényl)-tétrahydropyrazolo[1,5-a]pyrimidine-3-carboxylique
• [3-bromo-7-(3-fluorophényl)-2-phényltétrahydro-pyrazolo[1,5-a]pyrimidine-5-yl]-phénylméthanone
• [3-bromo-7-(3-bromophényl)-2-phényltétrahydro-pyrazolo[1,5-a]pyrimidine-5-yl]-phénylméthanone
• ester éthylique d'acide 7-(2,4-diméthylphényl)-5-(4-phénoxyphényl)-tétrahydropyrazolo[1,5-a]pyrimidine-3-carboxylique
• 3-bromo-7-(2,4-diméthylphényl)-5-(4-phénoxy-phényl)-2-phényltétrahydropyrazolo[1,5-a]pyrimidine
• 7-(2,4-diméthylphényl)-2-méthylsulfanyl-5-(4-phénoxyphényl)-tétrahydropyrazolo[1,5-a]pyrimidine-3-carbonitrile
• 7-(2,4-diméthylphényl)-5-(4-phénoxyphényl)-tétrahydropyrazolo[1,5-a]pyrimidine-3-carbonitrile
• ester éthylique d'acide 7-(3,4-diméthoxyphényl)-5-(4-phénoxyphényl)-tétrahydropyrazolo[1,5-a]pyrimidine-3-carboxylique
• 7-(3,4-diméthoxyphényl)-2-méthylsulfanyl-5-(4-phénoxyphényl)-tétrahydropyrazolo[1,5-a]pyrimidine-3-carbonitrile
• acide 3-[3-cyano-7-(4-hydroxyphényl)-6-méthyl-tétrahydropyrazolo[1,5-a]pyrimidine-5-yl]-2-hydroxy-benzoïque
• acide 3-(3-cyano-5,5a,6,10b-tétrahydro-3H-1,4,10c-triazacyclopenta[c]fluorène-5-yl)-2-hydroxy-benzoïque ; acide 3-(3-cyano-5,5a,6,10b-tétrahydro-4H-1,4,10c-triazacyclopenta[c]fluorène-5-yl)-2-hydroxy-benzoïque
• acide 3-(3-cyano-7-phénylsulfanyltétrahydro-pyrazolo[1,5-a]pyrimidine-5-yl)-2-hydroxybenzoique
• acide 3-[2-tertiobutyl-7-(4-chlorophényl)-7-méthyltétrahydropyrazolo[1,5-a]pyrimidine-5-yl]-2-hydroxy-benzoïque
• ester éthylique d'acide 5-(4-hydroxy-3-méthoxy-phényl)-7-(4-hydroxyphényl)-6-méthyltétrahydropyrazolo[1,5-a]pyrimidine-3-carboxylique
• 5-(4-hydroxy-3-méthoxyphényl)-7-(4-hydroxy-phényl)-6-méthyltétrahydropyrazolo[1,5-a]pyrimidine-3-carbonitrile
• ester éthylique d'acide 5-(4-hydroxy-3-méthoxy-phényl)-5,5a,6,10b-tétrahydro-3H-1,4,10c-triazacyclopenta-[c]fluorène-3-carboxylique ; ester éthylique d'acide 5-(4-hydroxy-3-méthoxyphényl)-5,5a,6,10b-tétrahydro-4H-1,4,10c-triazacyclopenta[c]fluorène-3-carboxylique
• 4-(2-tertiobutyl-5,5a,6,10b-tétrahydro-3H-1,4,10c-triazacyclopenta[c]fluorène-5-yl)-2-méthoxyphénol ; 4-(2-tertiobutyl-5,5a,6,10b-tétrahydro-4H-1,4,10c-triaza-cyclopenta[c]fluorène-5-yl)-2-méthoxyphénol
• 5-(4-hydroxy-3-méthoxyphényl)-2-méthylsulfanyl-5,5a,6,10b-tétrahydro-3H-1,4,10c-triazacyclopenta[c]-fluorène-3-carbonitrile ; 5-(4-hydroxy-3-méthoxyphényl)-2-méthylsulfanyl-5,5a,6,10b-tétrahydro-3H-1,4,10c-triaza-cyclopenta[c]fluorène-3-carbonitrile
• ester éthylique d'acide 5-(4-hydroxy-3-méthoxy-phényl)-7-phénylsulfanyltétrahydropyrazolo[1,5-a]-pyrimidine-3-carboxylique
• 4-(2-tertiobutyl-7-phénylsulfanyltétrahydro-pyrazolo[1,5-a]pyrimidine-5-yl)-2-méthoxyphénol
• 4-(3-bromo-2-phényl-7-phénylsulfanyltétrahydro-pyrazolo[1,5-a]pyrimidine-5-yl)-2-méthoxyphénol
• ester éthylique d'acide 5-(2-hydroxy-3-méthoxy-phényl)-7-phénylsulfanyltétrahydropyrazolo[1,5-a]-pyrimidine-3-carboxylique
• ester éthylique d'acide 7-(4-chlorophényl)-5-(2-hydroxy-3-méthoxyphényl)-7-méthyltétrahydropyrazolo[1,5-a]-pyrimidine-3-carboxylique
• 5-(4-hydroxybutyl)-5,5a,6,10b-tétrahydro-3H-1,4,10c-triazacyclopenta[c]fluorène-3-carbonitrile ; 5-(4-hydroxybutyl)-5,5a,6,10b-tétrahydro-4H-1,4,10c-triaza-cyclopenta[c]fluorène-3-carbonitrile
• 5-(4-hydroxybutyl)-2-méthylsulfanyl-7-phényl-sulfanyltétrahydropyrazolo[1,5-a]pyrimidine-3-carbonitrile
• 5-(4-hydroxybutyl)-7-phénylsulfanyltétrahydro-pyrazolo[1,5-a]pyrimidine-3-carbonitrile
• 7-(4-chlorophényl)-5-(4-hydroxybutyl)-7-méthyl-tétrahydropyrazolo[1,5-a]pyrimidine-3-carbonitrile
• 5-butyl-2-méthylsulfanyl-5,5a,6,10b-tétrahydro-3H-1,4,10c-triazacyclopenta[c]fluorène-3-carbonitrile ; 5-butyl-2-méthylsulfanyl-5,5a,6,10b-tétrahydro-4H-1,4,10c-triazacyclopenta[c]fluorène-3-carbonitrile
• 5-butyl-2-méthylsulfanyl-7-phénylsulfanyl-tétrahydropyrazolo[1,5-a]pyrimidine-3-carbonitrile
• 5-butyl-7-phénylsulfanyltétrahydropyrazolo[1,5-a]-pyrimidine-3-carbonitrile
• 5-butyl-7-(4-chlorophényl)-7-méthyl-2-méthyl-sulfanyltétrahydropyrazolo[1,5-a]pyrimidine-3-carbonitrile
• 5-cyclopropyl-7-(2,4-diméthylphényl)-3-phénylazo-tétrahydropyrazolo[1,5-a]pyrimidine-2-ol
• 2-tertiobutyl-5-cyclopropyl-7-(2,4-diméthyl-phényl)-tétrahydropyrazolo[1,5-a]pyrimidine
• 5-cyclopropyl-7-(2,4-diméthylphényl)-2-méthyl-sulfanyltétrahydropyrazolo[1,5-a]pyrimidine-3-carbonitrile
• 2-tertiobutyl-5-cyclopropyl-7-(3,4-diméthoxy-phényl)-tétrahydropyrazolo[1,5-a]pyrimidine
• 3-bromo-5-cyclopropyl-7-(3,4-diméthoxyphényl)-2-phényltétrahydropyrazolo[1,5-a]pyrimidine
• ester éthylique d'acide 5-cyclopropyl-7-(4-méthoxyphényl)-tétrahydropyrazolo[1,5-a]pyrimidine-3-carboxylique
• 5-cyclopropyl-3,5,5a,6,7,11b-hexahydro-1,4,11c-triazacyclopenta[c]phénanthrène-3-carbonitrile
• ester éthylique d'acide 7-(2,4-diméthylphényl)-5-pyridine-2-yl-tétrahydropyrazolo[1,5-a]-pyrimidine-3-carboxylique
• 7-(2,4-diméthylphényl)-3-phénylazo-5-pyridine-2-yl-tétrahydropyrazolo[1,5-a]pyrimidine-2-ol
• 3-bromo-7-(2,4-diméthylphényl)-2-phényl-5-pyridine-2-yl-tétrahydropyrazolo[1,5-a]pyrimidine
• 7-(2,4-diméthylphényl)-2-méthylsulfanyl-5-pyri-dine-2-yl-tétrahydropyrazolo[1,5-a]pyrimidine-3-carbo-nitrile
• 7-(3,4-diméthoxyphényl)-2-méthylsulfanyl-5-phénéthyltétrahydropyrazolo[1,5-a]pyrimidine-3-carbonitrile
• 7-(3,4-diméthoxyphényl)-5-phénéthyltétrahydro-pyrazolo[1,5-a]pyrimidine-3-carbonitrile
• ester éthylique d'acide 5-cyclopropyl-7-(2-hydroxyéthoxy)-tétrahydropyrazolo[1,5-a]pyrimidine-3-carboxylique
• 2-(2-tertiobutyl-5-cyclopropyltétrahydropyrazolo-[1,5-a]pyrimidine-7-yloxy)-éthanol
• ester éthylique d'acide 5-cyclopropyl-3,5,5a,6,7,8a-hexahydro-8-oxa-1,4,8b-triaza-as-indacène-3-carboxylique ; ester éthylique d'acide 5-cyclopropyl-4,5,5a,6,7,8a-hexahydro-8-oxa-1,4,8b-triaza-as-indacène-3-carboxylique
• 5-cyclopropyl-3-phénylazo-3,5,5a,6,7,8a-hexahydro-8-oxa-1,4,8b-triaza-as-indacène-2-ol ; 5-cyclo-propyl-3-phénylazo-4,5,5a,6,7,8a-hexahydro-8-oxa-1,4,8b-triaza-as-indacène-2-ol
• ester éthylique d'acide 7-cyclohexyloxy-5-cyclo-propyltétrahydropyrazolo[1,5-a]pyrimidine-3-carboxylique
• 7-cyclohexyloxy-5-cyclopropyl-2-méthylsulfanyl-tétrahydropyrazolo[l,5-a]pyrimidine-3-carbonitrile
• 7-(4-chlorophényl)-5-cyclohexyltétrahydro-pyrazolo[1,5-a]pyrimidine-3-carbonitrile
• ester éthylique d'acide 5-cyclohexyl-7-(2-hydroxyéthoxy)-tétrahydropyrazolo[1,5-a]pyrimidine-3-carboxylique
• ester éthylique d'acide 5-cyclohexyl-3,5,5a,6,7,8a-hexahydro-8-oxa-1,4,8b-triaza-as-indacène-3-carboxylique ; ester éthylique d'acide 5-cyclohexyl-4,5,5a,6,7,8a-hexahydro-8-oxa-1,4,8b-triaza-as-indacène-3-carboxylique
• ester éthylique d'acide 5-cyclohexyl-7-cyclo-hexyloxytétrahydropyrazolo[1,5-a]pyrimidine-3-carboxylique
• 7-(2,4-diméthylphényl)-3-phénylazo-5-propyl-tétrahydropyrazolo[1,5-a]pyrimidine-2-ol
• 7-(2,4-diméthylphényl)-2-méthylsulfanyl-5-propyl-tétrahydropyrazolo[1,5-a]pyrimidine-3-carbonitrile
• ester éthylique d'acide 5-tertiobutyl-7-(2,4-diméthylphényl)-tétrahydropyrazolo[1,5-a]pyrimidine-3-carboxylique
• 2,5-di-tertiobutyl-7-(3,4-diméthoxyphényl)-tétrahydropyrazolo[1,5-a]pyrimidine
• 3-bromo-5-tertiobutyl-7-(3,4-diméthoxyphényl)-2-phényltétrahydropyrazolo[1,5-a]pyrimidine
• ester éthylique d'acide 2-[3-cyano-6,7-bis-(4-méthoxyphényl)-tétrahydropyrazolo[1,5-a]pyrimidine-5-yl]-cyclopropanecarboxylique
• acide 3-cyano-6,7-bis-(4-méthoxyphényl)-tétra-hydropyrazolo[1,5-a]pyrimidine-5-carboxylique
• 4-[3-bromo-6-méthyl-2-phényl-5-(4-trifluoro-méthylphényl)-tétrahydropyrazolo[1,5-a]pyrimidine-7-yl]-phénol
• 7-(4-hydroxyphényl)-6-méthyl-2-méthylsulfanyl-5-(4-trifluorométhylphényl)-tétrahydropyrazolo[1,5-a]-pyrimidine-3-carbonitrile
• 7-(4-hydroxyphényl)-6-méthyl-5-(4-trifluoro-méthylphényl)-tétrahydropyrazolo[1,5-a]pyrimidine-3-carbonitrile
• 7-phényl-3-phénylazo-5-pyridine-2-yl-3,5,6,7-tétrahydropyrazolo[1,5-a]pyrimidine-2-ol
• ester éthylique d'acide 7-phénylsulfanyl-5-pyridine-2-yl-3,5,6,7-tétrahydropyrazolo[1,5-a]pyrimidine-3-carboxylique
• 3-phénylazo-7-phénylsulfanyl-5-pyridine-2-yl-3,5,6,7-tétrahydropyrazolo[1,5-a]pyrimidine-2-ol
• 3-bromo-7-phénylsulfanyl-5-pyridine-2-yl-3,5,6,7-tétrahydropyrazolo[15-a]pyrimidine
• 7-phénylsulfanyl-5-pyridine-2-yl-3,5,6,7-tétra-hydropyrazolo[1,5-a]pyrimidine-3-carbonitrile
• 7-(3,4-diméthoxyphényl)-2-méthylsulfanyl-5-pyridine-2-yl-tétrahydropyrazolo[1,5-a]pyrimidine-3-carbonitrile
• 3-bromo-7-(3,4-diméthoxyphényl)-2-phényl-5-pyridine-2-yl-tétrahydropyrazolo[1,5-a]pyrimidine
• ester éthylique d'acide 3-bromo-7-(3,4-diméthoxy-phényl)-2-phényltétrahydropyrazolo[1,5-a]pyrimidine-5-carboxylique
• 3-bromo-7-(3,4-diméthoxyphényl)-5-(5-nitro-furanne-2-yl)-2-phényltétrahydropyrazolo[1,5-a]pyrimidine
• ester éthylique d'acide 3-cyano-7-(3,4-diméthoxy-phényl)-2-méthylsulfanyltétrahydropyrazolo[1,5-a]-pyrimi-dine-5-carboxylique
• ester éthylique d'acide 3-cyano-7-(3,4-diméthoxy-phényl)-tétrahydropyrazolo[1,5-a]pyrimidine-5-carboxylique
• 3-bromo-7-(3,4-diméthoxyphényl)-2-phényl-5-pyridine-2-yl-4,5,6,7-tétrahydropyrazolo[1,5-a]pyrimidine
• 7-(3,4-diméthoxyphényl)-5-pyridine-2-yl-4,5,6,7-tétrahydropyrazolo[1,5-a]pyrimidine-3-carbonitrile
• 7-(3,4-diméthoxyphényl)-2-méthylsulfanyl-5-(5-nitrofuranne-2-yl)-tétrahydropyrazolo[1,5-a]pyrimidine-3-carbonitrile
• ester éthylique d'acide 7-(3,4-diméthoxyphényl)-5-pyridine-2-yl-4,5,6,7-tétrahydropyrazolo[1,5-a]-pyrimi-dine-3-carboxylique.

5. Procédé de production de composés de formules générales (IA) et/ou (IB) ainsi que de leurs sels acceptables du point de vue pharmaceutique 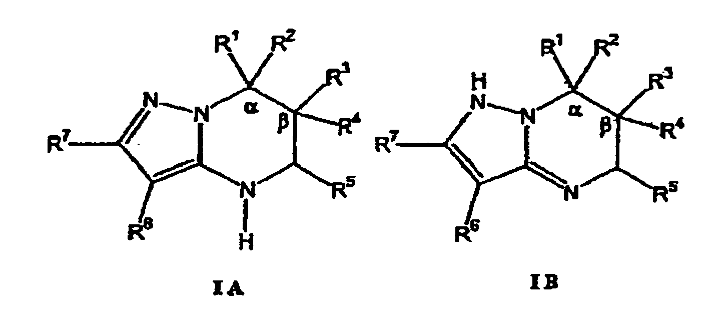 formules dans lesquelles
R¹, R², R³, R⁴, R⁵, R⁶ et R⁷ ont les définitions indiquées dans la revendication 1, **caractérisé en ce qu'**on fait réagir en présence d'un acide une pyrazolamine de formule générale (IIIA) ou (IIIB) 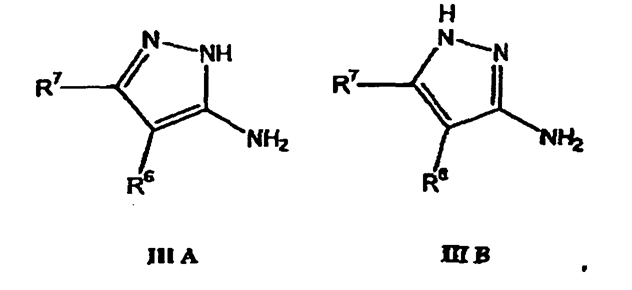 dans laquelle,
R⁶ et R⁷ sont tels que définis ci-dessus dans la présente revendication,
avec un aldéhyde de formule générale (IV) 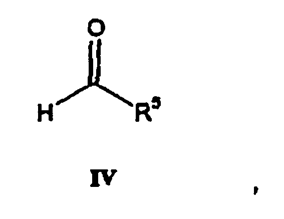 dans laquelle,
R⁵ est tel que défini ci-dessus dans la présente revendication,
et une oléfine de formule générale (V) 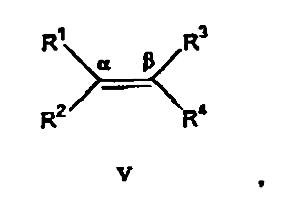 dans laquelle
R¹, R², R³ et R⁴ sont tels que définis ci-dessus dans la présente revendication, sous réserve que lorsque l'un des restes R¹ et R² forme un groupe W conjointement avec l'un des restes R³ et R⁴, l'extrémité de W désignée par α' soit liée à l'atome de carbone α de l'oléfine de formule générale (V) et que l'extrémité de W désignée par β' soit liée à l'atome de carbone β de l'oléfine de formule générale (V).

6. Procédé suivant la revendication 5, **caractérisé en ce que** la réaction de l'hétérocyclylamine de formule générale (IIIA) ou (IIIB) avec l'aldéhyde de formule générale (IV) et avec l'oléfine de formule générale (V) est conduite selon un mode opératoire en récipient unique.

7. Procédé suivant l'une quelconque des revendications 5 ou 6, **caractérisé en ce que** l'acide est l'acide trifluoracétique.

8. Procédé suivant l'une quelconque des revendications 5 à 7, **caractérisé en ce que** la réaction est conduite dans un solvant organique à une température allant de 0 à 100°C et pendant une durée de réaction de 0,25 à 12 h.

9. Procédé suivant l'une des revendications 5 à 8, **caractérisé en ce que** la réaction est conduite à une température allant de 15 à 40°C.

10. Médicament contenant au moins un composé de formule générale (IA) ou (IB), 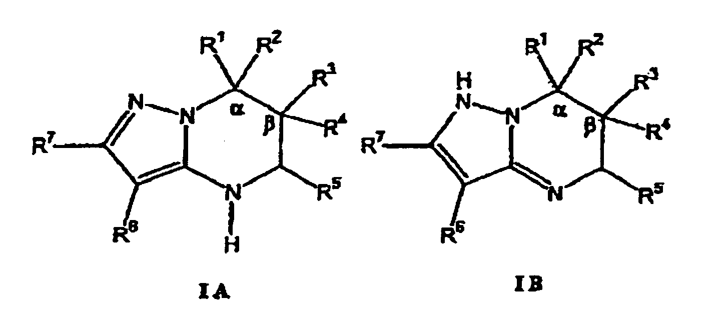 sous la forme représentée ou sous la forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou des diastéréoisomères,
ou sous forme de mélanges des stéréoisomères, en particulier des énantiomères ou des diastéréoisomères, dans un rapport de mélange quelconque ;
formule dans laquelle
R¹ et R² représentent, indépendamment l'un de l'autre, H, un groupe O-R⁹, S-R¹⁰, un reste [Alk en C₁ à C₆], aryle' ou -(alkyle en C₁ à C₆)-aryle', les substituants R²⁹, R³⁰ et R³¹ du reste aryle' étant, indépendamment les uns des autres, H, [Alk en C₁ à C₆], F, Cl, Br, I, OH, O-[Alk en C₁ à C₆], O-aryle¹ ou O-CH₂-aryle¹, l'un des restes R¹ et R² représentant H et l'autre de ces deux restes ne représentant pas H ou bien, au cas où l'un des restes R¹ et R² désigne un reste aryle', l'autre de ces deux restes représente H ou un reste alkyle en C₁ à C₆ ;
R³ et R⁴ représentent H, un reste aryle', -CH₂-aryle' ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle non substitué,
l'un au moins des restes R³ et R⁴ représentant H, ou bien l'un des restes R¹ et R² forme un groupe W conjointement avec l'un des restes R³ et R⁴,
W étant un groupe α'-CH=CH-CH₂-β', α'-CH=CH-CH₂-CH₂-β', α'-O-(CH₂)ₘ-β' où m a la valeur 2, 3, 4 ou 5, 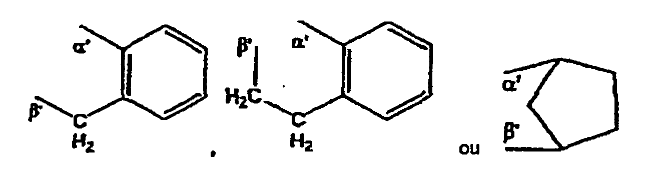 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² désigne H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre reste de R³ et R⁴ désigne H ou un reste méthyle, éthyle, n-propyle, 2-propyle, n-butyle, isobutyle, sec.-butyle, tertio-butyle, n-amyle, isoamyle, sec.-amyle, n-hexyle, isohexyle, sec.-hexyle ;
R⁵ est un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.hexyle, cyclo-propyle, cyclobutyle, cyclopentyle, cyclo-hexyle ou cycloheptyle, chacun de ces restes étant non substitué ou portant un ou plusieurs substituants identiques ou différents choisis dans le groupe comprenant F, Cl, Br, I, -CN, -NC, -NH₂, -NO₂, -SH, -OH, -CO₂H et -CO₂alkyle, aryle' ou -(CH₂)ₖ₋aryle', où k a la valeur 1, 2, 3 ou 4, hétérocyclyle ou C(=O)R¹¹ ;
R⁶ représente H, un reste méthyle, éthyle, -CN, le fluor, le chlore, le brome, l'iode, un groupe C(=O)R¹⁷ ou -N=N-aryle¹ ;
R⁷ représente H, un reste aryle¹, un groupe OR¹⁸, S(O)_{q}R¹⁹, où q a la valeur 0, 1 ou 2,
ou un reste méthyle non substitué, éthyle, n-propyle, 2-propyle, n-butyle, isobutyle, sec.-butyle, tertiobutyle, n-amyle, iso-amyle, sec.-amyle, n-hexyle, isohexyle ou sec.-hexyle ;
R⁹ désigne un reste méthyle non substitué, éthyle, n-propyle, 2-propyle, n-butyle, iso-butyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]ₛ-H dans lequel r a la valeur 1, 2, 3, 4, 5 ou 6 et s a la valeur 1, 2, 3, 4, 5 ou 6 ;
R¹⁰ est un reste aryle' ;
R¹¹ est un reste aryle' ou un groupe OR²⁵ ;
R¹⁷ est un groupe OR²⁶ ;
R¹⁸ représente H ou un groupe méthyle ;
R¹⁹ représente H, un reste aryle¹ ou un reste méthyle, éthyle, n-propyle, 2-propyle, n-butyle, isobutyle, sec.-butyle, tertio-butyle, n-amyle, isoamyle, sec.-amyle, n-hexyle, isohexyle ou sec.-hexyle chacun non substitué ;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁶ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ; et
Hétérocyclyle désigne un groupe furanne-2-yle, furanne-3-yle, thiène-2-yle, thiène-3-yle, pyridine-2-yle, pyridine-3-yle ou pyridine-4-yle, les groupes furannyle, thiényle et pyridinyle étant chacun non substitués ou portant chacun un ou plusieurs substituants identiques ou différents choisis dans le groupe comprenant F, Cl, Br, I, -CN, -NO₂, -CO₂H et -CO₂alkyle ;
Aryle' désigne un reste aryle¹, aryle² ou aryle³ ;
aryle¹ représente 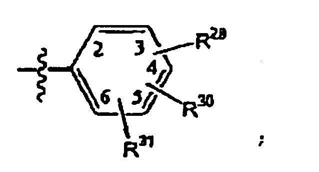
aryle² représente 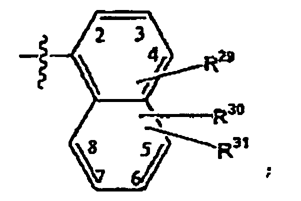
aryle³ représente 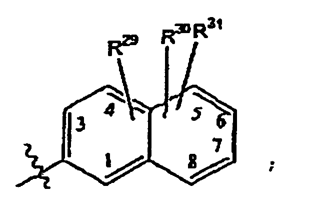
R²⁹, R³⁰ et R³¹ représentent, indépendamment les uns des autres, H, un reste [Alk en C₁ à C_{6]}, [cycloalk en C₃ à C₈], -(alkyle en C₁ à C₆)-[cycloalk en C₃ à C₈], [Ar], - (alkyle en C₁ à C₆) - [Ar], [Het], - (alkyle en C₁ à C₆) - [Het], F, Cl, Br, I, -CN, -NC, -OR³² , -SR³³, -NO, -NO₂, NH₂, NHR³⁴, NR³⁵R³⁶, -N-OH, -N-O[Alk en C₁ à C₆], -NHNH₂, -N=N-[Ar], -(C=O)R³⁷, 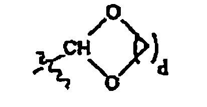 où d a la valeur 1, 2 ou 3, ou un groupe -(C=S)R³⁷, et peuvent occuper n'importe quelle position du noyau ;
R³² et R³³ représentent, indépendamment l'un de l'autre, H, un reste [Alk en C₁ à C₆], [cycloalk en C₃ à C₈], - (alkyle en C₁ à C₆) - [cycloalk en C₃ à C_{8]}, - [Ar], - (alkyle en C₁ à C₆) - [Ar], - [Het], - (alkyle en C₁ à C₆)-[Het], (C=O)R³⁸, -[(CH₂)_{w}-O]_{z}-H ou -[(CH₂)_{w}- O]_{z}-[Alk en C₁ à C₆], avec w = 1, 2, 3 ou 4 et z = 1, 2, 3, 4 ou 5 ;
R³⁴ est un reste [Alk en C₁ à C₆], -CH₂-[Ar] ou -(C=O)O-tertiobutyle ;
R³⁵ et R³⁶ représentent, indépendamment l'un de l'autre, un reste [Alk en C₁ à C₆] ou forment ensemble un reste -(CH₂)_{g}- où g a la valeur 4 ou 5 ;
R³⁷ représente H, un reste -[Alk en C₁ à C₆], - [cycloalk en C₃ à C₈], - (alkyle en C₁ à C₆)-[cycloalk en C₃ à C₈], -[Ar], - (alkyle en C₁ à C₆) - [Ar], - [Het], -(alkyle en C₁ à C₆) - [Het], -OR³⁹, -NH₂, -NHR³⁴, -NR³⁵R³⁶ ;
R³⁸ représente H, un reste -[Alk en C₁ à C₆], - [cycloalk en C₃ à C₈], - (alkyle en C₁ à C₆)-[cycloalk en C₃ à C₈], -[Ar], -(alkyle en C₁ à C₆)-[Ar] ;
et
R³⁹ représente H, un reste [Alk en C₁ à C₆], - [cycloalk en C₃ à C₈], - (alkyle en C₁ à C₆)-[cycloalk en C₃ à C₈], -[Ar], - (alkyle en C₁ à C₆)-[Ar], -[Het] ou - (alkyle en C₁ à C₆)-[Het],
[Alk] désigne un reste hydrocarboné acyclique, saturé
ou non saturé, ramifié ou à chaîne droite, qui est non substitué ou qui peut être substitué une ou plusieurs fois identiques ou différentes,
les substituants pouvant être choisis dans le groupe des substituants F, Cl, Br, I, -CN, -NC, -NH₂, -NO₂, -SH, -OH, -CO₂H et -CO₂-alkyle ;
[cycloalk] désigne un reste hydrocarboné cyclique, saturé ou non saturé qui est non substitué ou qui peut être substitué une ou plusieurs fois identiques ou différentes et, le cas échéant, condensé au benzène,
les substituants pouvant être choisis dans le groupe comprenant F, Cl, Br, I, -CN, -NC, -NH₂, -NO₂, -SH, -OH, -CO₂H et -CO₂-alkyle ;
[Ar] désigne un reste non substitué choisi dans le groupe constitué de phényle, naphtyle, anthracényle et biphényle ;
[Het] désigne un reste hétérocyclyle non substitué choisi dans le groupe comprenant les restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle.

11. Utilisation d'un composé de formule générale (IA) ou (IB) 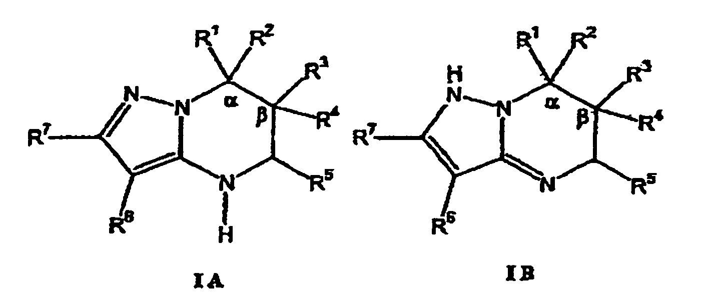 sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou des diastéréoisomères,
ou sous forme de mélanges des stéréoisomères, en particulier des énantiomères ou des diastéréoisomères, dans un rapport de mélange quelconque ;
formule dans laquelle
R¹ et R² représentent, indépendamment l'un de l'autre, H, un groupe O-R⁹, S-R¹⁰, un reste [Alk en C₁ à C₆], aryle' ou - (alkyle en C₁ à C₆)-aryle', les substituants R²⁹, R³⁰ et R³¹ du reste aryle' étant, indépendamment les uns des autres, H, [Alk en C₁ à C₆], F, Cl, Br, I, OH, O-[Alk en C₁ à C_{6]}, O-aryle¹ ou O-CH₂-aryle¹, l'un des restes R¹ et R² représentant H et l'autre de ces deux restes ne représentant pas H ou bien, au cas où l'un des restes R¹ et R² désigne un reste aryle', l'autre de ces deux restes représente H ou un reste alkyle en C₁ à C₆,
R³ et R⁴ représentent H, un reste aryle', -CH₂-aryle' ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle non substitué,
l'un au moins des restes R³ et R⁴ représentant H, ou bien l'un des restes R¹ et R² forme un groupe W conjointement avec l'un des restes R³ et R⁴,
W étant un groupe α'-CH=CH-CH₂-β', α'-CH=CH-CH₂-CH₂-β', α'-O-(CH₂)ₘ-β' où m a la valeur 2, 3, 4 ou 5, 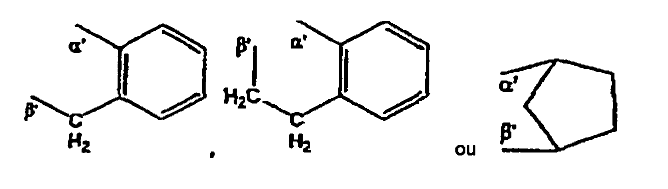 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² désigne H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre reste de R³ et R⁴ désigne H ou un reste méthyle, éthyle, n-propyle, 2-propyle, n-butyle, isobutyle, sec.-butyle, tertio-butyle, n-amyle, isoamyle, sec.-amyle, n-hexyle, isohexyle, sec.-hexyle ;
R⁵ est un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclo-propyle, cyclobutyle, cyclopentyle, cyclo-hexyle ou cycloheptyle, chacun de ces restes étant non substitué ou portant un ou plusieurs substituants identiques ou différents choisis dans le groupe comprenant F, Cl, Br, I, -CN, -NC, -NH₂, -NO₂, -SH, -OH, -CO₂H et -CO₂alkyle, aryle' ou -(CH₂)ₖ₋aryle', où k a la valeur 1, 2, 3 ou 4, hétérocyclyle ou C(=O)R¹¹ ;
R⁶ représente H, un reste méthyle, éthyle, -CN, le fluor, le chlore, le brome, l'iode, un groupe -C(=O)R¹⁷ ou -N=N-aryle¹ ;
R⁷ représente H, un reste aryle¹, un groupe OR¹⁸, S(O)_{q}R¹⁹, où q a la valeur 0, 1 ou 2, ou un reste méthyle non substitué, éthyle, n-propyle, 2-propyle, n-butyle, isobutyle, sec.-butyle, tertiobutyle, n-amyle, iso-amyle, sec.-amyle, n-hexyle, isohexyle ou sec.-hexyle,
R⁹ désigne un reste méthyle non substitué, éthyle, n-propyle, 2-propyle, n-butyle, iso-butyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe -[(CH₂)ᵣ-O]ₛ-H dans lequel r a la valeur 1, 2, 3, 4, 5 ou 6 et s a la valeur 1, 2, 3, 4, 5 ou 6 ;
R¹⁰ est un reste aryle' ;
R¹¹ est un reste aryle' ou un groupe OR²⁵ ;
R¹⁷ est un groupe OR²⁶ ;
R¹⁸ représente H ou un groupe méthyle ;
R¹⁹ représente H, un reste aryle¹ ou un reste méthyle, éthyle, n-propyle, 2-propyle, n-butyle, isobutyle, sec.-butyle, tertio-butyle, n-amyle, isoamyle, sec.-amyle, n-hexyle, isohexyle ou sec.-hexyle, chacun non substitué ;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁶ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ; et
Hétérocyclyle désigne un groupe furanne-2-yle, furanne-3-yle, thiène-2-yle, thiène-3-yle, pyridine-2-yle, pyridine-3-yle ou pyridine-4-yle, les groupes furannyle, thiényle et pyridinyle étant chacun non substitués ou portant chacun un ou plusieurs substituants identiques ou différents choisis dans le groupe comprenant F, Cl, Br, I, -CN, -NO₂, -CO₂H et -CO₂alkyle ;
Aryle' désigne un reste aryle¹, aryle² ou aryle³ ;
aryle¹ représente 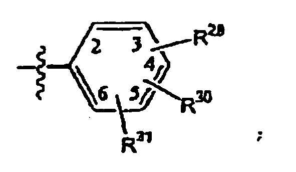
aryle² représente 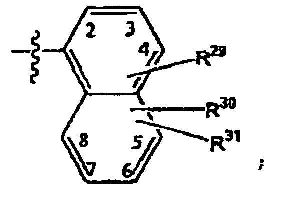
aryle³ représente 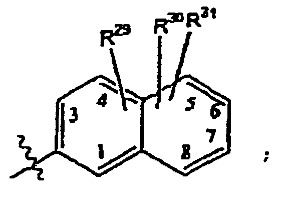
R²⁹, R³⁰ et R³¹ représentent, indépendamment des les uns des autres, H, un reste [Alk en C₁ à C₆], [cycloalk en C₃ à C₈], - (alkyle en C₁ à C₆)-[cycloalk en C₃ à C₈], [Ar], -(alkyle en C₁ à C₆)-[Ar], [Het], -(alkyle en C₁ à C₆)-[Het], F, Cl, Br, I, -CN, -NC, -OR³², -SR³³, -NO, -NO₂, NH₂, NHR³⁴, NR³⁵R³⁶, -N-OH, -N-O[Alk en C₁ à C₆], -NHNH₂, -N=N- [Ar], -(C=O)R³⁷, 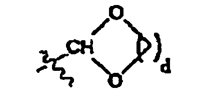 où d a la valeur 1, 2 ou 3, ou un groupe -(C=S)R³⁷, et peuvent occuper n'importe quelle position du noyau ;
R³² et R³³ représentent, indépendamment l'un de l'autre, H, un reste [Alk en C₁ à C₆], [cycloalk en C₃ à C₈], -(alkyle en C₁ à C₆)-[cycloalk en C₃ à C₈], -[Ar], - (alkyle en C₁ à C₆) - [Ar], - [Het], - (alkyle en C₁ à C₆)-[Het], (C=O)R³⁸,- [(CH₂)_{w}-O]_{z}-H ou -[(CH₂)_{w}- O]_{z}-[Alk en C₁ à C₆], avec w = 1, 2, 3 ou 4 et z = 1, 2, 3, 4 ou 5 ;
R³⁴ est un reste [Alk en C₁ à C₆], -CH₂-[Ar] ou -(C=O)O-tertiobutyle ;
R³⁵ et R³⁶ représentent, indépendamment l'un de l'autre, un reste [Alk en C₁ à C₆] ou forment ensemble un reste -(CH₂)_{g}- où g a la valeur 4 ou 5 ;
R³⁷ représente H, un reste - [Alk en C₁ à C₆], - [cycloalk en C₃ à C₈], -(alkyle en C₁ à C₆)-[cycloalk en C₃ à C₈], -[Ar], -(alkyle en C₁ à C₆)-[Ar], -[Het], -(alkyle en C₁ à C₆)-[Het], -OR³⁹, -NH₂, -NHR³⁴, -NR³⁵R³⁶ ;
R³⁸ et représente H, un reste -[Alk en C₁ à C₈], -[cycloalk en C₃ à C₈], -(alkyle en C₁ à C₆)-[cycloalk en C₃ à C₈], -[Ar], -(alkyle en C₁ à C₆)-[Ar] ;
R³⁹ représente H, un reste [Alk en C₁ à C₆], - [cycloalk en C₃ à C₈], -(alkyle en C₁ à C₆)-[cycloalk en C₃ à C₈], -[Ar], -(alkyle en C₁ à C₆) - [Ar], -[Het] ou - (alkyle en C₁ à C₆)-[Het],
[Alk] désigne un reste hydrocarboné acyclique, saturé ou non saturé, ramifié ou à chaîne droite, qui est non substitué ou qui peut être substitué une ou plusieurs fois identiques ou différentes,
les substituants pouvant être choisis dans le groupe des substituants F, Cl, Br, I, -CN, -NC, -NH₂, -NO₂, -SH, -OH, -CO₂H et -CO₂-alkyle ;
[cycloalk] désigne un reste hydrocarboné cyclique, saturé ou non saturé qui est non substitué ou qui peut être substitué une ou plusieurs fois identiques ou différentes et, le cas échéant, condensé au benzène,
les substituants pouvant être choisis dans le groupe comprenant F, Cl, Br, I, -CN, -NC, -NH₂, -NO₂, -SH, -OH, -CO₂H et -CO₂-alkyle ;
[Ar] désigne un reste non substitué choisi dans le groupe constitué de phényle, naphtyle, anthracényle et biphényle ;
[Het] désigne un reste hétérocyclyle non substitué choisi dans le groupe comprenant les restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle,
pour la préparation d'un médicament destiné au traitement de la douleur.

12. Utilisation d'un composé de formule générale (IA) ou (IB) 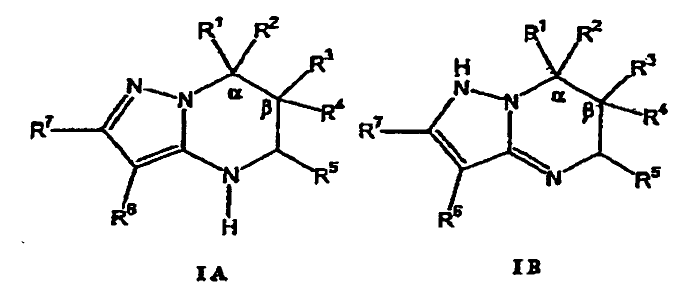 sous la forme représentée ou sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
formule dans laquelle R¹, R², R³, R⁴, R⁵, R⁶ et R⁷ ont la définition indiquée dans la revendication 11 ;
pour la préparation d'un médicament destiné au traitement et/ou à la prophylaxie de l'épilepsie, la schizophrénie, des maladies neurodégénératives, en particulier la maladie d'Alzheimer, la maladie de Huntington et la maladie de Parkinson, d'ischémies et d'infarctus cérébraux, de psychoses causées par des taux élevés d'aminoacides, des oedèmes cérébraux, des états de sous-alimentation du système nerveux central, en particulier en cas d'hypoxies et d'anoxies, de démence liée au SIDA, d'encéphalomyélite, du syndrome de Tourette, de l'asphyxie périnatale et/ou en cas d'acouphènes.

13. Utilisation d'un composé suivant la formule (IA) et/ou (IB) 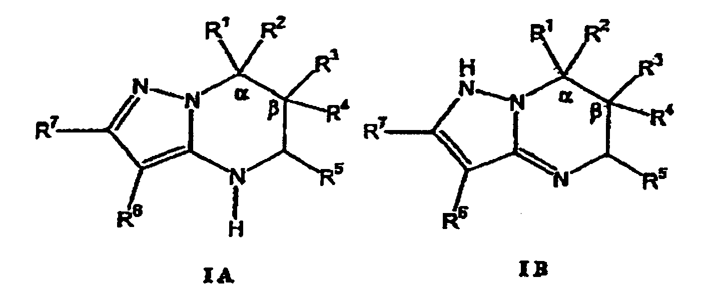 sous la forme représentée ou sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou diastéréoisomères, ou sous forme de mélanges des stéréoisomères, en particulier des énantiomères ou des diastéréoisomères, dans un rapport de mélange quelconque ;
formule dans laquelle
R¹ et R² représentent, indépendamment l'un de l'autre, H, un groupe O-R⁹, S-R¹⁰, un reste [Alk en C₁ à C₆], aryle' ou - (alkyle en C₁ à C₆)-aryle', les substituants R²⁹, R³⁰ et R³¹ du reste aryle' étant, indépendamment les uns des autres, H, [Alk en C₁ à C₆], F, Cl, Br, I, OH, O-[Alk en C₁ à C₆], O-aryle¹ ou O-CH₂-aryle¹, l'un des restes R¹ et R² représentant H et l'autre de ces deux restes ne représentant pas H ou bien, au cas où l'un des restes R¹ et R² désigne un reste aryle', l'autre de ces deux restes représente H ou un reste alkyle en C₁ à C₆,
R³ et R⁴ représentent H, un reste aryle', -CH₂-aryle¹ ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle non substitué,
l'un au moins des restes R³ et R⁴ représentant H, ou bien l'un des restes R¹ et R² forme un groupe W conjointement avec l'un des restes R³ et R⁴,
W étant un groupe α'-CH=CH-CH₂-β', α'-CH=CH-CH₂-CH₂-β', α'-O-(CH₂)ₘ-β' où m a la valeur 2, 3, 4 ou 5, 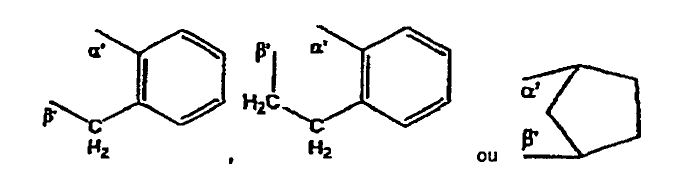 l'extrémité de W désignée par α' est liée à l'atome désigné par α du composé de formule générale (IA) ou (IB) et l'extrémité de W désignée par β' est liée à l'atome désigné par β du composé de formule générale (IA) ou (IB), l'autre reste de R¹ et R² désigne H ou un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle et l'autre reste de R³ et R⁴ désigne H ou un reste méthyle, éthyle, n-propyle, 2-propyle, n-butyle, isobutyle, sec.-butyle, tertio-butyle, n-amyle, isoamyle, sec.-amyle, n-hexyle, isohexyle, sec.-hexyle ;
R⁵ est un reste méthyle, éthyle, n-propyle, 2-propyle, n-butyle, isobutyle, sec.-butyle, tertiobutyle, n-amyle, isoamyle, sec.-amyle, n-hexyle, isohexyle, sec.-hexyle, cyclo-propyle, cyclobutyle, cyclopentyle, cyclo-hexyle ou cycloheptyle, chacun de ces restes étant non substitué ou portant un ou plusieurs substituants identiques ou différents choisis dans le groupe comprenant F, Cl, Br, I, -CN, -NC, -NH₂, -NO₂, -SH, -OH, -CO₂H et -CO₂alkyle, aryle' ou -(CH₂)ₖ₋aryle', où k a la valeur 1, 2, 3 ou 4, hétérocyclyle ou C(=O)R¹¹ ;
R⁶ représente H, un reste méthyle, éthyle, -CN, le fluor, le chlore, le brome, l'iode, un groupe -C(=O)R¹⁷ ou -N=N-aryle¹ ;
R⁷ représente H, un reste aryle¹, un groupe OR¹⁸, S(O)_{q}R¹⁹, où q a la valeur 0, 1 ou 2, ou un reste méthyle non substitué, éthyle, n-propyle, 2-propyle, n-butyle, isobutyle, sec.-butyle, tertiobutyle, n-amyle, iso-amyle, sec.-amyle, n-hexyle, isohexyle ou sec.-hexyle,
R⁹ désigne un reste méthyle non substitué, éthyle, n-propyle, 2-propyle, n-butyle, iso-butyle, sec.-butyle, tertiobutyle, n-amyle, isoamyle, sec.-amyle, n-hexyle, isohexyle, sec.-hexyle, cyclopropyle, cyclobutyle, cyclopentyle, cyclohexyle ou cycloheptyle ou un groupe [(CH₂)ᵣ-O]ₛ-H dans lequel r a la valeur 1, 2, 3, 4, 5 ou 6 et s a la valeur 1, 2, 3, 4, 5 ou 6 ;
R¹⁰ est un reste aryle' ;
R¹¹ est un reste aryle' ou un groupe OR²⁵ ;
R¹⁷ est un groupe OR²⁶ ;
R¹⁸ représente H ou un groupe méthyle ;
R¹⁹ représente H, un reste aryle¹ ou un reste méthyle, éthyle, n-propyle, 2-propyle, n-butyle, isobutyle, sec.-butyle, tertio-butyle, n-amyle, isoamyle, sec.-amyle, n-hexyle, isohexyle ou sec.-hexyle chacun non substitué ;
R²⁵ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle, R²⁵ ne représentant pas H lorsque R¹ est un reste aryle en même temps que R² est un reste alkyle ;
R²⁶ représente H, un reste méthyle, éthyle, n-propyle, 2-propyle, n-butyle, isobutyle, sec.-butyle, tertiobutyle, n-amyle, isoamyle, sec.-amyle, n-hexyle, isohexyle ou sec.-hexyle ; et
Hétérocyclyle désigne un groupe furanne-2-yle, furanne-3-yle, thiène-2-yle, thiène-3-yle, pyridine-2-yle, pyridine-3-yle ou pyridine-4-yle, les groupes furannyle, thiényle et pyridinyle étant chacun non substitués ou portant chacun un ou plusieurs substituants identiques ou différents choisis dans le groupe comprenant F, Cl, Br, I, -CN, -NO₂, -CO₂H et -CO₂alkyle ;
Aryle' désigne un reste aryle¹, aryle² ou aryle³ ;
aryle¹ représente 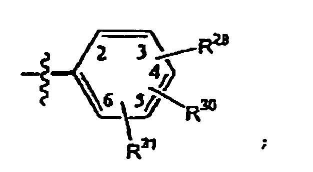
aryle² représente 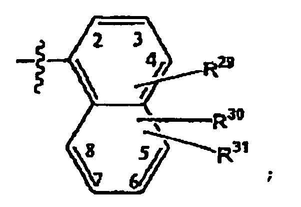
aryle ³ représente 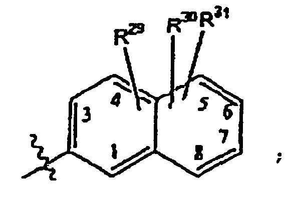
R²⁹, R³⁰ et R³¹ représentent, indépendamment des lns des autres, H, un reste [Alk en C₁ à C₆], [cycloalk en C₃ à C₈], - (alkyle en C₁ à C₆)-[cycloalk en C₃ à C₈], [Ar], -(alkyle en C₁ à C₆)-[Ar], [Het], - (alkyle en C₁ à C₆) - [Het], F, Cl, Br, I, -CN, -NC, -OR³², -SR³³, -NO, -NO₂, NH₂, NHR³⁴, NR³⁵R³⁶, -N-OH, -N-O[Alk en C₁ à C₆], -NHNH₂, -N=N-[Ar], - (C=O)R³⁷, 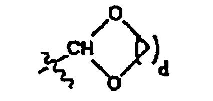 où d a la valeur 1, 2 ou 3, ou un groupe -(C=S)R³⁷, et peuvent occuper n'importe quelle position du noyau ;
R³² et R³³ représentent, indépendamment l'un de l'autre, H, un reste [Alk en C₁ à C₆], [cycloalk en C₃ à C₈], -(alkyle en C₁ à C₆)-[cycloalk en C₃ à C₈], -[Ar], - (alkyle en C₁ à C₆)-[Ar], -[Het], -(alkyle en C₁ à C₆)-[Het], (C=O)R³⁸, -[(CH₂)_{w}-O]_{z}-H ou -[(CH₂)_{w}- O]_{z}-[Alk en C₁ à C₆], avec w = 1, 2, 3 ou 4 et z = 1, 2, 3, 4 ou 5 ;
R³⁴ est un reste [Alk en C₁ à C₆], -CH₂-[Ar] ou -(C=O)O-tertiobutyle ;
R³⁵ et R³⁶ représentent, indépendamment l'un de l'autre, un reste [Alk en C₁ à C₆] ou forment ensemble un reste -(CH₂)_{g}- où g a la valeur 4 ou 5 ;
R³⁷ représente H, un reste - [Alk en C₁ à C₆], - [cycloalk en C₃ à C₈], - (alkyle en C₁ à C₆)-[cycloalk en C₃ à C₈], - [Ar], - (alkyle en C₁ à C₆) - [Ar], -[Het], - (alkyle en C₁ à C₆)-[Het], -OR³⁹, -NH₂, -NHR³⁴, -NR³⁵R³⁶ ;
R³⁸ et représente H, un reste -[Alk en C₁ à C₆], -[cycloalk en C₃ à C₈], -(alkyle en C₁ à C₆)-[cycloalk en C₃ à C₈], -[Ar], -(alkyle en C₁ à C₆) - [Ar] *i*
R³⁹ représente H, un reste [Alk en C₁ à C₆], - [cycloalk en C₃ à C₈], - (alkyle en C₁ à C₆)-[cycloalk en C₃ à C₈], -[Ar], -(alkyle en C₁ à C₆) - [Ar], - [Het] ou - (alkyle en C₁ à C₆) - [Het],
[Alk] désigne un reste hydrocarboné acyclique, saturé ou non saturé, ramifié ou à chaîne droite, qui est non substitué ou qui peut être substitué une ou plusieurs fois identiques ou différentes,
les substituants pouvant être choisis dans le groupe des substituants F, Cl, Br, I, -CN, -NC, -NH₂, -NO₂, -SH, -OH, -CO₂H et -CO₂-alkyle ;
[cycloalk] désigne un reste hydrocarboné cyclique, saturé ou non saturé qui est non substitué ou qui peut être substitué une ou plusieurs fois identiques ou différentes et, le cas échéant, condensé au benzène,
les substituants pouvant être choisis dans le groupe comprenant F, Cl, Br, I, -CN, -NC, -NH₂, -NO₂, -SH, -OH, -CO₂H et -CO₂-alkyle ;
[Ar] désigne un reste non substitué choisi dans le groupe constitué de phényle, naphtyle, anthracényle et biphényle ;
[Het] désigne un reste hétérocyclyle non substitué choisi dans le groupe comprenant les restes pyrrolidinyle, tétrahydrofuryle, pipéridinyle, pipérazinyle et morpholinyle ou un reste hétéroaryle non substitué choisi dans le groupe constitué des restes pyrrolyle, pyrazolyle, imidazolyle, pyridazinyle, pyrimidinyle, pyrazinyle, furannyle, thiényle et pyridinyle,
comme ligand de protéines de transport de nucléosides et/ou d'adénosine-kinase et/ou d'adénosine-désaminase et/ou de récepteurs A₁ et/ou de récepteurs A₂ et/ou de récepteurs A₃.

14. Utilisation d'un composé de formules (IA) et/ou (IB) 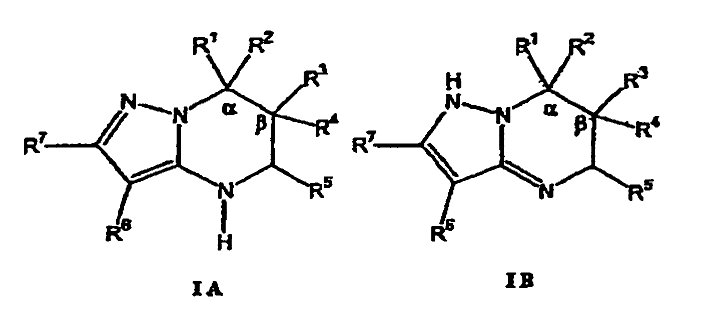 sous la forme représentée ou sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou diastéréoisomères, ou sous forme de mélanges des stéréoisomères, en particulier des énantiomères ou des diastéréoisomères, dans un rapport de mélange quelconque ;
formule dans laquelle R¹, R², R³, R⁴, R⁵, R⁶ et R⁷ sont tels que définis dans la revendication 13 ;
pour la préparation d'un médicament destiné à la prévention et/ou au traitement d'états et/ou de maladies qui sont influencés par une stimulation et/ou par une inhibition de protéines de transport de nucléosides et/ou de l'adénosine-kinase et/ou de l'adénosine-désaminase et/ou de récepteurs A₁ et/ou de récepteurs A₂ et/ou de récepteurs A₃.

15. Utilisation suivant l'une des revendications 13 ou 14, pour la préparation d'un médicament destiné à la prévention et/ou au traitement de la douleur, d'une douleur neuropathique, de maladies des voies respiratoires, du cancer, d'arythmies cardiaques, d'ischémies, de l'épilepsie, de la maladie de Huntington, de troubles et de maladies immunitaires, d'états et de maladies inflammatoires, de l'hypoxie du nouveau-né, de maladies neurodégénératives, de la maladie de Parkinson, de l'insuffisance rénale, de la schizophrénie, de troubles du sommeil, de l'apoplexie cérébrale, de thromboses, de l'incontinence d'urine, du diabète, du psoriasis, du choc septique, de traumatismes cérébraux, du glaucome et/ou de l'insuffisance cardiaque congestive.

16. Utilisation suivant l'une quelconque des revendications 13 à 15, **caractérisée en ce que**
R¹ et R² représentent, indépendamment l'un de l'autre, H, un groupe O-CH₂-CH₂-OH, un reste O-cyclohexyle, S-phényle, méthyle, phényle, 3-fluorophényle, 3-bromophényle, 4-bromophényle, 4-chlorophényle, 4-fluorophényle, 3-méthylphényle, 4-hydroxy-phényle, 4-méthoxyphényle, 2,4-diméthylphényle, 3,4-diméthoxyphényle, 2,3,4-triméthoxyphényle, 2-naphtyle ou -CH₂-phényle,
R³ et R⁴ représentent H, un reste méthyle ou 4-méthoxy-phényle, l'un au moins des restes R³ et R⁴ représentant H,
ou bien
l'un des restes R¹ et R² forme, conjointement avec l'un des restes R³ et R⁴, un groupe W
W étant un groupe α'-CH=CH-CH₂-β', α'-CH=CH-CH₂₋CH₂-β', α'-O-(CH₂)ₘ-β' avec m = 2, 3, 4 ou 5, 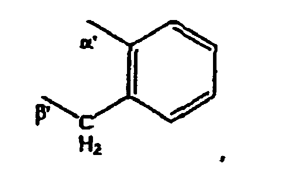 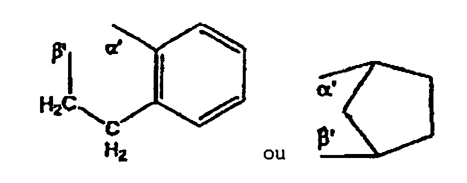 l'extrémité de W désignée par α' est liée à l'atome désigné par α du composé de formules générales (IA) et/ou (IB), l'extrémité de W désignée par β' est liée à l'atome désigné par β du composé de formules générales (IA) et/ou (IB), l'autre reste de R¹ et R² et l'autre reste de R³ et R⁴ représentent chacun H ;
R⁵ est un reste n-propyle, n-butyle, tertiobutyle, -(CH₂)₄-OH, cyclopropyle, ester éthylique d'acide cycloprop-2-yl-1-carboxylique, cyclohexyle, 4-trifluoro-phényle, 4-phénoxyphényle, 2-hydroxy-3-méthoxyphényle, 4-hydroxy-3-méthoxyphényle, 3-carboxy-2-hydroxyphényle, -(CH₂)₂-phényle, 5-carboxyfuranne-2-yle, 5 méthylfuranne-2-yle, 5-nitrofuranne-2-yle, 5-nitrothiène-2-yle, pyridine-2-yle, pyridine-3-yle, C(=O)-phényle, C(=O)OH ou C(=O)O-éthyle, R⁵ ne représentant pas un groupe C(=O)OH lorsque R¹ est un reste aryle en même que R² est un reste alkyle ;
R⁶ représente H, un groupe -CN, le brome, un groupe -C(=O)OH, -C(=O)O-éthyle ou -N=N-phényle ; et
R⁷ représente H, un reste phényle, un groupe OH, un reste-S-méthyle, -SO₂-(4-nitro-phényle) ou tertiobutyle.

17. Composition pharmaceutique, qui contient au moins un composé de formule générale (IA) ou (IB), 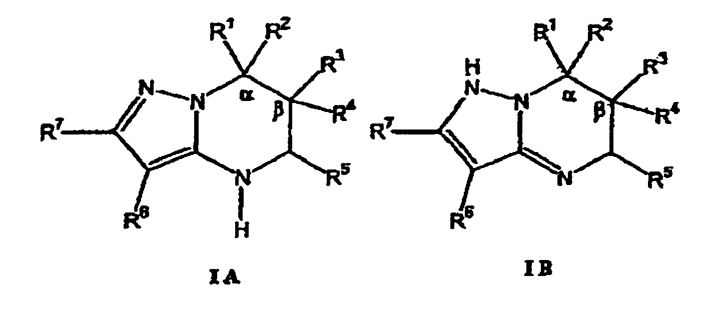 sous la forme représentée ou sous forme de son ou ses acides ou de sa ou ses bases ou sous forme de l'un de ses sels, en particulier des sels acceptables du point de vue physiologique, ou sous forme de l'un de ses produits de solvatation, en particulier des hydrates ;
sous forme de son racémate, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
formule dans laquelle R¹, R², R³, R⁴, R⁵, R⁶ et R⁷ sont tels que définis dans la revendication 1 ;
ainsi qu'au moins une substance pharmaceutique auxiliaire.
